# EUROPÄISCHE PATENTSCHRIFT

(11) **EP 1 768 973 B1**
(45) Veröffentlichungstag und Bekanntmachung des Hinweises auf die Patenterteilung: **05.12.2007**
(21) Anmeldenummer: 05760072.8
(22) Anmeldetag: 13.07.2005
(51) Int. Cl.: C07D 401/04, C07D 215/56, A61K 31/47, A61K 31/4709, A61P 31/12

(54) **SUBSTITUIERTE CHINOLONE**
SUBSTITUTED QUINOLONES
QUINOLONES SUBSTITUEES

(30) Priorität: 21.07.2004 DE 102004035203
(43) Veröffentlichungstag der Anmeldung: 04.04.2007
(73) Patentinhaber: AiCuris GmbH & Co. KG, 42117 Wuppertal (DE)
(72) Erfinder: SCHOHE-LOOP, Rudolf, 42327 Wuppertal (DE); ZIMMERMANN, Holger, 42113 Wuppertal (DE); HENNINGER, Kerstin, 42115 Wuppertal (DE); PAULSEN, Daniela, 42115 Wuppertal (DE); RÖLLE, Thomas, 51381 Leverkusen (DE); LANG, Dieter, 42553 Velbert (DE); THEDE, Kai, 42115 Wuppertal (DE); FÜRSTNER, Chantal, 45478 Mülheim an der Ruhr (DE); BRÜCKNER, David, 45128 Essen (DE); KÖBBERLING, Johannes, 41516 Grevenbroich (DE); BAUSER, Marcus, 42115 Wuppertal (DE)
(74) Vertreter: Weller, Wolfgang
(86) Internationale Anmeldenummer: PCT/EP2005/007601
(87) Internationale Veröffentlichungsnummer: WO 2006/008046

(56) Entgegenhaltungen:
- WO-A-00/40561
- WO-A-97/04779
- DATABASE CHEMCATS [Online] Chemical Abstracts Service, Columbus, Ohio, US; 1. Januar 2004 (2004-01-01), XP002348331 gefunden im STN & "Ambiter Screening Library" 1. Januar 2004 (2004-01-01), AMBITER , 46 QUAI LOUIS BLERIOT, PARIS, F-75016, FRANCE

## Beschreibung

Die Erfindung betrifft substituierte Chinolone und Verfahren zu ihrer Herstellung sowie ihre Verwendung zur Herstellung von Arzneimitteln zur Behandlung und/oder Prophylaxe von Krankheiten, insbesondere zur Verwendung als antivirale Mittel, insbesondere gegen Cytomegaloviren.

WO 00/040561 und US 4,959,363 beschreiben Chinolone mit Wirkung gegen Viren der Herpes-Familie. EP-A 612731 beschreibt Chinolone als antivirale Mittel, besonders gegen HIV. WO 02/009758, WO02/085886 und WO03/050107 beanspruchen Chinolone als Breitspektrum Antibiotika. In WO 97/004775 und WO 97/004779 werden Chinolone als Inhibitoren von PDE4 und TNFα unter anderem für die Behandlung von antiinflammatorischen Erkrankungen und HIV. beschrieben. EP-A 276700 beschreibt 8-Cyano-Chinolone als Antibiotika. WO02/026713 beschreibt Chinolone als antiparasitäre Verbindungen.

Auf dem Markt sind strukturell andersartige, antiviral wirkende Mittel vorhanden, deren Anwendungsbreite aufgrund eines ausgeprägten Nebenwirkungsprofils und möglicher Resistenzentwicklung stark eingeschränkt ist. Neue Mittel für eine bessere und wirksamere Therapie sind daher wünschenswert.

Eine Aufgabe der vorliegenden Erfindung ist es daher, neue Verbindungen mit gleicher oder verbesserter antiviraler Wirkung zur Behandlung von viralen Infektionskrankheiten bei Menschen und Tieren zur Verfügung zu stellen.

Überraschenderweise wurde gefunden, dass die in der vorliegenden Erfindung beschriebenen substituierten Chinolone antiviral wirksam sind.

Gegenstand.der Erfindung sind Verbindungen der Formel 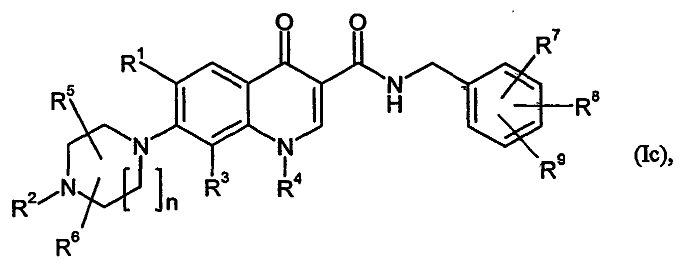 in welcher
- n: für eine Zahl 1 oder 2 steht,
- R¹: für Fluor, Chlor oder Trifluormethyl steht,
- R²: für Wasserstoff oder C₁-C₆-Alkyl steht,
wobei Alkyl substituiert sein kann mit 1 bis 2 Substituenten, wobei die Substituenten unabhängig voneinander ausgewählt werden aus der Gruppe, bestehend aus Hydroxy, Aminocarbonyl, C₁-C₆-Alkoxy, C₁-C₆-Alkylcarbonyl, C₁-C₆-Alkylaminocarbonyl, C₃-C₈-Cycloalkylaminocarbonyl, C₁-C₆-Alkylaminocarbonylaminocarbonyl, C₁-C₆-Alkylsulfonylaminocarbonyl, Phenoxy, 5- oder 6-gliedriges Heteroaryloxy, 5- bis 7-gliedriges Heterocyclyl, 5- oder 6-gliedriges Heteroaryl, 5- bis 7-gliedriges Heterocyclylcarbonyl und 5- oder 6-gliedriges Heteroarylcarbonyl,
worin Alkoxy seinerseits substituiert sein kann mit einem Substituenten, wobei der Substituent ausgewählt wird aus der Gruppe, bestehend aus Hydroxy, Phenyl, 5- bis 7-gliedriges Heterocyclyl und 5- oder 6-gliedriges Heteroaryl,
oder
- R²: für C₁-C₆-Alkylcarbonyl, gegebenenfalls einmal mit C₁-C₄-Alkoxy substituiertes C₁-C₆-Alkylaminocarbonyl oder C₃-C₈-Cycloalkylaminocarbonyl steht,
wobei Alkylcarbonyl substituiert sein kann mit einem Substituenten, wobei der Substituent ausgewählt wird aus der Gruppe, bestehend aus Amino, C₁-C₆-Alkylamino, C₃-C₈-Cycloalkylamino und 4- bis 7-gliedriges Heterocyclyl,
- R³: für Halogen, Cyano, Methoxy, Monofluormethoxy, Difluormethoxy, Trifluormethoxy oder Ethinyl steht,
- R⁴: für C₁-C₆-Alkyl oder C₃-C₈-Cycloalkyl steht,
wobei Alkyl substituiert sein kann mit 1 bis 3 Substituenten, wobei die Substituenten unabhängig voneinander ausgewählt werden aus der Gruppe, bestehend aus Halogen, Hydroxy, Amino, Cyano, Trifluormethyl, Hydroxycarbonyl, Aminocarbonyl, C₁-C₆-Alkoxy, C₁-C₆-Alkylamino, C₁-C₆-Alkylcarbonyl und C₁-C₆-Alkoxycarbonyl,
und
wobei Cycloalkyl substituiert sein kann mit 1 bis 3 Substituenten, wobei die Substituenten unabhängig voneinander ausgewählt werden aus der Gruppe, bestehend aus Halogen, Hydroxy, Amino, Cyano, Trifluormethyl, Hydroxycarbonyl, Aminocarbonyl, C₁-C₆-Alkyl, C₁-C₆-Alkoxy, C₁-C₆-Alkylamino, C₁-C₆-Alkylcarbonyl und C₁-C₆-Alkoxycarbonyl,
- R⁵ und R⁶: unabhängig voneinander für Wasserstoff, Methyl oder Ethyl stehen,
- R⁷ und R⁸: unabhängig voneinander für Halogen, Hydroxy, Cyano, Trifluormethyl, Monofluormethoxy, Difluormethoxy, Trifluormethoxy, C₁-C₃-Alkyl oder C₁-C₃-Alkoxy stehen,
- R⁹: für Wasserstoff, Halogen, Hydroxy, Cyano, Trifluormethyl, Monofluormethoxy, Difluormethoxy, Trifluormethoxy, C₁-C₃-Alkyl oder C₁-C₃-Alkoxy steht,
und ihre Salze, ihre Solvate und die Solvate ihrer Salze.

Erfindungsgemäße Verbindungen sind die Verbindungen der Formel (Ic), (I), (Ia) und (Ib) und deren Salze, Solvate und Solvate der Salze; die von Formel (Ic), (I), (Ia) und (Ib) umfassten Verbindungen der nachfolgend genannten Formeln und deren Salze, Solvate und Solvate der Salze sowie die von Formel (Ic), (I), (Ia) und (Ib) umfassten, nachfolgend als Ausführungsbeispiele genannten Verbindungen und deren Salze, Solvate und Solvate der Salze, soweit es sich bei den von Formel (Ic), (I), (Ia) und (Ib) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:

Alkyl per se und "Alk" und "Alkyl" in Alkoxy Alkylamino, Alkylcarbonyl, Alkoxycarbonyl, Alkylaminocarbonyl, Alkylaminocarbonylaminocarbonyl und Alkylsulfonylaminocarbonyl stehen für einen linearen oder verzweigten Alkylrest mit in der Regel 1 bis 6, vorzugsweise 1 bis 4, besonders bevorzugt 1 bis 3 Kohlenstoffatomen, beispielhaft und vorzugsweise für Methyl, Ethyl, n-Propyl, Isopropyl, *tert-*Butyl, n-Pentyl und n-Hexyl.

Alkoxy steht beispielhaft und vorzugsweise für Methoxy, Ethoxy, n-Propoxy, Isopropoxy, *tert-*Butoxy, n-Pentoxy und n-Hexoxy.

Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, *tert*-Butylamino, n-Pentylamino, n-Hexylamino, *N*,*N*-Dimethylamino, *N,N-*Diethylamino, *N*-Ethyl-*N*-methylamino, *N*-Methyl-*N*-n-propylamino, *N*-Isopropyl-*N*-n-propylamino, *N*-*tert*-Butyl-*N*-methylamino, *N*-Ethyl-*N*-n-pentylamino und *N*-n-Hexyl-*N-*methylamino. C₁-C₃-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.

Alkylcarbonyl steht beispielhaft und vorzugsweise für Acetyl und Propanoyl.

Alkoxycarbonyl steht beispielhaft und vorzugsweise für Methoxycarbonyl, Ethoxycarbonyl, n-Propoxycarbonyl, Isopropoxycarbonyl, *tert*-Butoxycarbonyl, n-Pentoxycarbonyl und n-Hexoxycarbonyl.

Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, Isopropylaminocarbonyl, *tert-*Butylaminocarbonyl, n-Pentylaminocarbonyl, n-Hexyl-aminocarbonyl, *N,N-*Dimethylaminocarbonyl, *N,N-*Diethylaminocarbonyl, *N*-Ethyl-*N*-methylaminocarbonyl, *N-*Methyl-*N*-n-propylaminocarbonyl, *N*-Isopropyl-*N-* n-propyl-aminocarbonyl, *N*-*tert*-Butyl-*N*-methylaminocarbonyl, *N*-Ethyl-*N*-n-pentylaminocarbonyl und *N*-n-Hexyl-*N*-methylaminocarbonyl. C₁-C₃-Alkylaminocarbonyl steht beispielsweise für einen Monoalkylaminocarbonylrest mit 1 bis 3 Kohlenstoffatomen oder für einen Dialkylaminocarbonylrest mit jeweils 1 bis 3 Kohlenstoffatomen pro Alkylsubstituent.

Alkylaminocarbonylaminocarbonyl steht für einen Alkylaminocarbonylaminocarbonylrest mit einem oder zwei (unabhängig voneinander gewählten) Alkylsubstituenten, beispielhaft und vorzugsweise für Methylaminocarbonylaminocarbonyl, Ethylaminocarbonylaminocarbonyl, n-Propylaminocarbonylaminocarbonyl, Isopropylaminocarbonylaminocarbonyl, *tert*-Butylaminocarbonylaminocarbonyl, n-Pentylaminocarbonylaminocarbonyl, n-Hexylaminocarbonylaminocarbonyl, *N,N-*Dimethylaminocarbonylaminocarbonyl, *N,N-*Diethylaminocarbonylaminocarbonyl, *N*-Ethyl-*N*-methylaminocarbonylaminocarbonyl, *N*-Methyl-*N*-n-propylaminocarbonylaminocarbonyl, *N*-Isopropyl-*N*-n-propyl-aminocarbonylaminocarbonyl, *N*-*tert*-Butyl-*N*-methylaminocarbonylaminocarbonyl, *N*-Ethyl-*N*-n-pentylaminocarbonylaminocarbonyl und *N-*n-Hexyl-*N-*methylaminocarbonylaminocarbonyl. C₁-C₃-Alkylaminocarbonylaminocarbonyl steht beispielsweise für einen Monoalkylaminocarbonylaminocarbonylrest mit 1 bis 3 Kohlenstoffatomen oder für einen Dialkylaminocarbonylaminocarbonylrest mit jeweils 1 bis 3 Kohlenstoffatomen pro Alkylsubstituent.

Alkylsulfonylaminocarbonyl steht beispielhaft und vorzugsweise für Methylsulfonylaminocarbonyl, Ethylsulfonylaminocarbonyl, n-Propylsulfonylaminocarbonyl, Isopropylsulfonylaminocarbonyl, *tert*-Butylsulfonylaminocarbonyl, n-Pentylsulfonylaminocarbonyl und n-Hexylsulfonylaminocarbonyl.

Cycloalkyl steht für eine Cycloalkylgruppe mit in der Regel 3 bis 8, bevorzugt 3 bis 5 Kohlenstoffatomen, beispielhaft und vorzugsweise für Cycloalkyl sind genannt Cyclopropyl, Cyclobutyl, Cyclopentyl, Cyclohexyl und Cycloheptyl.

Cycloalkylamino steht beispielhaft und vorzugsweise für Cyclopropylamino, Cyclobutylamino, Cyclopentylamino, Cyclohexylamino und Cycloheptylamino.

Cycloalkylaminocarbonyl steht beispielhaft und vorzugsweise für Cyclopropylaminocarbonyl, Cyclobutylaminocarbonyl, Cyclopentylaminocarbonyl, Cyclohexylaminocarbonyl und Cycloheptylaminocarbonyl.

Heteroaryl per se und "Heteroaryl" in Heteroaryloxy und Heteroarylcarbonyl steht für einen aromatischen, mono- oder bicyclischen Rest mit in der Regel 5 bis 10, vorzugsweise 5 bis 6 Ringatomen und bis zu 5, vorzugsweise bis zu 4 Heteroatomen aus der Reihe S, O und N, wobei Heteroaryl einen Oxo-Substituenten tragen kann, beispielhaft und vorzugsweise für Thienyl, Furyl, Pyrrolyl, Thiazolyl, Oxazolyl, Isoxazolyl, Oxadiazolyl, Pyrazolyl, Imidazolyl, Tetrazolyl, Pyridyl, Pyrimidyl, Pyridazinyl, Pyrazinyl, Indolyl, Indazolyl, Benzofuranyl, Benzothiophenyl, Chinolinyl, Isochinolinyl.

Heteroaryloxy steht beispielhaft und vorzugsweise für Thienyloxy, Furyloxy, Pyrrolyloxy, Thiazolyloxy, Oxazolyloxy, Isoxazolyloxy, Oxadiazolyloxy, Pyrazolyloxy, Imidazolyloxy, Tetrazolyloxy, Pyridyloxy, Pyrimidyloxy, Pyridazinyloxy, Pyrazinyloxy, Indolyloxy, Indazolyloxy, Benzofuranyloxy, Benzothiophenyloxy, Chinolinyloxy, Isochinolinyloxy.

Heteroarylcarbonyl steht beispielhaft und vorzugsweise für Thienylcarbonyl, Furylcarbonyl, Pyrrolylcarbonyl, Thiazolylcarbonyl, Oxazolylcarbonyl, Isoxazolylcarbonyl, Oxadiazolylcarbonyl, Pyrazolylcarbonyl, Imidazolylcarbonyl, Tetrazolylcarbonyl, Pyridylcarbonyl, Pyrimidylcarbonyl, Pyridazinylcarbonyl, Pyrazinylcarbonyl, Indolylcarbonyl, Indazolylcarbonyl, Benzofuranylcarbonyl, Benzothiophenylcarbonyl, Chinolinylcarbonyl, Isochinolinylcarbonyl.

Heterocyclyl per se und "Heterocyclyl" in Heterocyclylcarbonyl steht für einen mono- oder polycyclischen, vorzugsweise mono- oder bicyclischen, heterocyclischen Rest mit in der Regel 4 bis 10, vorzugsweise 5 bis 8 Ringatomen und bis zu 3, vorzugsweise bis zu 2 Heteroatomen und/oder Heterogruppen aus der Reihe N, O, S, SO, SO₂. Die Heterocyclyl-Reste können gesättigt oder teilweise ungesättigt sein. Bevorzugt sind 5- bis 8-gliedrige, monocyclische gesättigte Heterocyclylreste mit bis zu zwei Heteroatomen aus der Reihe O, N und S, wie beispielhaft und vorzugsweise Tetrahydrofuran-2-yl, Tetrahydrofuran-3-yl, Pyrrolidin-2-yl, Pyrrolidin-3-yl, Pyrrolinyl, Piperidin-1-yl, Piperidin-2-yl, Piperidin-3-yl, Piperidin-4-yl, Piperazin-1-yl, Piperazin-2-yl, Morpholin-2-yl, Morpholin-3-yl, Morpholin-4-yl, Thiomorpholin-2-yl, Thiomorpholin-3-yl, Thiomorpholin-4-yl, Perhydroazepin-1-yl, Perhydroazepin-2-yl, Perhydroazepin-3-yl, Perhydroazepin-4-yl.

Pyrrolinyl per se und "Pyrrolinyl" in Pyrrolinylcarbonyl steht für 3,4-Dihydro-2*H*-pyrrol-2-yl, 3,4-Dihydro-2*H*-pyrrol-3-yl, 3,4-Dihydro-2*H*-pyrrol-4-yl, 3,4-Dihydro-2*H*-pyrrol-5-yl, 2,3-Dihydro-1*H*-pyrrol-1-yl, 2,3-Dihydro-1*H*-pyrrol-2-yl, 2,3-Dihydro-1*H*-pyrrol-3-yl, 2,3-Dihydro-1*H*-pyrrol-4-yl, 2,3-Dihydro-1*H*-pyrrol-5-yl, 2,5-Dihydro-1*H*-pyrrol-1-yl, 2,5-Dihydro-1H-pyrrol 2-yl, 2;5-Dihydro-1H-pyrrol-3-yl, 2,5-Dihydro-1H-pyrrol-4-yl und 2,5-Dihydro-1H-pyrrol-5-yl.

Heterocyclylcarbonyl steht beispielhaft und vorzugsweise für Tetrahydrofuran-2-ylcarbonyl, Tetrahydrofuran-3-ylcarbonyl, Pyrrolidin-2-ylcarbonyl, Pyrrolidin-3-ylcarbonyl, Pyrrolinylcarbonyl, Piperidin-1-ylcarbonyl, Piperidin-2-ylcarbonyl, Piperidin-3-ylcarbonyl, Piperidin-4-ylcarbonyl, Piperazin-1-ylcarbonyl, Piperazin-2-ylcarbonyl, Morpholin-2-ylcarbonyl, Morpholin-3-ylcarbonyl, Morpholin-4-ylcarbonyl, Thiomorpholin-2-ylcarbonyl, Thiomorpholin-3-ylcarbonyl, Thiomorpholin-4-ylcarbonyl, Perhydroazepin-1-ylcarbonyl, Perhydroazepin-2-ylcarbonyl, Perhydroazepin-3-ylcarbonyl, Perhydroazepin-4-ylcarbonyl.

Halogen steht für Fluor, Chlor, Brom und Jod, bevorzugt Fluor und Chlor.

Bevorzugt sind solche Verbindungen der Formel (Ic), welche der Formel 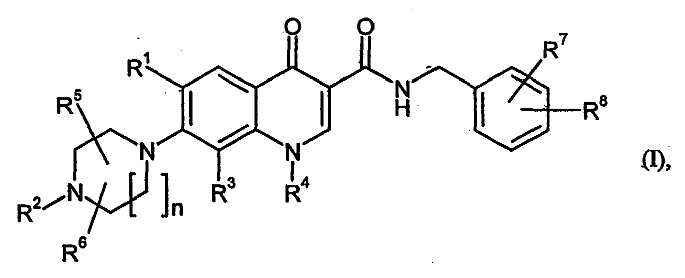 entsprechen,
in welcher
- n: für eine Zahl 1 oder 2 steht,
- R¹: für Fluor, Chlor oder Trifluormethyl steht,
- R²: für Wasserstoff oder C₁-C₆-Alkyl steht,
wobei Alkyl substituiert sein kann mit 1 bis 2 Substituenten, wobei die Substituenten unabhängig voneinander ausgewählt werden aus der Gruppe, bestehend aus Hydroxy, Aminocarbonyl, C₁-C₆-Alkoxy, C₁-C₆-Alkylcarbonyl, C₁-C₆-Alkylaminocarbonyl, C₃-C₈-Cycloalkylaminocarbonyl, C₁-C₆-Alkylsulfonylaminocarbonyl, Phenoxy, 5- oder 6-gliedriges Heteroaryloxy, 5- bis 7-gliedriges Heterocyclyl, 5- oder 6-gliedriges Heteroaryl, 5- bis 7-gliedriges Heterocyclylcarbonyl und 5- oder 6-gliedriges Heteroarylcarbonyl,
worin Alkoxy seinerseits substituiert sein kann mit einem Substituenten, wobei der Substituent ausgewählt wird aus der Gruppe, bestehend aus Hydroxy, Phenyl, 5- bis 7-gliedriges Heterocyclyl und 5- oder 6-gliedriges Heteroaryl,
oder
- R²: für C₁-C₆ Alkylcarbonyl, gegebenenfalls einmal mit C₁-C₄-Alkoxy substituiertes C₁-C₆-Alkylaminocarbonyl oder C₃-C₈-Cycloalkylaminocarbonyl steht,
wobei Alkylcarbonyl substituiert sein kann mit einem Substituenten, wobei der Substituent ausgewählt wird aus der Gruppe, bestehend aus Amino, C₁-C₆-Alkylamino, C₃-C₈-Cycloalkylamino und 4- bis 7-gliedriges Heterocyclyl,
- R³: für Halogen, Cyano, Methoxy, Monofluormethoxy, Difluormethoxy, Trifluormethoxy oder Ethinyl steht,
- R⁴: für C₁-C₆-Alkyl oder C₃-C₈-Cycloalkyl steht,
wobei Alkyl substituiert sein kann mit 1 bis 3 Substituenten, wobei die Substituenten unabhängig voneinander ausgewählt werden aus der Gruppe, bestehend aus Halogen, Hydroxy, Amino, Cyano, Trifluormethyl, Hydroxycarbonyl, Aminocarbonyl, C₁-C₆-Alkoxy, C₁-C₆-Alkylamino, C₁-C₆-Aklcarbonyl und C₁-C₆-Alkoxycarbonyl,
und
wobei Cycloalkyl substituiert sein kann mit 1 bis 3 Substituenten, wobei die Substituenten unabhängig voneinander ausgewählt werden aus der Gruppe, bestehend aus Halogen, Hydroxy, Amino, Cyano, Trifluormethyl, Hydroxycarbonyl, Aminocarbonyl, C₁-C₆-Alkyl, C₁-C₆-Alkoxy, C₁-C₆-Alkylamino, C₁-C₆-Alkylcarbonyl und C₁-C₆-Alkoxycarbonyl,
- R⁵ und R⁶: unabhängig voneinander für Wasserstoff, Methyl oder Ethyl stehen,
- R⁷ und R⁸: unabhängig voneinander für Halogen, Hydroxy, Cyano, Trifluormethyl, Monofluormethoxy, Difluormethoxy, Trifluormethoxy, C₁-C₃-Alkyl oder C₁-C₃-Alkoxy stehen,
und ihre Salze, ihre Solvate und die Solvate ihrer Salze.

Bevorzugt sind auch solche Verbindungen der Formel (I), in welcher
- n: für eine Zahl 1 oder 2 steht,
- R¹: für Fluor, Chlor oder Trifluormethyl steht,
- R²: für Wasserstoff oder C₁-C₆-Alkyl steht,
wobei Alkyl substituiert sein kann mit 1 bis 2 Substituenten, wobei die Substituenten unabhängig voneinander ausgewählt werden aus der Gruppe, bestehend aus Hydroxy, Aminocarbonyl, C₁-C₆-Alkoxy, C₁-C₆-Alkylaminocarbonyl, Phenoxy, 5- oder 6-gliedriges Heteroaryloxy, 5- bis 7-gliedriges Heterocyclyl, 5- oder 6-gliedriges Heteroaryl, 5- bis 7-gliedriges Heterocyclylcarbonyl und 5- oder 6-gliedriges Heteroarylcarbonyl,
worin Alkoxy seinerseits substituiert sein kann mit einem Substituenten, wobei der Substituent ausgewählt wird aus der Gruppe, bestehend aus Hydroxy, Phenyl, 5-bis 7-gliedriges Heterocyclyl und 5- oder 6-gliedriges Heteroaryl,
- R³: für Halogen, Cyano, Methoxy, Monofluormethoxy, Difluormethoxy, Trifluormethoxy oder Ethinyl steht,
- R⁴: für C₁-C₆-Alkyl oder C₃-C₈-Cycloalkyl steht,
wobei Alkyl substituiert sein kann mit 1 bis 3 Substituenten, wobei die Substituenten unabhängig voneinander ausgewählt werden aus der Gruppe, bestehend aus Hydroxy, Amino, Cyano, Trifluormethyl, Hydroxycarbonyl, Aminocarbonyl, C₁-C₆-Alkoxy, C₁-C₆-Alkylamino, C₁-C₆-Alkylcarbonyl und C₁-C₆-Alkoxycarbonyl,
und
wobei Cycloalkyl substituiert sein kann mit 1 bis 3 Substituenten, wobei die Substituenten unabhängig voneinander ausgewählt werden aus der Gruppe, bestehend aus Halogen, Hydroxy, Amino, Cyano, Trifluormethyl, Hydroxycarbonyl, Aminocarbonyl, C₁-C₆-Alkyl, C₁-C₆-Alkoxy, C₁-C₆-Alkylamino, C₁-C₆-Alkylcarbonyl und C₁-C₆-Alkoxycarbonyl,
- R⁵ und R⁶: unabhängig voneinander für Wasserstoff, Methyl oder Ethyl stehen,
- R⁷ und R⁸: unabhängig voneinander für Halogen, Hydroxy, Cyano, Monofluormethoxy, Difluormethoxy, Trifluormethoxy, C₁-C₃-Alkyl oder C₁-C₃-Alkoxy stehen,
und ihre Salze, ihre Solvate und die Solvate ihrer Salze.

Bevorzugt sind auch solche Verbindungen der Formel (I), welche der Formel 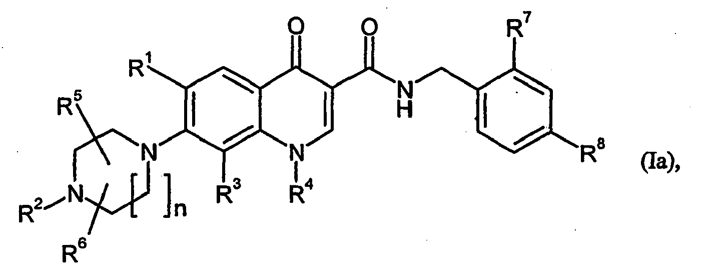 entsprechen,
in welcher
- n: für eine Zahl 1 oder 2 steht,
- R¹: für Fluor steht,
- R²: für Wasserstoff oder C₁-C₆-Alkyl steht,
wobei Alkyl substituiert sein kann mit 1 bis 2 Substituenten, wobei die Substituenten unabhängig voneinander ausgewählt werden aus der Gruppe, bestehend aus Hydroxy, C₁-C₆-Alkoxy, 5- oder 6-gliedriges Heterocyclyl und 5- oder 6-gliedriges Heterocyclylcarbonyl,
worin Alkoxy seinerseits substituiert sein kann mit einem Substituenten, wobei der Substituent ausgewählt wird aus der Gruppe, bestehend aus Hydroxy und 5- oder 6-gliedriges Heterocyclyl,
oder
- R²: für C₁-C₆-Alkylcarbonyl steht,
wobei Alkylcarbonyl substituiert ist mit einem Substituenten Amino,
- R³: für Fluor, Chlor, Methoxy, Monofluormethoxy, Difluormethoxy, Trifluormethoxy oder Ethinyl steht,
- R⁴: für C₁-C₄-Alkyl oder C₃-C₅-Cycloalkyl steht,
wobei Alkyl substituiert sein kann mit 1 bis 3 Substituenten, wobei die Substituenten unabhängig voneinander ausgewählt werden aus der Gruppe, bestehend aus Hydroxy und C₁-C₃-Alkoxy,
und
wobei Cycloalkyl substituiert sein kann mit 1 bis 3 Substituenten, wobei die Substituenten unabhängig voneinander ausgewählt werden aus der Gruppe, bestehend aus Halogen, Hydroxy, Trifluormethyl, C₁-C₃-Alkyl und C₁-C₃-Alkoxy,
- R⁵ und R⁶: unabhängig voneinander für Wasserstoff oder Methyl stehen,
- R⁷ und R⁸: unabhängig voneinander für Fluor, Chlor, Cyano, Trifluormethyl, Difluormethoxy, Trifluormethoxy, C₁-C₃-Alkyl oder C₁-C₃-Alkoxy stehen,
und ihre Salze, ihre Solvate und die Solvate ihrer Salze.

Bevorzugt sind auch solche Verbindungen der Formel (I), welche der Formel 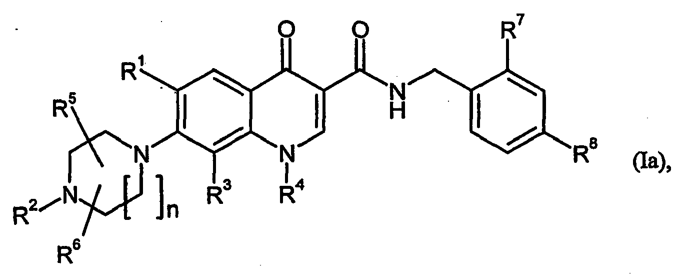 entsprechen,
in welcher
- n: für eine Zahl 1 oder 2 steht,
- R¹: für Fluor steht,
- R²: für Wasserstoff oder C₁-C₆-Alkyl steht,
wobei Alkyl substituiert sein kann mit 1 bis 2 Substituenten, wobei die Substituenten unabhängig voneinander ausgewählt werden aus der Gruppe, bestehend aus Hydroxy, C₁-C₆-Alkoxy, 5- oder 6-gliedriges Heterocyclyl und 5- oder 6-gliedriges Heterocyclylcarbonyl,
worin Alkoxy seinerseits substituiert sein kann mit einem Substituenten, wobei der Substituent ausgewählt wird aus der Gruppe, bestehend aus Hydroxy und 5- oder 6-gliedriges Heterocyclyl,
- R³: für Fluor, Chlor, Methoxy, Monofluormethoxy, Difluormethoxy, Trifluormethoxy oder Ethinyl steht,
- R⁴: für C₁-C₄-Alkyl oder C₃-C₅-Cycloalkyl steht,
wobei Alkyl substituiert sein kann mit 1 bis 3 Substituenten, wobei die Substituenten unabhängig voneinander ausgewählt werden aus der Gruppe, bestehend aus Hydroxy und C₁-C₃-Alkoxy,
und
wobei Cycloalkyl substituiert sein kann mit 1 bis 3 Substituenten, wobei die Substituenten unabhängig voneinander ausgewählt werden aus der Gruppe, bestehend aus Halogen, Hydroxy, Trifluormethyl, C₁-C₃-Alkyl und C₁-C₃-Alkoxy,
- R⁵ und R⁶: unabhängig voneinander für Wasserstoff oder Methyl stehen,
- R⁷ und R⁸: unabhängig voneinander für Fluor, Chlor, Cyano, Difluormethoxy, Trifluormethoxy, C₁-C₃-Alkyl oder C₁-C₃-Alkoxy stehen,
und ihre Salze, ihre Solvate und die Solvate ihrer Salze.

Bevorzugt sind besonders solche Verbindungen der Formel (I) oder (Ia), welche der Formel 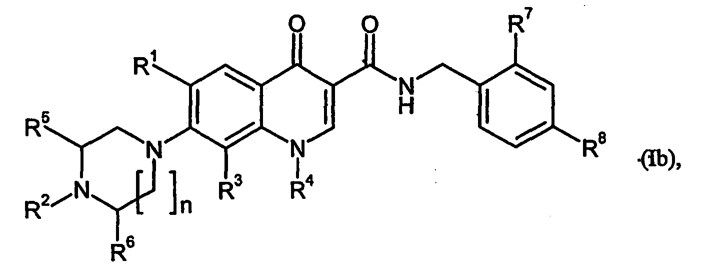 entsprechen,
in welcher
- n: für eine Zahl 1 oder 2 steht,
- R¹: für Fluor steht,
- R²: für Wasserstoff oder C₁-C₃-Alkyl steht,
wobei Alkyl substituiert sein kann mit 1 bis 2 Substituenten, wobei die Substituenten unabhängig voneinander ausgewählt werden aus der Gruppe, bestehend aus Hydroxy, Morpholin-2-ylcarbonyl, Morpholin-3-ylcarbonyl, Morpholin-4-ylcarbonyl, Piperidin-1-ylcarbonyl, Piperidin-2-ylcarbonyl, Piperidin-3-ylcarbonyl, Piperidin-4-ylcarbonyl, Pyrrolidin-2-ylcarbonyl, Pyrrolidin-3-ylcarbonyl und gegebenenfalls mit einem Substituenten Hydroxy substituiertes C₁-C₃-Alkoxy,
oder
- R²: für C₁-C₄-Alkylcarbonyl steht,
wobei Alkylcarbonyl substituiert ist mit einem Substituenten Amino,
- R³: für Chlor, Methoxy, Difluormethoxy oder Trifluormethoxy steht,
- R⁴: für Methyl, Ethyl oder Cyclopropyl steht,
wobei Ethyl substituiert sein kann mit 1 bis 3 Substituenten Fluor,
und
wobei Cyclopropyl substituiert sein kann mit 1 bis 2 Substituenten Fluor,
- R⁵ und R⁶: unabhängig voneinander für Wasserstoff oder Methyl stehen,
- R⁷ und R⁸: unabhängig voneinander für Chlor, Trifluormethyl, Trifluormethoxy oder Methyl stehen,
und ihre Salze, ihre Solvate und die Solvate ihrer Salze.

Bevorzugt sind besonders solche Verbindungen der Formel (I) oder (Ia), welche der Formel 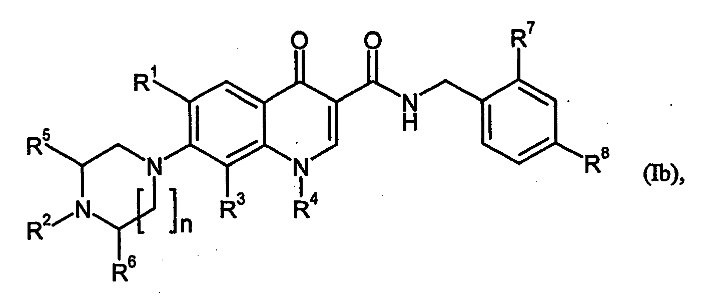 entsprechen,
in welcher
- n: für eine Zahl 1 oder 2 steht,
- R¹: für Fluor steht,
- R²: für Wasserstoff oder C₁-C₃-Alkyl steht,
wobei Alkyl substituiert sein kann mit 1 bis 2 Substituenten, wobei die Substituenten unabhängig voneinander ausgewählt werden aus der Gruppe, bestehend aus Hydroxy, Morpholin-2-ylcarbonyl, Morpholin-3-ylcarbonyl, Morpholin-4-ylcarbonyl, Piperidin-1-ylcarbonyl, Piperidin-2-ylcarbonyl, Piperidin-3-ylcarbonyl, Piperidin-4-ylcarbonyl, Pyrrolidin-2-ylcarbonyl, Pyrrolidin-3-ylcarbonyl und gegebenenfalls mit einem Substituenten Hydroxy substituiertes C₁-C₃-Alkoxy,
- R³: für Chlor, Methoxy, Difluormethoxy oder Trifluormethoxy steht,
- R⁴: für Methyl, Ethyl oder Cyclopropyl steht,
wobei Cyclopropyl substituiert sein kann mit 1 bis 2 Substituenten Fluor,
- R⁵ und R⁶: unabhängig voneinander für Wasserstoff oder Methyl stehen,
- R⁷ und R⁸: unabhängig voneinander für Chlor oder Methyl stehen,
und ihre Salze, ihre Solvate und die Solvate ihrer Salze.

Bevorzugt sind auch solche Verbindungen der Formel (Ic), (I), (Ia) und (Ib), in welcher R¹ für Fluor steht.

Bevorzugt sind auch solche Verbindungen der Formel (Ic), (I), (Ia) und (Ib), in welcher R³ für Chlor, Methoxy oder Difluormethoxy steht.

Bevorzugt sind auch solche Verbindungen der Formel (Ic), (I), (Ia) und (Ib), in welcher R⁴ für Cyclopropyl oder 2-Fluor-cycloprop-1-yl steht.

Bevorzugt sind auch solche Verbindungen der Formel (Ic), (I), (Ia) und (Ib), in welcher R⁴ für 2,2,2-Trifluorethyl steht.

Bevorzugt sind auch solche Verbindungen der Formel (Ic), (I), (Ia) und (Ib), in welcher R⁷ und R⁸ für Chlor stehen.

Bevorzugt sind auch solche Verbindungen der Formel (Ic), (I), (Ia) und (Ib), in welcher R⁷ für Chlor oder Methyl und R⁸ für Trifluormethoxy steht.

Bevorzugt sind auch solche Verbindungen der Formel (Ic), (I), (Ia) und (Ib), in welcher R² für Wasserstoff, Aminomethylcarbonyl oder -2,3-Dihydroxyprop-1-yl und R⁵ und R⁶ für Methyl stehen.

Bevorzugt sind auch solche Verbindungen der Formel (Ic), (I), (Ia) und (Ib), in welcher R² für Wasserstoff und R⁵ und R⁶ für Methyl stehen.

Bevorzugt sind auch solche Verbindungen der Formel (Ic), in welcher R⁹ für Wasserstoff steht.

Die in den jeweiligen Kombinationen bzw. bevorzugten Kombinationen von Resten im einzelnen angegebenen Restedefinitionen werden unabhängig von den jeweiligen angegebenen Kombinationen der Reste beliebig auch durch Restedefinitionen anderer Kombination ersetzt.

Ganz besonders bevorzugt sind Kombinationen von zwei oder mehreren der oben genannten Vorzugsbereiche.

Gegenstand der Erfindung ist weiterhin ein Verfahren zur Herstellung der Verbindungen der Formel (Ic), wobei Verbindungen der Formel 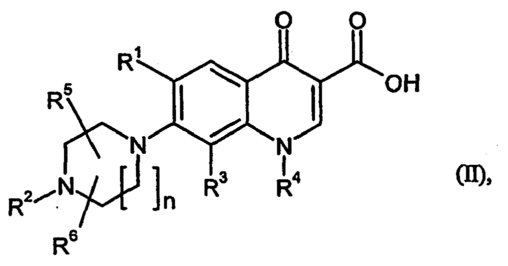 in welcher
n, R¹, R², R³, R⁴, R⁵ und R⁶ die oben angegebene Bedeutung aufweisen,
mit Verbindungen der Formel 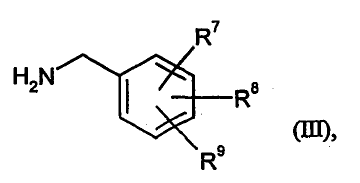 in welcher
R⁷, R⁸ und R⁹ die oben angegebene Bedeutung aufweisen,
umgesetzt werden.

Die Umsetzung erfolgt im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, *N*-Methylpiperidin, 4-Dimethylaminopyridin oder Diisopropylethylamin.

Als Dehydratisierungsreagenzien eignen sich hierbei beispielsweise Carbodiimide wie z.B. *N,N'-*Diethyl-, *N,N,*'-Dipropyl-, N,N'-Diisopropyl-, *N*,*N*'-Dicyclohexylcarbodiimid, N-(3-Dimethylaminoisopropyl)-*N*'-ethylcarbodiimid-hydrochlorid (EDC), *N*-Cyclohexylcarbodiimid-*N*'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert-*Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid, oder *O*-(Benzotriazol-1-yl)-*N*,*N*,*N'*;*N'*-tetramethyluroniumhexafluorophosphat (HBTU), 2-(2-Oxo-1-(2H)-pyridyl)-1,1,3,3 tetramethyluroniumtetrafluoroborat (TPTU) oder *O*-(7-Azabenzotriazol-1-yl)-*N*,*N*,*N*,*N'*-tetramethyl-uroniumhexafluorophosphat (HATU), oder 1-Hydroxybenztriazol (HOBt), oder Benzotriazol-1-yloxytris(dimethylamino)-phosphoniumhexafluorophosphat (BOP), oder Benzotriazol-1-yloxytris(pyrrolidino)-phosphoniumhexafluorophosphat (PyBOP), oder *N*-Hydroxysuccinimid, oder Mischungen aus diesen, mit Basen.

Vorzugsweise wird die Kondensation mit HATU, Benzotriazol-1-yloxytris(pyrrolidino)-phosphoniumhexafluorophosphat (PyBOP) öder mit EDC in Gegenwart von HOBt durchgeführt

Die Verbindungen der Formel (II) sind bekannt oder können hergestellt werden, indem Verbindungen der Formel 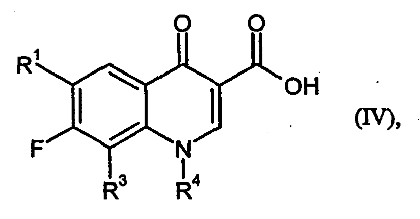 in welcher
R¹, R³ und R⁴ die oben angegebene Bedeutung aufweisen,
mit Verbindungen der Formel 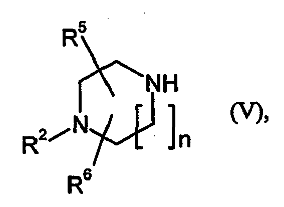 in welcher
n, R², R⁵ und R⁶ die oben angegebene Bedeutung aufweisen,
umgesetzt werden.

Die Umsetzung kann nach den in A. Da Silva, M. De Almeida, V. De Souza, M. Couri, Current Medicinal Chemistry, 2003, 10, 21-39 oder J. P. Sanchez, et al., Journal of Medicinal Chemistry 1995, 38(22); 4478-87 beschriebenen Verfahren durchgeführt werden.

Die Verbindungen der Formel (III) und (V) sind bekannt oder lassen sich nach bekannten Verfahren aus den entsprechenden Edukten synthetisieren.

Die Verbindungen der Formel (V) tragen gegebenenfalls während der Umsetzung dem Fachmann bekannte Schutzgruppen, die entweder direkt nach der Umsetzung von Verbindungen der Formel (IV) mit Verbindungen der Formel (V) zu Verbindungen der Formel (II) oder nach der weiteren Umsetzung zu Verbindungen der Formel (Ic) abgespalten werden.

Die Verbindungen der Formel (IV) sind bekannt oder lassen sich nach bekannten Verfahren aus den entsprechenden Edukten synthetisieren, wie z.B. in A. Da Silva, M. De Almeida, V. De Souza, M. Couri, Current Medicinal Chemistry, 2003, 10, 21-39 beschrieben.

In einem alternativen Verfahren lässt sich der Substituent R² in Verbindungen der Formel (Ic) durch Umsetzung von Verbindungen der Formel 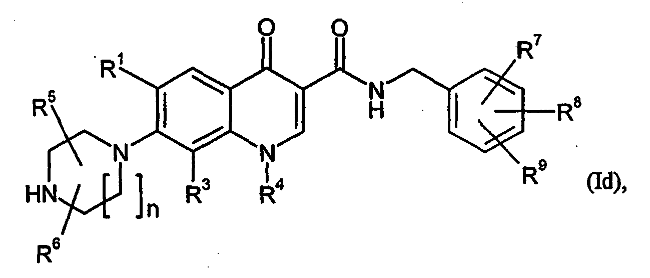 in welcher
n, R¹, R³, R⁴, R⁵, R⁶, R⁷, R⁸ und R⁹ die oben angegebene Bedeutung aufweisen,
mit Elektrophilen, wie z. B. Carbonsäurechloriden, gegebenenfalls substituiertem Chloracetamid, gegebenenfalls substituiertem 2-Chlorpropionsäureamid, Chlormethylketonen oder gegebenenfalls substituiertem 3-Brompropionsäureamid, in Gegenwart einer Base oder durch Umsetzung mit Isocyanaten, Michael-Akzeptoren oder Epoxiden einführen.

In einem alternativen Verfahren können zur Herstellung der Verbindungen der Formel (Ic) die nukleophilische Substitution an der 7-Position des Chinolons und die Amidbildung in der Reihenfolge der Umsetzung vertauscht werden.

Die Herstellung der erfindungsgemäßen Verbindungen kann durch das folgende Syntheseschema verdeutlicht werden.

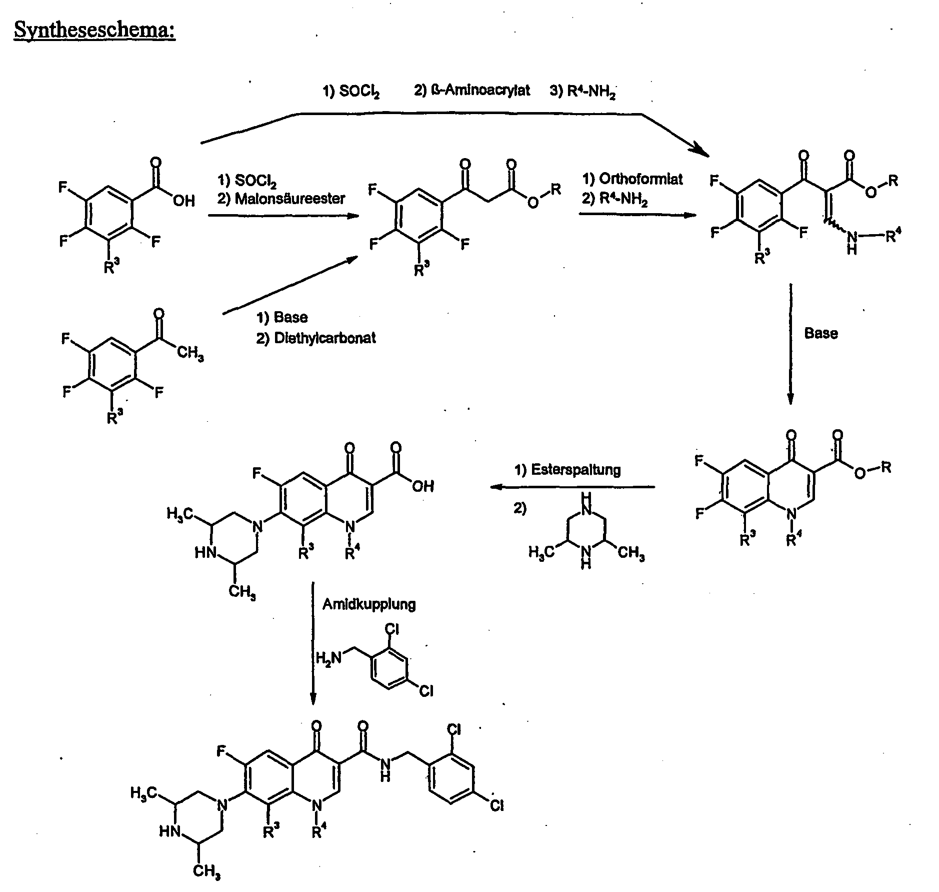

Die erfindungsgemäßen Verbindungen zeigen ein nicht vorhersehbares, überraschendes Wirkspektrum. Sie zeigen eine antivirale Wirkung gegenüber Vertretern der Gruppe der Herpes viridae (Herpesviren), vor allem gegenüber Cytomegaloviren (CMV), insbesondere gegenüber dem humanen Cytomegalovirus (HCMV).

Als Indikationsgebiete können beispielsweise genannt werden:
1) Behandlung und Prophylaxe von HCMV-Infektionen bei AIDS-Patienten (Retinitis, Pneumonitis, gastrointestinale Infektionen).
2) Behandlung und Prophylaxe von Cytomegalovirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.
3) Behandlung und Prophylaxe von HCMV-Infektionen bei Neugeborenen und Kleinkindern.
4) Behandlung einer akuten HCMV-Infektion bei Schwangeren.
5) Behandlung der HCMV-Infektion bei immunsupprimierten Patienten bei Krebs und KrebsTherapie.
6) Behandlung von HCMV-positiven Krebspatienten mit dem Ziel, HCMV-vermittelte Tumorprogression zu verringern (vgl. J. Cinatl, et al., FEMS Microbiology Reviews 2004, 28, 59-77).

Weiterer Gegenstand der vorliegenden Erfindung ist der Einsatz der erfindungsgemäßen Verbindungen zur Behandlung und/oder Prophylaxe von Krankheiten, vor allem von Infektionen mit Viren, insbesondere den vorstehend genannten Viren, und den dadurch hervorgerufenen Infektionskrankheiten. Unter einer Virusinfektion wird nachfolgend sowohl eine Infektion mit einem Virus als auch eine durch eine Infektion mit einem Virus hervorgerufene Krankheit verstanden.

Weiterer Gegenstand der vorliegenden Erfindung ist die Verwendung der erfindungsgemäßen Verbindungen zur Herstellung eines Arzneimittels Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Bevorzugt werden die erfindungsgemäßen Verbindungen zur Herstellung von Arzneimitteln verwendet, die zur Prophylaxe und/oder Behandlung von Infektionen mit einem Vertreter der Gruppe der Herpes viridae, besonders einem Cytomegalovirus, insbesondere dem humanen Cytomegalovirus, geeignet sind.

Weiterer Gegenstand der vorliegenden offenbarung ist ein Verfahren zur Behandlung und/oder Prophylaxe von Erkrankungen, insbesondere der zuvor genannten Erkrankungen, unter Verwendung einer antiviral wirksamen Menge der erfindungsgemäßen Verbindungen.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt: antivirale Wirkstoffe wie Valgancyclovir, Gancyclovir oder Acyclovir.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch, topisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nicht überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme, Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei intravenöser Applikation Mengen von etwa 0.001 bis 10 mg/kg, vorzugsweise etwa 0.01 bis 5 mg/kg Körpergewicht zur Erzielung wirksamer Ergebnisse zu verabreichen, und bei oraler Applikation beträgt die Dosierung etwa 0.01 bis 25 mg/kg, vorzugsweise 0.1 bis 10 mg/kg Körpergewicht

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- ca.: circa
- BINAP: 2,2'-Bis(diphenylphosphino)-1,1'-binaphthyl
- CDCl₃: Deuterochloroform
- DC: Dünnschichtchromatographie
- DCI: direkte chemische Ionisation (bei MS)
- DCM: Dichlormethan
- DIEA: *N,N*-Diisopropylethylamin
- DMSO: Dimethylsulfoxid
- DMF: *N,N-*Dimethylformamid
- d. Th.: der Theorie
- EE: Ethylacetat (Essigsäureethylester)
- EI: Elektronenstoß-Ionisation (bei MS)
- ESI: Elektrospray-Ionisation (bei MS)
- Fp.: Schmelzpunkt
- ges.: gesättigt
- ggfs.: gegebenenfalls
- H: Stunde
- HPLC: Hochdruck-, Hochleistungsflüssigchromatographie
- konz.: konzentriert
- LC-MS: Flüssigchromatographie-gekoppelte Massenspektroskopie
- LDA: Lithium-Diisopropylamid
- min: Minuten
- MS: Massenspektroskopie
- MTBE: Methyl-*tert*-butylether
- NMR: Kernresonanzspektroskopie
- Pd-C: Palladium auf Kohle
- proz.: prozentig
- PyBOP: 1-Benzotriazolyloxy-tripyrrolidinophosphoniumhexafluorophosphat
- RP-HPLC: Reverse Phase HPLC
- RT: Raumtemperatur
- Rₜ: Retentionszeit (bei HPLC)
- THF: Tetrahydrofuran

### Allgemeine Methoden LC-MS und HPLC:

**Methode 1 (LC-MS):** Instrument: Micromass Quattro LCZ mit HPLC Agilent Serie 1100; Säule: Phenomenex Synergi 2µ Hydro-RP Mercury 20 mm x 4 mm; Eluent A: 11 Wasser + 0.5 ml 50%ige Ameisensäure, Eluent B: 11 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 208- 400 nm.

**Methode 2 (LC-MS):** Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 11 Wasser + 0.5 ml 50%ige Ameisensäure, Eluent B: 11 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

**Methode 3 (LC-MS):** Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 11 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

**Methode 4 (präparative HPLC):** Säule: RP18; Gradient unter Zusatz von 0.2% Diethylamin zum Acetonitril: 30% Acetonitril / 70% Wasser → 95% Acetonitril / 5% Wasser.

**Methode 5 (präparative HPLC, Ameisensäure):** Säule: Grom-Sil 120 ODS-4HE, 10 µm, SNr. 3331, 250 mm x 30 mm. Eluent A: Ameisensäure 0.1% in Wasser, Eluent B: Acetonitril; Fluss: 50 ml/min. Programm: 0-3 min: 10% B; 3-27 min: Gradient bis 95% B; 27-34 min: 95% B; 34.01-3.8 min: 10% B.

**Methode 6 (präparative HPLC, Salzsäure):** Säule: Grom-Sil 120 ODS-4HE, 10 µm, SNr. 3331, 250 mm x 30 mm. Eluent A: Salzsäure 0.1% in Wasser, Eluent B: Acetonitril; Fluss: 50 ml/min. Programm: 0-2 min 10% B, 3-43 min: Gradient bis 100% B, 43.01-45 min: 100% B.

**Methode 7 (präparative HPLC):** Säule: Grom-Sil 120 ODS-4HE, 10 µm, SNr. 3331, 250 mm x 30 mm. Eluent A: Wasser, Eluent B: Acetonitril, Fluss: 50 ml/min. Programm: 0-3 min: 10% B; 3-27 min: Gradient bis 95% B; 27-34 min: 95% B; 34.01-38 min: 10% B.

**Methode 8 (präparative HPLC, Trifluoressigsäure):** Säule: Grom-Sil 120 ODS-4HE, 10 µm, SNr. 3331, 250 mm x 30 mm. Eluent A: Trifluoressigsäure 0.1% in Wasser, Eluent B: Acetonitril.

Fluss: 50 ml/min. Programm: 0-3 min: 10% B; 3-27 min: Gradient bis 95% B; 27-34 min: 95% B; 34.01-38 min: 10% B.

**Methode 9 (analytische HPLC):** Instrument: HP 1100 mit DAD-Detektion; Säule: Kromasil 100 RP-18, 60 mm x 2.1 mm, 3.5 µm; Eluent A: 5 ml Perchlorsäure (70%ig) /1 Wasser, Eluent B: Acetonitril; Gradient: 0 min 2%B, 0.5 min 2%B, 4.5 min 90%B, 9 min 90%B, 9.2 min 2%B, 10 min 2%B; Fluss: 0.75 ml/min; Säulen-Temperatur: 30°C; UV-Detektion: 210 nm.

**Methode 10 (analytische HPLC):** Instrument: HP 1100 mit DAD-Detektion; Säule: Kromasil 100 RP-18, 60 mm x 2.1 mm, 3.5 µm; Eluent A: 5 ml Perchlorsäure (70%ig) /1 Wasser, Eluent B: Acetonitril; Gradient: 0 min 2%B, 0.5 min 2%B, 4.5 min 90%B, 6.5 min 90%B, 6.7 min 2%B, 7.5 min 2%B; Fluss: 0.75 ml/min; Säulen-Temperatur: 30°C; UV-Detektion: 210 nm.

**Methode 11 (LC-MS):** Instrument MS: Micromass TOF (LCT); Instrument HPLC: 2-Säulen-Schaltung, Waters2690; Säule: YMC-ODS-AQ, 50 mm x 4.6 mm, 3.0 µm; Eluent A: Wasser + 0.1 % Ameisensäure, Eluent B: Acetonitril + 0.1 % Ameisensäure; Gradient: 0.0 min 100%A → 0.2 min 95%A → 1.8 min 25%A → 1.9 min 10%A → 2.0 min 5%A → 3.2 min 5%A; Ofen: 40°C; Fluss: 3.0 ml/min; UV-Detektion: 210 nm.

**Methode 12 (präparatives LC-MS):** Instrument MS: Micromass Micromass ZMD; Instrument HPLC: Waters Prep LC 4000; Säule: Kromasil, 50 mm x 20 mm, 100Å, C18 5 µm; Eluent A: Wasser + 0.1% Ameisensäure, Eluent B: Acetonitril + 0.1% Ameisensäure; Gradient: 0.0 min 70%A → 0.75 min 70%A → 5.5 min 100%B → 6.5 min 100%B → 7.0 min 70%A → Fluss: 40.0 ml/min.

**Methode 13 (LC-MS):** Instrument: Micromass Platform LCZ mit HPLC Agilent Serie 1100; Säule: Thermo Hypersil GOLD 3µ 20 mm x 4 mm; Eluent A: 1 1 Wasser + 0.5 ml 50%ige Ameisensäure, Eluent B: 11 Acetonitril + 0.5 ml 50%ige Ameisensäure; Gradient: 0.0 min 100%A → 0.2 min 100%A → 2.9 min 30%A → 3.1 min 10%A → 5.5 min 10%A; Ofen: 50°C; Fluss: 0.8 ml/min; UV-Detektion: 210 nm.

Die Beispiel-Verbindungen, die einen basischen Stickstoff enthalten, können abhängig von der Reinigungsmethode als freie Base oder in verschiedenen Salzformen isoliert werden. Die Herstellungsmethode beschreibt oft die Reinigung durch HPLC unter Ameisensäure-Zusatz (Methode 5), was zum Hydroformiat führt oder unter Zusatz von anderen Säuren, wie z. B. Salzsäure (Methode 6) statt Ameisensäure, wobei das Produkt als Hydrochlorid isoliert wird. Alternativ kann das Produkt auch durch Verrühren in Acetonitril oder durch präparative HPLC ohne Säure-Zusatz (Methode 7) gereinigt werden, wobei das Produkt als freie Base isoliert wird.

Sowohl aus der freien Base wie auch aus dem Hydroformiat kann durch anschließende Versetzung mit Chlorwasserstoff in Dioxan und Abdampfen am Rotationsverdampfer wiederum das Hydrochlorid der Verbindung erhalten werden.

### Ausgangsverbindungen

### Beispiel 1A

### 8-Chlor-1-cyclopropyl-6-fluor-7-[4-(2-hydroxyethyl)-1,4-diazepan-1-yl]-4-oxo-1,4-dihydrochinolin-3-carbonsäure

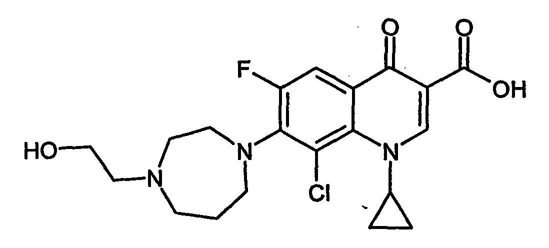

350 mg (1.2 mmol) 8-Chlor-1-cyclopropyl-6,7-difluor-4-oxo-1,4-dihydrochinolin-3-carbonsäure (Herstellung siehe: DE 3420743) werden gemäß DE 3635218 in 3 ml trockenem Pyridin gelöst und mit 202 mg (1.4 mmol) Hexahydro-1H-1,4-diazepin-1-ethanol 4 Stunden zum Rückfluss erhitzt. Nach Stehen über Nacht wird eingeengt, mit Wasser aufgenommen und mit verdünnter Salzsäure auf pH 6 gebracht. Die Lösung wird in der Siedehitze mit Kochsalz gesättigt. Nach Abkühlen auf Raumtemperatur wird mehrmals mit Dichlormethan extrahiert. Die organischen Extrakte werden über wenig Kieselgel filtriert und eingeengt Man erhält so 288 mg Zielverbindung. Die Verbindung wird als Rohprodukt in die nächsten Reaktionsstufen eingesetzt.
LC-MS (Methode 3): Rₜ =1.32 min,
MS (ES+) = 424 (M+H)⁺

Analog zur Herstellungsvorschrift von Beispiel 1A werden die Beispiele 2A bis 12A hergestellt:

### Beispiel 2A

### 8-Chlor-1-cyclopropyl-6-fluor-7-{4-[2-(2-hydroxyethoxy)ethyl]piperazin-1-yl}-4-oxo-1,4-dihydrochinolin-3-carbonsäure

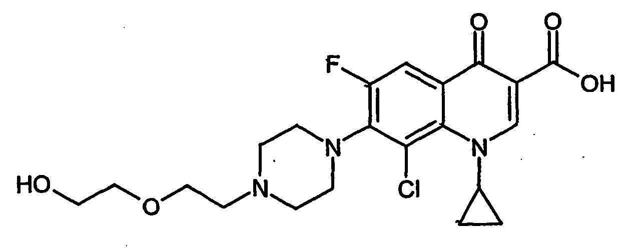

Die Herstellung erfolgt analog zu Beispiel 1A aus 8-Chlor-l-cyclopropyl-6,7-difluor-4-oxo-1,4-dihydrochinolin-3-carbonsäure (Herstellung siehe: DE 3420743).
LC-MS (Methode 2): Rₜ = 1.08 min,
MS (ES+) = 454 (M+H)⁺

### Beispiel 3A

### 8-Chlor-6-fluor-1-[(1R,2S)-2-fluorcyclopropyl]-7-{4-[2-(2-hydroxyethoxy)ethyl]piperazin-1-yl}-4-oxo-1,4-dihydrochinolin-3-carbonsäure

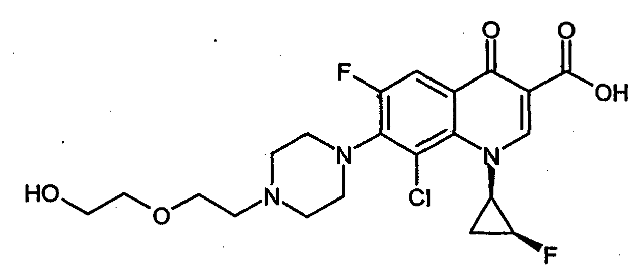

Die Herstellung erfolgt analog zu Beispiel 1A aus 8-Chlor-6,7-difluor-1-[(1R,2S)-2-fluorcyclopropyl]-4-oxo-1,4-dihydrochinolin-3-carbonsäure (Herstellung siehe: Journal of Medicinal Chemistry (1994), 37(20), 3344-52).
LC-MS (Methode 2): Rₜ = 1.28 min,
MS (ES+) = 472 (M+H)⁺

### Beispiel 4A

### 8-Chlor-6-fluor-1-[(1S,2R)-2-fluorcyclopropyl]-7-{4-[2-(2-hydroxyethoxy)ethyl]piperazin-1-yl}-4-oxo-1,4-dihydrochinolin-3-carbonsäure

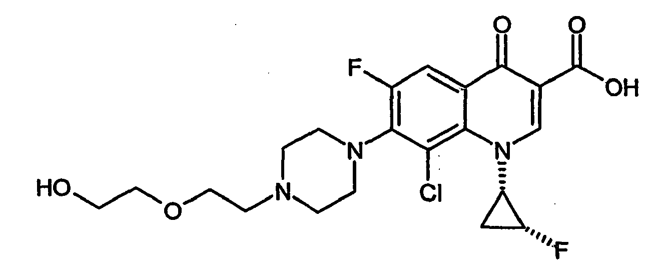

Die Herstellung erfolgt analog zu Beispiel 1A aus 8-Chlor-6,7-difluor-1-[(1S,2R)-2-fluorcyclopropyl]-4-oxo-1,4-dihydrochinolin-3-carbonsäure (Herstellung siehe: Journal of Medicinal Chemistry (1994), 37(20), 3344-52). Die Verbindung wird als Rohprodukt in die nächsten Reaktionsstufen eingesetzt.
LC-MS (Methode 3): Rₜ =1.82 min,
MS (ES+) = 472 (M+H)⁺

### Beispiel 5A

### 8-Chlor-6-fluor-1-[(1S,2R)-2-fluorcyclopropyl]-7-[4-(2-morpholin-4-yl-2-oxoethyl)piperazin-1-yl]-4-oxo-1,4-dihydrochinolin-3-carbonsäure

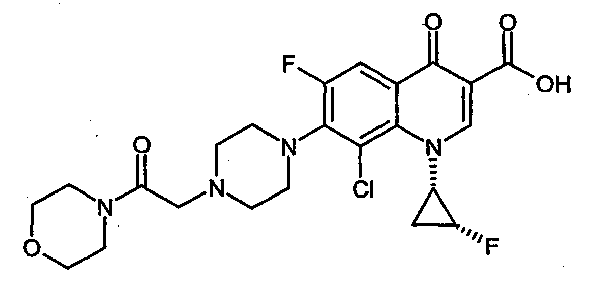

Die Herstellung erfolgt analog zu Beispiel 1A aus 4-[2-(Piperazin-1-yl)acetyl]morpholin und 8-Chlor-6,7-difluor-l-[(1S,2R)-2-fluorcyclopropyl]-4-oxo-1,4-dihydrochinolin-3-carbonsäure (Herstellung siehe: Journal of Medicinal Chemistry (1994), 37(20), 3344-52). Die Verbindung wird als Rohprodukt in die nächsten Reaktionsstufen eingesetzt.
LC-MS (Methode 1): Rₜ = 1.37 min,
MS (ES+) = 511 (M+H)⁺

### Beispiel 6A

### 6-Fluor-1-[(1R,2S)-2-fluorcyclopropyl]-7-{4-[2-(2-hydroxyethoxy)ethyl]piperazin-1-yl}-8-methoxy-4-oxo-1,4-dihydrochinolin-3-carbonsäure

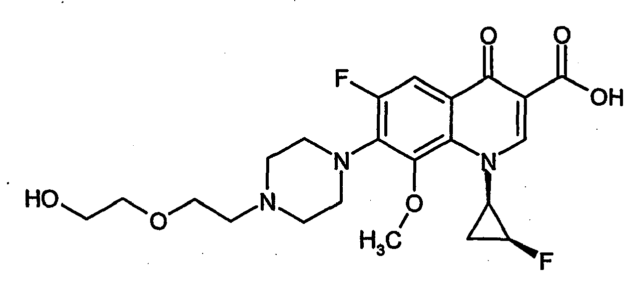

Die Herstellung erfolgt analog zu Beispiel 1A aus 6,7-Difluor-1-[(1R,2S)-2-fluorcyclopropyl]-8-methoxy-4-oxo-1,4-dihydrochinolin-3-carbonsäure (Herstellung siehe: WO 96/01262). Die Verbindung wird als Rohprodukt in die nächsten Reaktionsstufen eingesetzt.
LC-MS (Methode 3): Rₜ =1.25 min,
MS (ES+) = 468 (M+H)⁺

### Beispiel 7A

### 8-Chlor-6-fluor-1-[(1SR,2RS)-2-fluorcyclopropyl]-7-{4-[2-(2-hydroxyethyl)piperazin-1-yl]-4-oxo-1,4-dihydrochinolin-3-carbonsäure

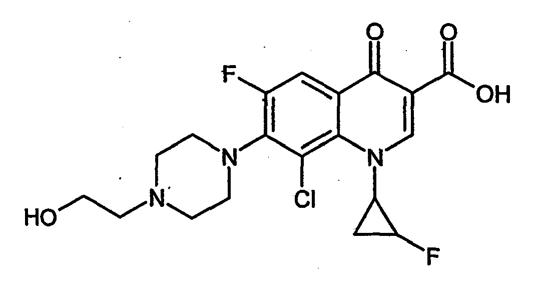

Die Herstellung erfolgt analog zu Beispiel 1A aus racemischer 8-Chlor-6,7-difluor-1-[cis-2-fluorcyclopropyl]4-oxo-1,4-dihydrochinolin-3-carbonsäure (Herstellung analog siehe: Journal of Medicinal Chemistry (1994), 37(20), 3344-52). Die Verbindung wird als Rohprodukt in die nächsten Reaktionsstufen eingesetzt.
LC-MS (Methode 1): Rₜ = 1.24 min,
MS (ES+) = 428 (M+H)⁺

### Beispiel 8A

### 8-Chlor-6-fluor-1-[(1SR,2RS)-2-fluorcyclopropyl]-7-(4-methylpiperazin-1-yl)-4-oxo-1,4-dihydrochinolin-3-carbonsäure

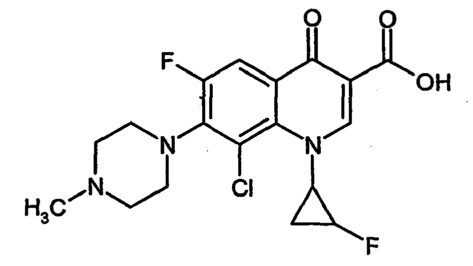

Die Herstellung erfolgt analog zu Beispiel 1A aus racemischer 8-Chlor-6,7-difluor-1-[cis-2-fluorcyclopropyl]-4-oxo-1,4-dihydrochinolin-3-carbonsäure (Herstellung analog siehe: Journal of Medicinal Chemistry (1994), 37(20), 3344-52). Die Verbindung wird als Rohprodukt in die nächsten Reaktionsstufen eingesetzt.
LC-MS (Methode 2): Rₜ = 1.01 min,
MS (ES+) = 398 (M+H)⁺

### Beispiel 9A

### 8-Chlor-6-fluor-1-[cis-2-fluorcyclopropyl]-7-{4-[2-(2-hydroxyethoxy)ethyl]piperazin-1-yl}-4-oxo-1,4-dihydrochinolin-3-carbonsäure

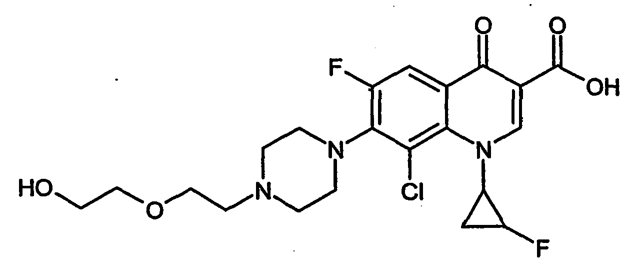

Die Herstellung erfolgt analog zu Beispiel 1A aus racemischer 8-Chlor-6,7-difluor-1-[cis-2-fluorcyclopropyl]-4-oxo-1,4-dihydrochinolin-3-carbonsäure (Herstellung analog siehe: Journal of Medicinal Chemistry (1994), 37(20), 3344-52). Die Verbindung wird als Rohprodukt in die nächsten Reaktionsstufen eingesetzt.
LC-MS (Methode 2): Rₜ = 0.99 min,
MS (ES+) = 472 (M+H)⁺

### Beispiel 10A

### 1-Cyclopropyl-6-fluor-7-{4-[2-(2-hydroxyethoxy)ethyl]piperazin-1-yl}-8-methoxy-4-oxo-1,4-dihydrochinolin-3-carbonsäure

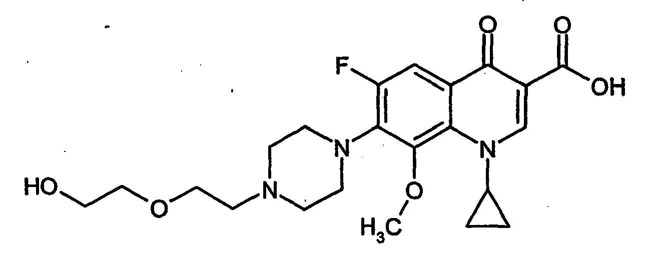

Die Herstellung erfolgt analog zu Beispiel 1A aus (T-4)-(1-Cyclopropyl-6,7-difluor-1,4-dihydro-8-methoxy-4-oxo-3-chinolincarboxylato-O3,O4)bordifluorid (Herstellung siehe: Journal of Medicinal Chemistry (1995), 38(22), 4478-87). Die Verbindung wird als Rohprodukt in die nächsten Reaktionsstufen eingesetzt.
LC-MS (Methode 2): Rₜ = 0.95 min,
MS (ES+) = 450 (M+H)⁺

### Beispiel 11A

### 7-[(3RS,5SR)-3,5-Dimethylpiperazin-1-yl]-6-fluor-1-[(1R,2S)-2-fluorcyclopropyl]-8-methoxy-4-oxo-1,4-dihydrochinolin-3-carbonsäure

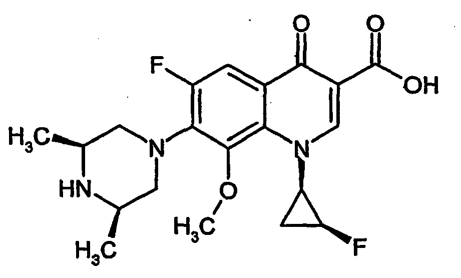

Die Herstellung erfolgt analog zu Beispiel 1A aus 6,7-Difluor-1-[(1R,2S)-2-fluorcyclopropyl]-8-methoxy-4-oxo-1,4-dihydrochinolin-3-carbonsäure (Herstellung siehe: WO 96/01262).
LC-MS (Methode 3): Rₜ = 1.38 min,
MS (ES+) = 408 (M+H)⁺

### Beispiel 12A

### 1-Cyclopropyl-8-difluormethoxy-6-fluor-7-{4-[2-(2-hydroxyethoxy)ethyl]piperazin-1-yl}-4-oxo-1,4-dihydrochinolin-3-carbonsäure

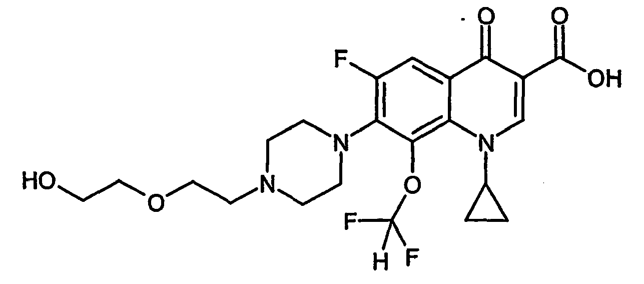

Die Herstellung erfolgt analog zu Beispiel 1A aus (T-4)-(1-Cyclopropyl-8-difluormethoxy-6,7-difluor-1,4-dihydro-4-oxo-3-chinolincarboxylato-03,04)bordifluorid (Herstellung siehe: EP 352123). Die Verbindung wird als Rohprodukt in die nächsten Reaktionsstufen eingesetzt.

### Beispiel 13A

### 1-Cyclopropyl-6-fluor-7-{4-[2-methoxy-ethyl]piperazin-1-yl}-8-methoxy-4-oxo-1,4-dihydrochinolin-3-carbonsäure

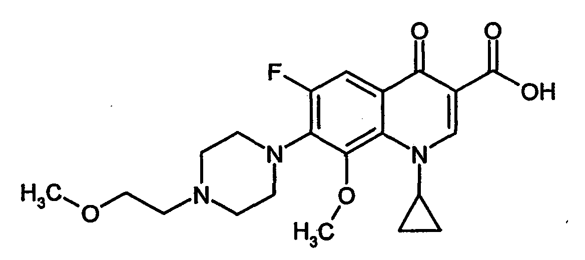

Die Herstellung erfolgt analog zu Beispiel 1A aus (T-4)-(1-Cyclopropyl-6,7-difluor-1,4-dihydro-8-methoxy-4-oxo-3-chinolincarboxylato-03,04)bordifluorid (Herstellung siehe: Journal of Medicinal Chemistry (1995), 38(22), 4478-87). Die Verbindung wird als Rohprodukt in die nächsten Reaktionsstufen eingesetzt.
¹H-NMR (300 MHz, DMSO-d₆): δ = 1.05 (m, 2H),1.13 (m, 2H), 3.10-3.90 (m, 18 H: darin 3.82 (s, 3H)), 4.18(m, 1H), 7.82 (d, 1H), 8.73 (s, 1H), 10.78 (bs, 1H).

### Beispiel 14A

### 3-[(2,2,2-Trifluorethyl)amino]-2-(2,4,5-trifluor-3-methoxybenzoyl)acrylsäureethylester (E + Z)

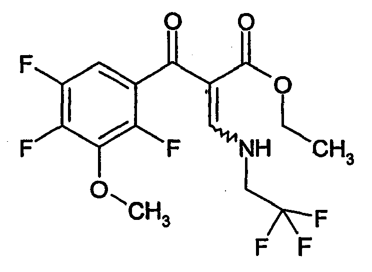

2.00 g (5.79 mmol) 3-Oxo-3-(2,4,5-trifluor-3-methoxyphenyl)-propansäureethylester werden in 3.8 ml (4.14 g, 40.55 mmol) Essigsäureanhydrid und 4.82 ml (4.29 g, 28.96 mmol) Orthoameisensäuretriethylester für 2 h unter Rückfluss gerührt. Das Lösungsmittel wird anschließend am Rotationsverdampfer vollständig entfernt und der Rückstand in 10 ml Ethanol gelöst. Zur eisgekühlten Lösung werden 1.03 g (10.43 mmol) 2,2,2-Trifluor-1-aminoethan zugetropft, auf Raumtemperatur gebracht und bei dieser Temperatur wird über Nacht nachgerührt. Zur Aufarbeitung wird das Lösungsmittel entfernt und der Rückstand ohne Reinigungsschritte als Rohprodukt weiter umgesetzt.
LC-MS (Methode 2): Rₜ = 2.37 min, MS (ES+) = 386 (M+H)⁺.

Die folgenden Beispiele 15A bis 22A werden analog zu Beispiel 14A aus den entsprechenden Aminen hergestellt.

| **Beispiel-Nr.** | **Struktur** | **Analytikdaten** |
|---|---|---|
| | | **LC-MS (Methode)/ Meßwerte** |
| **15A** | 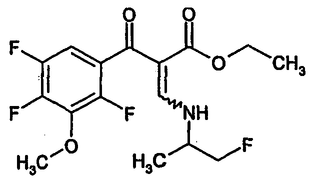 | LC-MS (Methode 2): Rₜ = 2.28 min |
| (racemisch) | | MS (ES+): m/z = 364 (M+H)⁺ |
| **16A** | 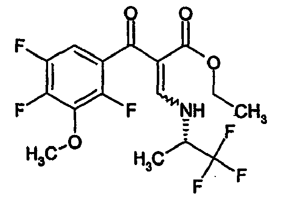 | LC-MS (Methode 1): Rₜ = 2.47 min |
| (S-Enantiomer) | | MS (ES+): m/z = 400 (M+H)⁺ |
| **17A** | 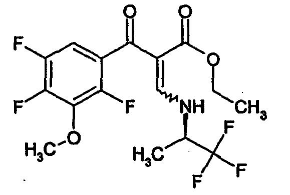 | LC-MS (Methode 1): Rₜ = 2.46 min |
| (R-Enantiomer) | | MS (ES+): m/z = 400 (M+H)⁺ |
| **18A** | 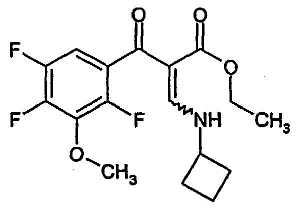 | LC-MS (Methode 3): Rₜ = 2.72 min |
| | | MS (ES+): m/z = 358 (M+H)⁺ |
| **19A** | 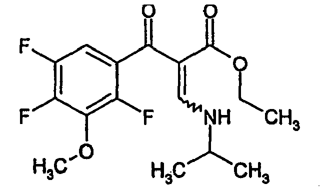 | LC-MS (Methode 1): Rₜ = 2.56 min |
| | | MS (ES+): m/z = 346 (M+H)⁺ |
| **20A** | 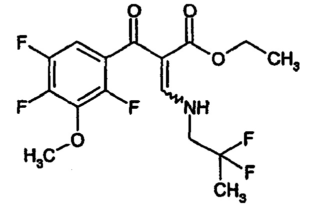 | LC-MS (Methode 1): Rₜ = 2.52 min |
| | | MS (ES+): m/z = 3 82 (M+H)⁺ |
| **21A** | 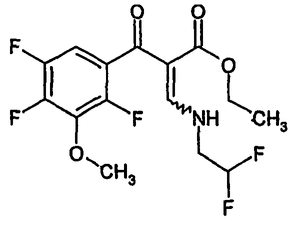 | LC-MS (Methode 2): Rₜ = 2.22 min |
| | | MS (ES+): m/z = 368 (M+H)⁺ |
| **22A** | 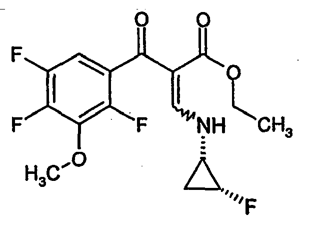 | LC-MS (Methode 1): Rₜ = 2.40 min |
| (1S,2R)-Enantiomer | | MS (ES+): m/z = 382 (M+H)⁺ |

### Beispiel 23A

### 6,7-Difluor-8-methoxy-4-oxo-1-(2,2,2-trifluorethyl)-1,4-dihydrochinolin-3-carbonsäureethylester

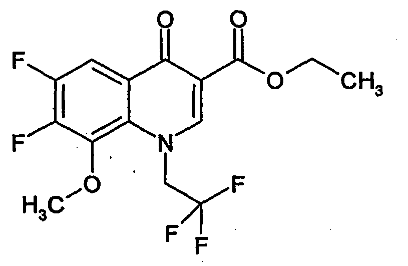

Unter Argon-Atmosphäre und Eiskühlung werden 0.32 g (8.11 mmol) 60%iges Natriumhydrid in 5 ml Tetrahydrofuran vorgelegt und eine Lösung von 2.23 g (5.79 mmol) der Verbindung aus Beispiel 14A in 15 ml Tetrahydrofuran wird langsam zugetropft. Die Mischung wird anschließend auf Raumtemperatur erwärmt, bei dieser Temperatur 2 h nachgerührt und über Nacht stehen gelassen. Zur Aufarbeitung werden 2 ml Essigsäure zugetropft, 5 min nachgerührt, mit Essigsäureethylester verdünnt, mehrmals mit Wasser und einmal mit gesättigter Natriumhydrogencarbonat-Lösung gewaschen, die organische Phase über Magnesiumsulfat getrocknet, filtriert und das Lösungsmittel wird vollständig am Rotationsverdampfer entfernt. Das Rohprodukt wird säulenchromatographisch über Kieselgel 60 (Laufmittel: Dichlormethan/Methanol 100/1 → 100/2) vorgereinigt und nach der Feinreinigung über die präparative RP-HPLC (Methode 5) werden 1.8 g Produkt erhalten.
HPLC (Methode 10): Rₜ = 4.34 min,
MS (DCI (NH₃)) = 366 (M+H)⁺.
¹H-NMR (300 MHz, CDCl₃): δ = 1.41 (t, 3H), 4.15 (s, 3H), 4.41 (q, 2H), 5.23 (q, 2H), 8.11 (dd, 1H), 8.33 (s, 1H).

Die in der folgenden Tabelle aufgeführten Beispiele 24A bis 31A werden analog zu Beispiel 23A aus den entsprechenden Aminen hergestellt. Für die Herstellung von 2-Amino-1-fluorpropan, siehe Journal of Organic Chemistry 1981, 46 (24), 4938-4948.

| **Beispiel-Nr.** | **Struktur** | **Edukt Beispiel-Nr.** | **Analytikdaten** |
|---|---|---|---|
| | | | **LC-MS (Methode)/ Meßwerte** |
| | | | **HPLC (Methode)/ Meßwert** |
| | | | **MS (Methode)/ Meßwert** |
| **24A** | 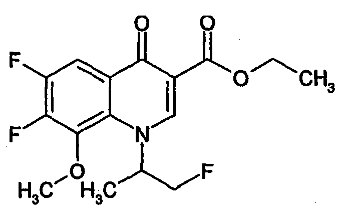 | **15A** | HPLC (Methode 10): Rₜ = 4.11 min |
| racemisch | | | MS (DCI(NH₃)): m/z = 344 (M+H)⁺ |
| **25A** | 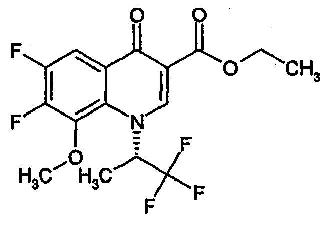 | **16A** | LC-MS (Methode 1): Rₜ = 2.22 min |
| (S)-Enantiomer | | | MS (ES+): m/z = 380 (M+H)⁺ |
| **26A** | 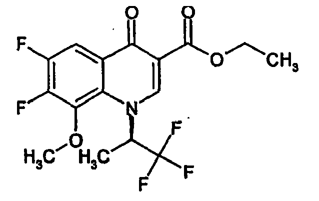 | **17A** | LC-MS (Methode 1): Rₜ = 2.22 min |
| (R)-Enantiomer | | | MS (ES+): m/z = 380 (M+H)⁺ |
| **27A** | 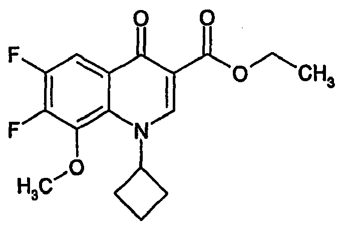 | **18A** | LC-MS (Methode 3): Rₜ = 2.33 min |
| | | | MS (ES+): m/z = 338 (M+H)⁺ |
| **28A** | 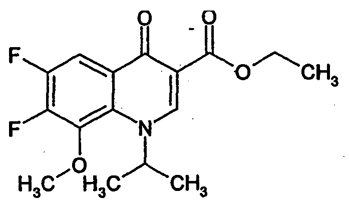 | **19A** | LC-MS (Methode 1): Rₜ = 2.16 min |
| | | | MS (ES+): m/z = 326 (M+H)⁺ |
| **29A** | 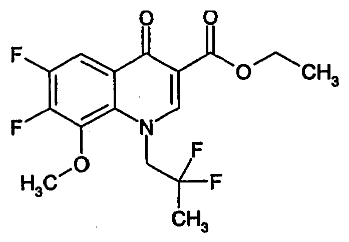 | **20A** | LC-MS (Methode 1): Rₜ = 2.11 min |
| | | | MS (ES+): m/z = 362 (M+H)⁺ |
| **30A** | 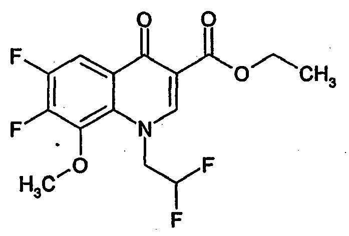 | **1A** | LC-MS (Methode 2): Rₜ = 1.83 min |
| | | | MS (ES+): m/z = 348 (M+H)⁺ |
| **31A** | 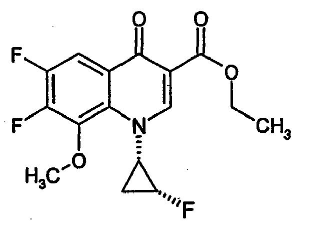 | **22A** | LC-MS (Methode 2): Rₜ = 1.76 min |
| (1S,2R)-Enantiomer | | | MS (ES+): m/z = 342 (M+H)⁺ |

### Beispiel 32A

### 6,7-Difluor-8-methoxy-4-oxo-1-(2,2,2-trifluorethyl)-1,4-dihydrochinolin-3-carbonsäure

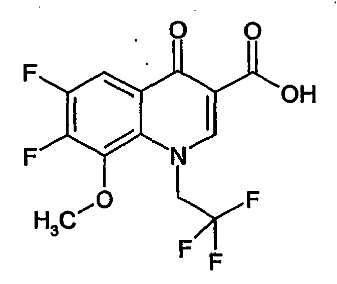

800 mg (2.19 mmol) der Verbindung aus Beispiel 23A werden in einer Mischung aus 25 ml Essigsäure-Wasser-Schwefelsäure 12:8:1 vorgelegt und über Nacht unter Rückfluss gerührt. Zur Aufarbeitung wird das Lösungsmittel am Rotationsverdampfer größtenteils entfernt, der Rückstand unter Eiskühlung vorsichtig mit gesättigter Natriumhydrogencarbonat-Lösung auf pH 3 gestellt, die Suspension mit Wasser verdünnt, der Niederschlag abgesaugt und nach dem Trocknen des Filterrückstandes im Hochvakuum werden 575 mg der Titelverbindung erhalten.
LC-MS (Methode 3): Rₜ = 2.41 min, MS (ES+) = 338 (M+H)⁺.
¹H-NMR (300 MHz, CDCl₃): δ = 4.21 (s, 3H), 5.37 (q, 2H), 8.11 (dd, 1H), 8.62 (s, 1H), 14.05 (bs, 1H).

Die folgenden Beispiele 33A bis 40A werden analog zu Beispiel 32A hergestellt.

| **Beispiel-Nr.** | **Edukt** | **Edukt** | **Analytikdaten** |
|---|---|---|---|
| | | | **LC-MS (Methode)/Meßwerte** |
| | | | **HPLC (Methode)/ Meßwert** |
| | | | **MS (Methode)/ Meßwert** |
| **33A** | 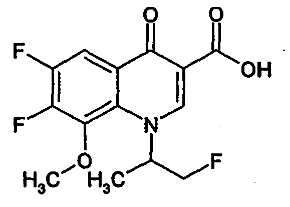 | **24A** | HPLC (Methode 10): Rₜ = 4.17 min |
| racemisch | | | MS (ESI+): m/z = 316 (M+H)⁺ |
| **34A** | 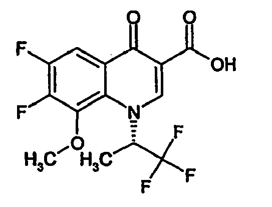 | **25A** | HPLC (Methode 10): Rₜ = 4.54 min |
| (S)-Enantiomer | | | MS (ESI+):m/z=374 (M+Na)⁺ |
| **35A** | 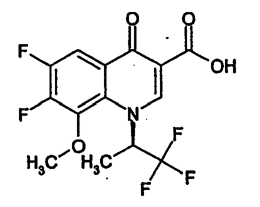 | **26A** | LC-MS (Methode 3): Rₜ = 2.47 min |
| (R)-Enantiomer | | | MS (ES+): m/z = 352 (M+H)⁺ |
| **36A** | 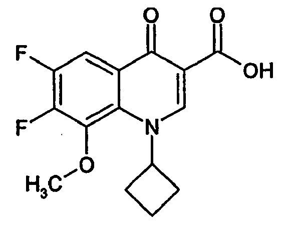 | **7A** | LC-MS (Methode 3): Rₜ = 2.35 min |
| | | | MS (ES+): m/z = 310 (M+H)⁺ |
| **37A** | 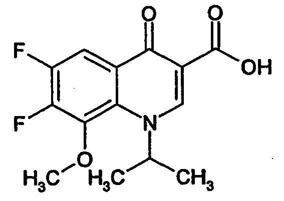 | **28A** | LC-MS (Methode 3): Rₜ = 2.27 min |
| | | | MS (ES+): m/z = 298 (M+H)⁺ |
| **38A** | 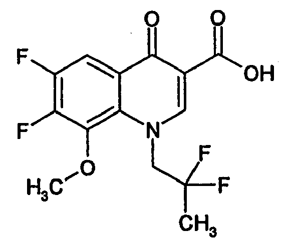 | **29A** | LC-MS (Methode 1): Rₜ = 2.22 min |
| | | | MS (ES+): m/z = 334 (M+H)⁺ |
| **39A** | 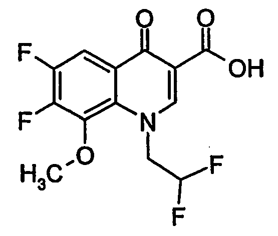 | **30A** | HPLC (Methode 9): Rₜ = 4.15 min |
| | | | MS (DCI (NH₃)): m/z = 337 (M+NH₄)⁺ |
| **40A** | 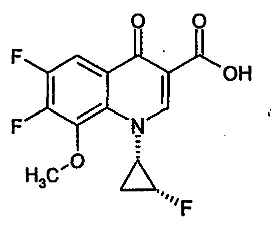 | **31A** | LC-MS (Methode 2): Rₜ =1.84 min |
| (1S,2R)-Enantiomer | | | MS (ES+): m/z = 313 (M+H)⁺ |

### Beispiel 41A

### [6,7-Difluor-8-methoxy-4-oxo-1-(2,2,2-trifluorethyl)-1,4-dihydrochinolin-3-yl]carbonyldifluorborat

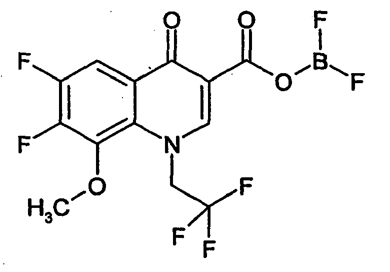

1.5 g (4.30 mmol) der Verbindung aus Beispiel 32A werden in 10 ml Tetrahydrofuran vorgelegt, anschließend mit 6.81 ml (7.63 g, 53.75 mmol) Bortrifluorid-Diethylether-Komplex versetzt und über Nacht bei 70°C nachgerührt. Zur Aufarbeitung wird das auf Raumtemperatur abgekühlte Reaktionsgemisch mit 50 ml Diethylether versetzt, 20 min verrührt und der ausfallende Niederschlag wird abgesaugt. Nach dem Trocknen des Rückstandes im Hochvakuum werden 1150 mg der Titelverbindung erhalten und ohne Reinigung weiter umgesetzt.
HPLC (Methode 9): Rₜ = 4.25 min,
MS (DCI (NH₃)) = 402 (M+NH₄)⁺.
¹H-NMR (300 MW, DMSO-d₆): δ = 4.21 (s, 3H), 6.12 (q, 2H), 8.38 (dd, 1H), 9.66 (s, 1H).

Die folgenden Beispiele 42A bis 49A werden analog zu Beispiel 41A hergestellt.

| **Beispiel-Nr.** | **Struktur** | **Edukt** | **Analytikdaten** |
|---|---|---|---|
| | | | **LC-MS (Methode)/ Meßwerte** |
| **42A** | 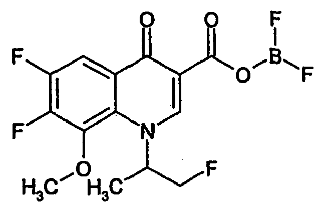 | **33A** | LC-MS (Methode 1): Rₜ = 1.96 min |
| | | | MS (ES+): m/z = 364 (M+H)⁺ |
| **43A** | 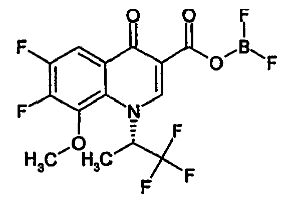 | **34A** | LC-MS (Methode 2): Rₜ = 1.98 min |
| (S)-Enantiomer | | | MS (ES+): m/z = 400 (M+H)⁺ |
| **44A** | 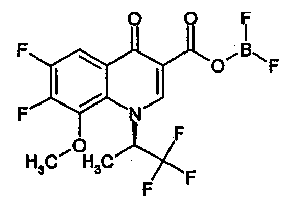 | **35A** | LC-MS (Methode 2): Rₜ = 1.98 min |
| (R)-Enantiomer | | | MS (ES+): m/z = 400 (M+H)⁺ |
| **45A** | 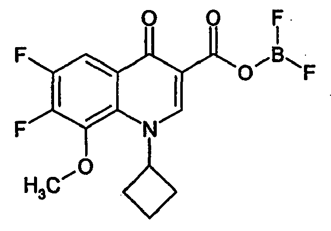 | **36A** | LC-MS (Methode 1): Rₜ = 1.92 min |
| | | | MS (ES+): m/z = 358 (M+H)⁺ |
| **46A** | 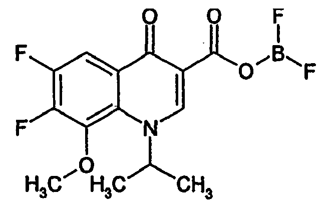 | **37A** | LC-MS (Methode 3): Rₜ = 1.83 min |
| | | | MS (ES+): m/z = 34b (M+H)⁺ |
| **47A** | 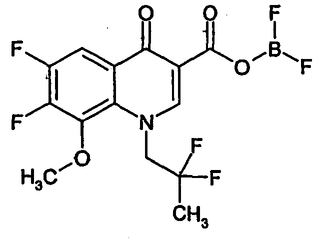 | **38A** | LC-MS (Methode 2): Rₜ =1.89 min |
| | | | MS (ES+): m/z = 382 (M+H)⁺ |
| **48A** | 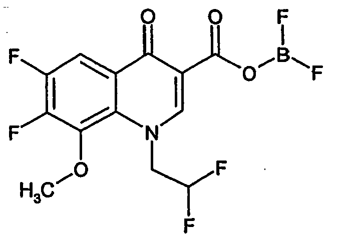 | **39A** | LC-MS (Methode 3): Rₜ = 2.09 min |
| | | | MS (ES+): m/z= 368 (M+H)⁺ |
| **49A** | 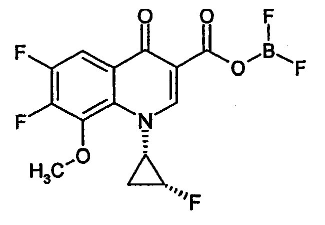 | **40A** | LC-MS (Methode 2): Rₜ =1.74 min |
| (1S,2R)-Enantiomer | | | MS (ES+): m/z = 361 (M+H)⁺ |

### Beispiel 50A

### 7-[(3RS,5SR)-3,5-Dimethylpiperazin-1-yl]-6-fluor-8-methoxy4-oxo-1-(2,2,2-trifluorethyl)-1,4-dihydrochinolin-3-carbonsäure-Hydroformiat

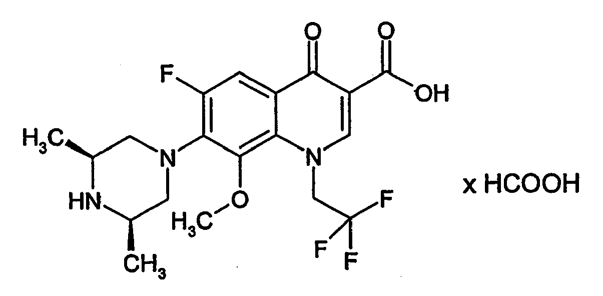

300.0 mg (0.78 mmol) der Verbindung aus Beispiel 41A und 213.6 mg (1.87 mmol) cis-2,6-Dimethylpiperazin werden in 6 ml Acetonitril über Nacht bei 50°C gerührt. Das Lösungsmittel wird vollständig am Rotationsverdampfer entfernt, der Rückstand wird mit einer Mischung aus 12 ml Ethanol und 6 ml Triethylamin 1 h unter Rückfluss gerührt. Zur Aufarbeitung wird das Lösungsmittel am Rotationsverdampfer abgetrennt und nach der Feinreinigung über die präparative RP-HPLC (Methode 5) werden 260 mg der Zielverbindung erhalten.
HPLC (Methode 9): Rₜ = 3.76 min,
MS (ESI+) = 432 (M+H)⁺.
¹H-NMR (300 MHz, DMSO-d₆): δ = 1.03 (d, 6H), 2.82 (m, 2H), 3.04 (m, 2H), 3.28 (m, 2H), 3.78 (s, 3H), 5.77 (q, 2H), 7.82 (d, 1H), 8.19 (s, 1H), 8.52 (s, 1H).

Die folgenden Beispiele 51A bis 62A werden analog zu Beispiel 50A hergestellt.

| **Beispiel-Nr.** | **Struktur** | **Edukt** | **Analytikdaten** |
|---|---|---|---|
| | | | **LC-MS (Methode)/ Meßwerte** |
| | | | **HPLC (Methode)/ Meßwert** |
| | | | **MS (Methode)/ Meßwert** |
| **51A** | 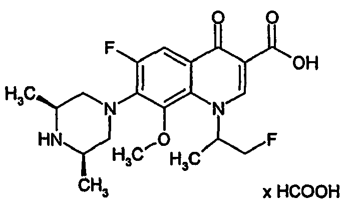 | **42A** | LC-MS (Methode 9): Rₜ = 3.67 min |
| racemisch | | | MS (ESI+): m/z = 410 (M+H)⁺ |
| **52A** | 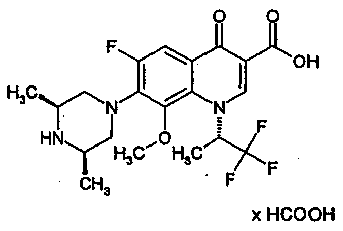 | **43A** | HPLC (Methode 10): Rₜ = 3.76 min |
| (S)-Enantiomer | | | MS (ESI+): m/z = 446 (M+H)⁺ |
| **53A** | 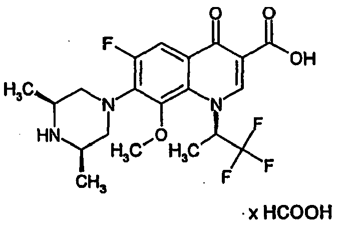 | **44A** | HPLC (Methode 10): Rₜ = 3.77 min |
| (R)-Enantiomer | | | MS (ESI+): m/z = 446 (M+H)⁺ |
| **54A** | 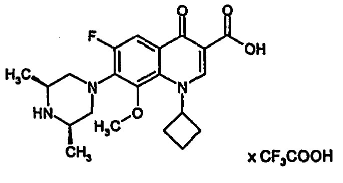 | **45A** | LC-MS (Methode 2): Rₜ = 1.16 min |
| | | | MS (ES+): m/z = 404 (M+H)⁺ |
| **55A** | 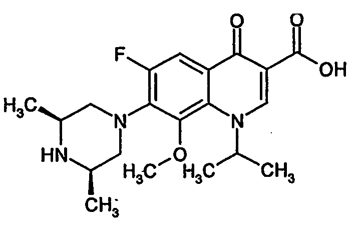 | **46A** | HPLC (Methode 9): Rₜ = 3.54 min |
| | | | MS (ESI+): m/z = 392 (M+H)⁺ |
| **56A** | 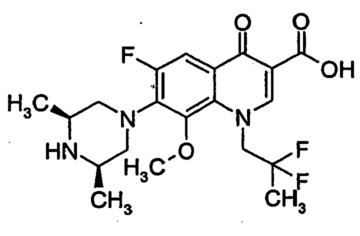 | **47A** | LC-MS (Methode 2): Rₜ = 1.10 min |
| | | | MS (ES+): m/z = 428 (M+H)⁺ |
| **57A** | 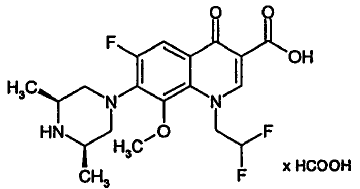 | **48A** | HPLC (Methode 9): Rₜ = 3.51 min |
| | | | MS (ESI+): m/z = 414 (M+H)⁺ |
| **58A** | 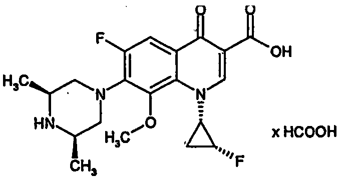 | **49A** | LC-MS (Methode 2): Rₜ =1.02 min |
| (1S,2R)-Enantiomer | | | MS (ES+): m/z = 407 (M+H)⁺ |
| **59A** | 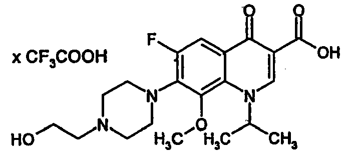 | **46A** | HPLC (Methode 10): Rₜ = 3.51 min |
| | | | MS (ESI+): m/z = 408 (M+H)⁺ |
| **60A** | 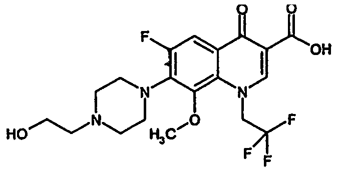 | **41A** | HPLC (Methode 9): Rₜ = 3.59 min |
| | | | MS (ESI+): m/z = 448 (M+H)⁺ |
| **61A** | 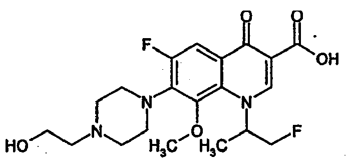 | **42A** | HPLC (Methode 9): Rₜ = 3.52 min |
| racemisch | | | MS (ESI+): m/z = 426 (M+H)⁺ |
| **62A** | 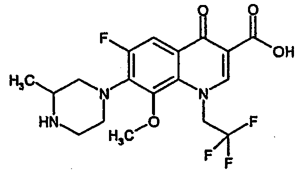 | **41A** | LC-MS (Methode 2): Rₜ =1.14 min. |
| | | | MS (ES+): m/z = 418 (M+H)⁺ |

### Beispiel 63A

### 8-Cyano-l-cyclopropyl-7-[(3RS,5SR)-3,5-dimethylpiperazin-1-yl]-6-fluor-4-oxo-1,4-dihydrochinolin-3-carbonsäure - Hydrochlorid

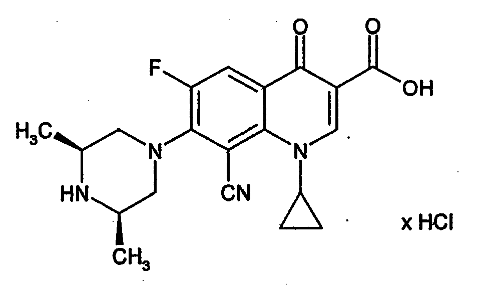

Eine Lösung von 500.0 mg (1.63 mmol) 7-Chlor-8-cyano-1-cyclopropyl-6-fluor-4-oxo-1,4-dihydrochinolin-3-carbonsäure (Herstellung siehe: DE 19854357) und 446.8 mg (3.91 mmol) cis-2,6-Dimethylpiperazin in 50 ml Acetonitril wird über Nacht bei 50°C gerührt. Das Lösungsmittel wird vollständig am Rotationsverdampfer entfernt, der Rückstand in 50 ml Wasser aufgenommen und mit 1N Natronlauge auf pH 11 eingestellt (der Rückstand löst sich). Dann wird mit 1N Salzsäure auf pH 7 eingestellt. Der Niederschlag wird abfiltriert, mit Wasser und Diethylether gewaschen und im Hochvakuum getrocknet. Man erhält 157 mg der Titelverbindung. Das Filtrat wird mit Dichlormethan extrahiert, die organische Phase eingeengt und der Rückstand durch präparative RP-HPLC gereinigt. Man erhält zusätzliche 351 mg der Titelverbindung.
LC-MS (Methode 2): Rₜ = 0.83 min
MS (ES+): m/z = 385 (M+H)⁺.

### Beispiel 64A

### 2-Chlor-4-methoxy-benzonitril

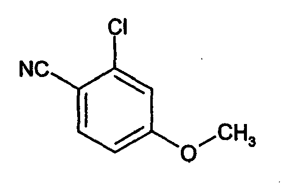

2.0 g 2-Chlor-4-hydroxybenzonitril werden in 50 ml THF unter Argon bei 0°C mit 2.6 g Natriumhydrid (60%ig in Öl) versetzt. Nach 10 min werden 9.24 g Methyliodid zugegeben und die Mischung über Nacht bei RT gerührt. Zur Aufarbeitung werden vorsichtig 2 ml Eisessig zugegeben, die Mischung einrotiert und der Rückstand mit 1N Salzsäure und Ethylacetat extraktiv aufgearbeitet. Die organische Phase wird mit Natriumsulfat getrocknet und einrotiert. Nach HPLC-Reinigung (Methode 5) erhält man 0.70 g Produkt.
MS (DCI / NH₃): m/z =184.9 (M+NH₄)⁺.
¹H-NMR (300 MHz, CDCl₃): δ = 7.58 (d, 1H), 7.01 (d, 1H), 6.87 (dd, 1H).

### Beispiel 65A

### 2-Brom-4-chlor-benzonitril

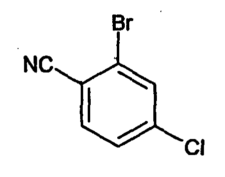

588 mg 2-Brom-4-chlorbenzoesäure und 300 mg Harnstoff werden in Dichlormethan / Methanol gelöst und auf 364 mg Aluminiumoxid einrotiert. Der Rückstand wird in der Mikrowelle bei 150°C insgesamt 60 min bestrahlt. Nach Abkühlung wird mit Ethylacetat und Wasser verrührt, abfiltriert und die wässrige Phase abgetrennt. Die organische Phase wird mit Natriumhydrogencarbonat-Lösung gewaschen, über Natriumsulfat getrocknet, einrotiert und dann im Hochvakuum getrocknet. Das Produkt wird ohne zusätzliche Reinigung weiter umgesetzt.
¹H-NMR (300 MHz, CDCl₃): δ = 7.72 (d, 1H), 7.60 (d, 1H), 7.42 (dd, 1H).

### Beispiel 66A

### 2-Chlor-4-(trifluormethoxy)-phenyl-trifluormethylsulfonat

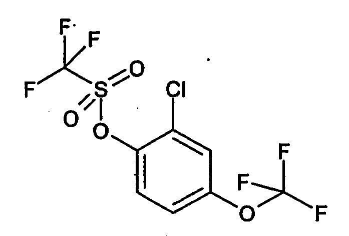

4.00 g 2-Chlor-4-trifluormethoxy-phenol in 50 ml Toluol und 50 ml einer 30%igen Kaliumphosphat-Lösung in Wasser werden bei 0°C vorgelegt, langsam mit 3.82 ml Trifluormethansulfonsäureanhydrid versetzt und 1.5 h bei RT gerührt. Die wässrige Phase wird abgetrennt und die organische Phase mit Wasser gewaschen, über Natriumsulfat getrocknet und eingeengt. Das Rohprodukt wird ohne Reinigung zu Beispiel 67A weiter umgesetzt.

### Beispiel 67A

### 2-Chlor-4-trifluormethoxy-benzonitril

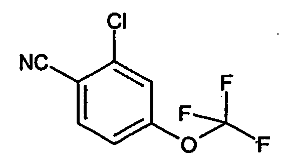

3.00 g der Verbindung aus Beispiel 66A werden in 12 ml entgastem DMF mit 2.04 g Zinkcyanid und 1.00 g Tetrakis(triphenylphosphin)palladium gelöst und unter Argon bei 120°C 2 h erhitzt. Nach Abkühlung wird die Reaktionsmischung mit Ethylacetat verdünnt und zweimal mit gesättigter Natriumhydrogencarbonat-Lösung, dann mit gesättigter Natriumchlorid-Lösung ausgeschüttelt. Die organische Phase wird über Natriumsulfat getrocknet und eingeengt. Der Rückstand wird durch Kieselgel-Chromatographie (Cyclohexan / Ethylacetat 10: 1) gereinigt.
¹H-NMR (300 MHz, DMSO-d₆): δ = 7.62 (dd, 1H), 7.95 (d, 1H), 8.18.(d, 1H).

### Beispiel 68A

### 2-Methyl-4-(trifluormethoxy)benzamid

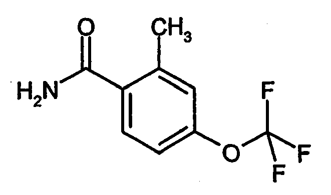

795 mg (3.61 mmol) 2-Methyl-4-(trifluormethoxy)benzoesäure werden mit 4 ml (54.8 mmol) Thionylchlorid und einem Tropfen DMF 30 min unter Rückfluss erhitzt. Nach dem Abkühlen wird die Reaktionslösung langsam in eisgekühlte konz. wässrige Ammoniaklösung getropft. Der entstandene Niederschlag wird abgesaugt, in 30 ml Wasser aufgenommen und 1 h bei 60°C gerührt. Man lässt abkühlen, filtriert den Feststoff ab und trocknet ihn im Vakuum. Ausbeute: 562 mg (71% d. Th.)
LC-MS (Methode 2): Rₜ = 1.61 min.
MS (ESI⁺): m/z = 220 (M+H)⁺
¹H-NMR (400MHz, DMSO-d₆): δ = 7.79 (bs, 1H), 7.42-7.50 (m, 2H), 7.19-7.28 (m, 2H), 2.39 (s, 3H).

### Beispiel 69A

### 2-Methyl-4-(trifluormethoxy)-benzylamin

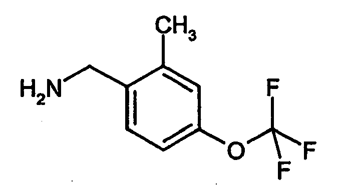

18.8 ml (18.8 mmol) Boran-THF-Komplex (1M) wird unter Argon und Eiskühlung vorgelegt. Es wird eine Lösung von 823 mg (3.76 mmol) 2-Methyl-4-(trifluormethoxy)benzamid (Beispiel 68A) in 80 ml THF zugetropft und anschließend 8 h unter Rückfluss gerührt. Unter Eiskühlung werden 80 ml 1N Salzsäure (bis zur Beendigung der Gasentwicklung) zugetropft und 1 h unter Rückfluss erhitzt. Anschließend wird das Reaktionsgemisch mit 1N Natronlauge alkalisch gestellt, dreimal mit Dichlormethan extrahiert, die vereinigten organischen Phasen über Natriumsulfat getrocknet und das Lösungsmittel im Vakuum entfernt. Man erhält ein Öl, das ohne weitere Reinigung weiter umgesetzt wird. Ausbeute: 732 mg (95% d. Th.).
LC-MS (Methode 3): Rₜ = 1.41 min.
MS (ESI): m/z = 206 (M+H)⁺
¹H-NMR (400MHz, CDCl₃): δ = 7.32-7.40 (m, 1H), 6.99-7.11 (m, 2H), 3.95-4.01 (m, 2H), 2.40 (s, 3H).

### Beispiel 70A

### 2-Brom-4-chlor-benzylamin

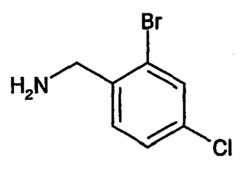

13.9 ml Boran-THF Komplex werden unter Eiskühlung vorgelegt. Langsam wird eine Lösung von 2.0 g 2-Brom-4-chlorbenzonitril (Beispiel 65A) in 60 ml THF dazugegeben. Danach wird die Reaktionsmischung 1 h unter Rückfluss erhitzt, abgekühlt und unter Eiskühlung mit 20 ml 1N Salzsäure tropfenweise versetzt. Man erhitzt 1 h unter Rückfluss und lässt abkühlen. Zur Auf arbeitung wird die Lösung mit 1N Natronlauge alkalisch gestellt und mit Dichlomethan extrahiert. Die organische Phase wird über Natriumsulfat getrocknet und einrotiert. Das Rohprodukt wird ohne Reinigung weiter umgesetzt.
¹H-NMR (300 MHz, CDCl₃): δ = 3.89(s, 2H), 7.35-7.45 (m [ABM], 2H), 7.55 (d, 1H).

### Beispiel 71A

### 4-Brom-2-chlor-benzylamin - Hydrochlorid

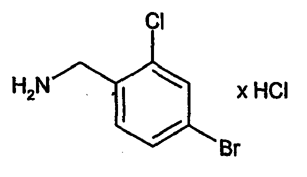

46.2 ml Boran-THF Komplex werden unter Eiskühlung vorgelegt. Langsam wird eine Lösung von 2.0 g 4-Brom-2-chlorbenzonitril in 240 ml THF dazugegeben. Danach wird die Reaktionsmischung 1 h unter Rückfluss erhitzt, abgekühlt und unter Eiskühlung mit 20 ml 1N Salzsäure tropfenweise versetzt. Man erhitzt 1 h unter Rückfluss und lässt abkühlen. Zur Aufarbeitung wird die Lösung mit 1N Natronlauge alkalisch gestellt und mit Dichlomethan extrahiert. Die organische Phase wird über Natriumsulfat getrocknet und einrotiert. Der Rückstand wird mit 6 ml Chlorwasserstoff in Dioxan (4N) versetzt und das ausgefallene Hydrochlorid abgesaugt. Man erhält 1.3 g Produkt.
¹H-NMR (300 MHz, DMSO-d₆): δ = 4.09 (s, 2H), 7.58 (dd, 1H), 7.68 (dd, 1H), 7.83 (d, 1H), 8.55 (bs, 3H).

### Beispiel 72A

### 4-Brom-2-methyl-benzylamin

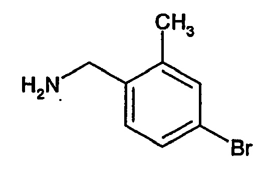

Die Herstellung erfolgt analog zu Beispiel 70A aus 4-Brom-2-methyl-benzonitril.
¹H-NMR (300 MHz, CDCl₃): δ = ca. 1.7 (br.s, NH2), 2.60 (s, 3H), 3.81(s, 2H), 7.19 (d, 1H), 7.28 (s, 1H), 7.30 (d, 1H).

### Beispiel 73A

### 2-Chlor-4-methoxy-benzylamin - Hydrochlorid

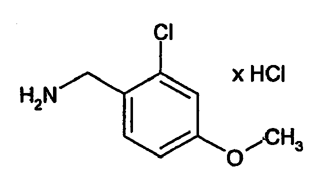

Die Herstellung erfolgt analog zu Beispiel 71A aus der Verbindung aus Beispiel 64A.
¹H-NMR (300 MHz, DMSO-d₆): δ = 3.80 (s, 3H), 4.04 (s, 2H), 7.01 (dd, 1H), 7.12 (d, 1H), 7.53 (d, 1H), 8.38 (bs, 3H).

### Beispiel 74A

### 2-Chlor-4-trifluormethoxy-benzylamin - Hydrochlorid

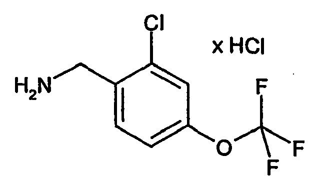

Die Herstellung erfolgt analog zu Beispiel 71A aus der Verbindung aus Beispiel 67A.
¹H-NMR (300 MHz, DMSO-d₆): δ = 4.15 (s, 2H), 7.52 (d, 1H); 7.70 (s, 1H), 7.78 (d, 1H), 8.56 (bs, 3H).

### Beispiel 75A

### 2-Chlor-4-trifluormethyl-benzylamin - Hydrochlorid

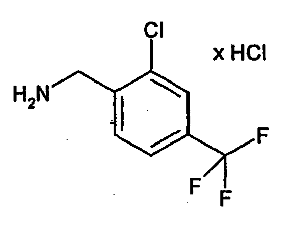

Die Herstellung erfolgt analog zu Beispiel 71A aus 2-Chlor-4-trifluormethyl-benzonitril.
¹H-NMR (300 MHz, DMSO-d₆): δ = 4.22 (s, 2H), 7.30-7.90 (m [AB], 2H), 7.40 (s, 1H), 8.00(s, 1H), 8.60 (bs, 3H).

### Beispiel 76A

### 2,4-Dichlor-6-methyl-benzylamin - Hydrochlorid

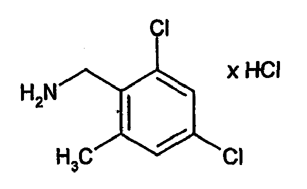

Die Herstellung erfolgt analog zu Beispiel 71A aus 2,4-Dichlor-6-methyl-benzonitril.
¹H-NMR (300 MHz, DMSO-d₆): δ = 2.5 (s, 3H), 4.10 (s, 2H), 7.40 (s, 1H), 7.60 (s, 1H), 8.40 (bs, 3H).
LC-MS (Methode 13): Rₜ =2.44 min, MS (ES+) = 190 (M+H)⁺.

### Beispiel 77A

### 4-Chlor-2-trifluormethyl-benzylamin - Hydrochlorid

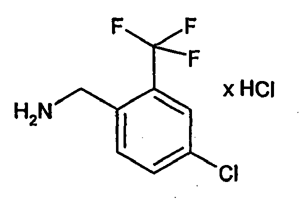

Die Herstellung erfolgt analog zu Beispiel 71A aus 4-Chlor-2-trifluormethyl-benzonitril.
¹H-NMR (300 MHz, DMSO-d₆): δ = 4.18 (d, 2H), 7.82 (d, 1H); 7.88-7.98 (m, 2H), 8.58 (bs, 3H).

### Beispiel 78A

### 2-Methyl-4-trifluormethyl-benzylamin - Hydrochlorid

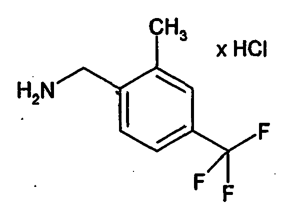

Die Herstellung erfolgt analog zu Beispiel 71A aus 2-Methyl-4-trifluormethyl-benzonitril.
¹H-NMR (400 MHz, DMSO-d₆): δ = 2.44 (s, 3H), 4.10 (s, 2H), 7.52 (s, 3H), 8.55 (bs, 3H).

### Beispiel 79A

### 8-Chlor-1-cyclopropyl-N-(2,4-dichlorbenzyl)-6,7-difluor-4-oxo-1,4-dihydrochinolin-3-carbonsäureamid

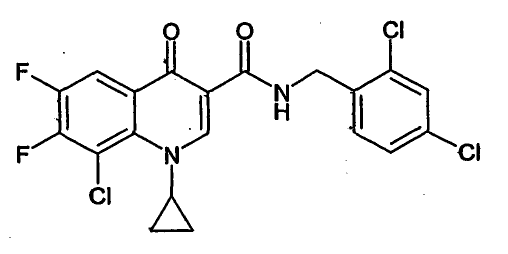

15.0 g 8-Chlor-1-cyclopropyl-6,7-difluor-4-oxo-1,4-dihydrochinolin-3-carbonsäure (Herstellung siehe DE 3420743 oder Y. Kimura et al., J. Med. Chem. 1994, 37 (20), 3344) werden in 500 ml DMF gelöst und mit 31.3 g PyBOP und 10.6 g 2,4-Dichlorbenzylamin versetzt. Nach einem Tag wird das Lösungsmittel entfernt und der Rückstand durch Flashchromatographie über Kieselgel (Toluol / Ethylacetat 95:5) gereinigt.
LC-MS (Methode 1): Rₜ= 3.10 min, MS (ES+) = 457 (M+H)⁺.

### Beispiel 80A

### 7-[(3R,5S)-4-(Chloracetyl)-3,5-dimethylpiperazin-1-yl]-1-cyclopropyl-N-(2,4-dichlorbenzyl)-6-fluor-8-methoxy-4-oxo-1,4-dihydrochinolin-3-carbonsäureamid

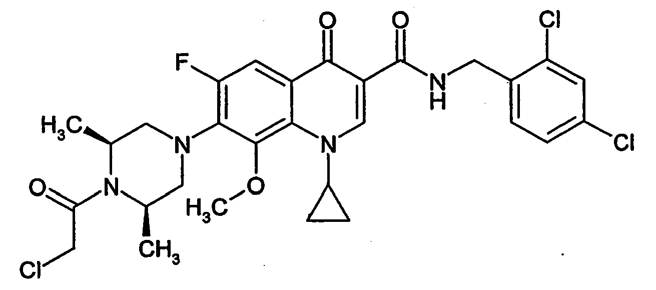

0.17 ml (0.25 g, 2.19 mmol) Chloracetylchlorid werden in 5 ml Dichlormethan vorgelegt, die Lösung auf 0°C gekühlt, anschließend 1.00 g (1.83 mmol) der Verbindung aus Beispiel 12 zugegeben, auf Raumtemperatur erwärmt und bei dieser Temperatur wird 1 h nachgerührt. Zur Aufarbeitung wird aus der Lösung mittels Unterdrucksäulenchromatographie über Kieselgel 60 bei einem Laufmittelgemisch von Dichlormethan: Ethanol 95:5 die Zielverbindung mit 0.49g isoliert.
LC-MS (Methode 3): Rₜ = 3.05 min, MS (ES+) = 623 (M+H)⁺.
¹H-NMR (400 MHz, DMSO-d₆): δ = 0.95 (m, 2H), 1.09 (m, 2H), 1.40 (m, 6H), 3.28 (m, 2H), 3.69 (s, 3H), 4.03-4.70 (m, 9H: darin 4.58 (d, 2H)), 7.35-7.47 (m, 2H), 7.63 (d, 1H), 7.78 (d, 1H), 8.69 (s, 1H), 10.25 (t, 1H).

### Beispiel 81A

### 7-[(3RS,5SR)-4-(Chloracetyl)-3,5-dimethylpiperazin-1-yl]-N-(2,4-dichlorbenzyl)-6-fluor-8-methoxy-1-(2,2,2-trifluorethyl)-4-oxo-1,4-dihydrochinolin-3-carbonsäureamid

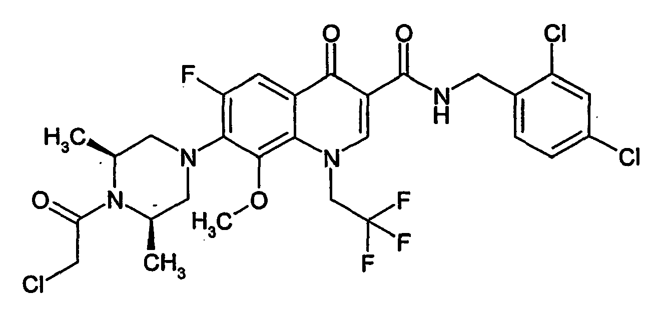

Analog zur Herstellung von Beispiel 80A wird aus der Verbindung aus Beispiel 47 die Titelverbindung erhalten.
LC-MS (Methode 2): Rₜ = 2.91 min, MS (ES+) = 666 (M+H)⁺.

### Beispiel 82A

### 7-[(3RS,5SR)-4-(Azidoacetyl)-3,5-dimethylpiperazin-1-yl]-1-cyclopropyl-N-(2,4-dichlorbenzyl)-6-fluor-8-methoxy-4-oxo-1,4-dihydrochinolin-3-carbonsäureamid - Hydrochlorid

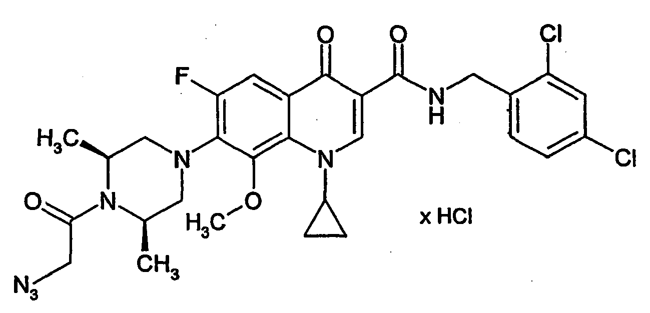

50.0 mg der Verbindung aus Beispiel 80A und 15.6 mg (0.24 mmol) Natriumazid werden in 3 ml N,N-Dimethylformamid in einem geschlossenen Reaktionsgefäß bei 90°C über Nacht gerührt. Nach der Feinreinigung über die präparative RP-HPLC (Methode 6) werden 46 mg der Zielverbindung erhalten.
LC-MS (Methode 1): Rₜ = 3.03 min, MS (ES+) = 630 (M+H)⁺.

### Beispiel 83A

### 7-[(3RS,5SR)-4-(Azidoacetyl)-3,5-dimethylpiperazin-1-yl]-N-(2,4-dichlorbenzyl)-6-fluor-8-methoxy-1-(2,2,2-trifluorethyl)-4-oxo-1,4-dihydrochinolin-3-carbonsäureamid

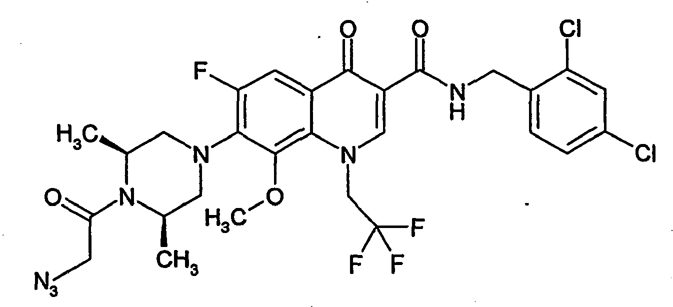

Analog zu Beispiel 82A aber bei RT in Gegenwart von 0.1 Äq. Kaliumiodid wird aus der Verbindung aus Beispiel 81A die Titelverbindung hergestellt.
LC-MS (Methode 3): Rₜ = 3.17 min, MS (ES+): m/z = 671 (M+H)⁺.

### Beispiel 84A

### 4-[3-{1-Cyclopropyl-[(2,4-dichlorbenzyl)amino]carbonyl}-1-fluor-8-methoxy-4-oxo-1,4-dihydrochinolin-7-yl]-(2RS,6SR)-2,6-dimethylpiperazin-1-yl}ethansäureethylester - Hydrochlorid

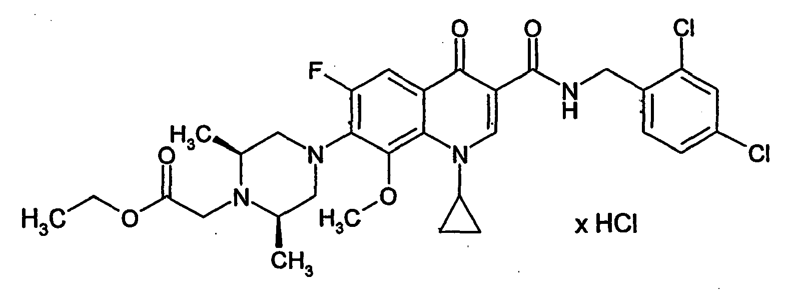

1 g der Verbindung aus Beispiel 12 wird mit 343 mg Ethylbromacetat, 312 mg Kaliumiodid und 590 mg Kaliumcarbonat in 60 ml Acetonitril für 2 h unter Rückfluss erhitzt. Nach Abkühlung wird die Reaktionsmischung über präparative HPLC (Methode 6) getrennt. Man erhält 862 mg (75% d. Th.) der Titelverbindung.
LC-MS (Methode 2): Rₜ = 2.39 min, MS (ESI): m/z = 633 (M+H)⁺.
¹H-NMR (400 MHz, DMSO-d₆): δ = 0.98 (m, 2H), 1.12 (m, 2H), 1.29 (t, 3H), 1.33 (d, 6H), 3.35-3.69 (m, 4H), 3.72-3.90 (m, 5H: darin 3.79 (s, 3H)), 4.11 (m, 1H), 4.23-4.51 (m, 4H: darin 4.29.(q, 2H)), 4.59 (d, 2H), 7.39 (d, 1H), 7.42 (dd, 1H), 7:53 (d, 1H), 7.78 (d, 1H), 8.69 (s, 1H), 10.22 (t, 1H).

### Beispiel 85A

### 4-[3-{1-Cyclopropyl-[(2,4-dichlorbenzyl)amino]carbonyl}-1-fluor-8-methoxy-4-oxo-1,4-dihydrochinolin-7-yl]-(2RS,6SR)-2,6-dimethylpiperazin-1-yl}ethansäure

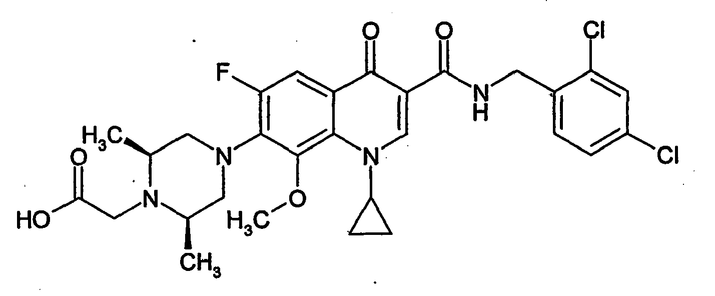

200 mg der Verbindung aus Beispiel 84A werden in 5 ml Dioxan gelöst, anschließend mit 5 ml 1M Lithiumhydroxid-Lösung versetzt und 2 h bei 50°C nachgerührt. Zur Aufarbeitung wird das Lösungsmittel am Rotationsverdampfer entfernt, der Rückstand mit Wasser aufgenommen, mit 1M Salzsäure angesäuert (pH 3-4). Der ausfallende Niederschlag wird abfiltriert, mit Wasser nachgewaschen und im Hochvakuum getrocknet. Man erhält 140 mg (73% d. Th.) der Titelverbindung.
LC-MS (Methode 1): Rₜ = 2.06 min, MS (ESI): m/z = 605 (M+H)⁺.
¹H-NMR (300 MHz, DMSO-d₆): δ = 0.99 (m, 2H), 1.18 (m, 2H), 1.38 (d, 6H), 3.46 (m, 2H), 3.55 (m, 2H), 3.70 (s, 3H), 3.78 (m, 4H), 3.95 (m, 1H), 4.68 (d, 2H), 7.20 (dd, 1H), 7.38 (m, 2H), 7.86 (d, 1H), 1H), 8.84 (s, 1H), 10.28 (t, 1H).

### Beispiel 86A

### 7-[(3RS,5SR)-4-(Chloracetyl)-3,5-dimethylpiperazin-1-yl]-6-fluor-8-methoxy-N-(2-methyl-4-trifluormethoxy-benzyl)-1-(2,2,2-trifluorethyl)-4-oxo-1,4-dihydrochinolin-3-carbonsäureamid

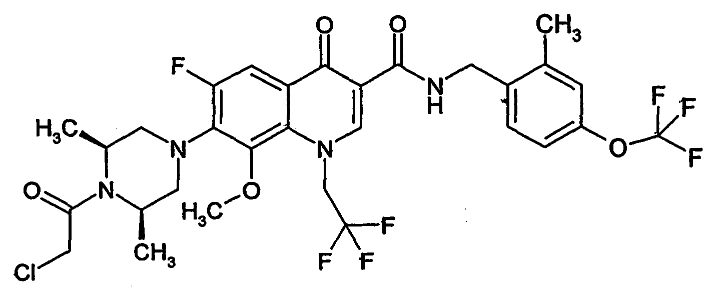

Die Titelverbindung wird analog zu Beispiel 80A aus der Verbindung aus Beispiel 52 hergestellt.

LC-MS (Methode 1): Rₜ = 3.09 min; MS (ES+): m/z = 695 (M+H)⁺.

### Beispiel 87A

### 7-[(3RS,5SR)-4-(Azidoacetyl)-3,5-dimethytpiperazm-1-yl]-6-fluor-8-methoxy-N-(2-methyl-4-trifluormethoxy-benzyl)-1-(2,2,2-trifluorethyl)-4-oxo-1,4-dihydrochinolin-3-carbonsäureamid

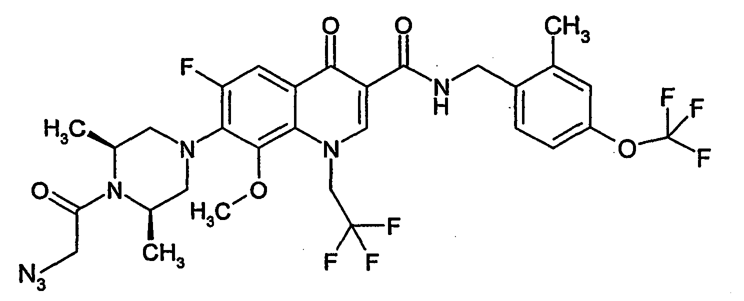

Die Titelverbindung wird analog zu Beispiel 82A aus der Verbindung aus Beispiel 86A hergestellt.
LC-MS (Methode 3): Rₜ = 3.15 min; MS (ES+): m/z = 702 (M+H)⁺
¹H-NMR (400 MHz, CDCl₃): δ = 1.51 (d, 6H), 2.40 (s, 3H), 3.30 (d, 2H), 3.44 (br.d, 2H), 3.75 (s, 3H), 4.00 (br.s, 2H), 4.62 (d, 2H), 5.20 (q, 2H), 7.00-7.06 (m, 2H), 7.35 (d, 1H), 7.95 (d, 1H), 8.60 (s, 1H), 9.99 (t, 1H).

### Ausführungsbeispiele

### Beispiel 1

### 8-Chlor-1-cyclopropyl-N-(2,4-dichlorbenzyl)-6-fluor-7-[4-(2-hydroxyethyl)-1,4-diazepan-1-yl]-4-oxo-1,4-dihydrochinolin-3-carbonsäureamid

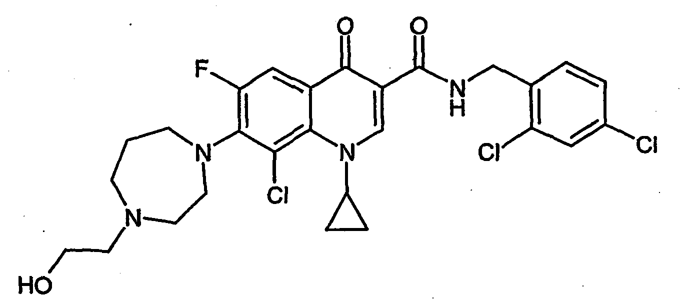

110 mg (0.26 mmol) der Verbindung aus Beispiel 1A werden in 2 ml Dimethylformamid gelöst, mit 35 mg (0.26 mmol) 1-Hydroxybenztriazol, 46 mg (0.26 mmol) 2,4-Dichlorbenzylamin und 55 mg (0.29 mmol) N-(3-Dimethylaminopropyl)-N'-ethylcarbodiimid Hydrochlorid versetzt. Nach zwei Tagen Rühren bei Raumtemperatur wird mit 2 ml Wasser verdünnt. Der Ansatz wird durch präparative HPLC (Methode 4) gereinigt. Man erhält 34.5 mg der Zielverbindung.
LC-MS (Methode 3): Rₜ = 1.95 min,
MS (ES+) = 581 (M+H)⁺

Analog zur Herstellungsvorschrift von Beispiel 1 werden die Beispiele 2 bis 6 hergestellt:

### Beispiel 2

### 8-Chlor-1-cyclopropyl-N-(2,4-dichlorbenzyl)-6-fluor-7{4-[2-(2-hydroxyethoxy)ethyl]piperazin-1-yl}-4-oxo-1,4-dihydrochinolin-3-carbonsäureamid

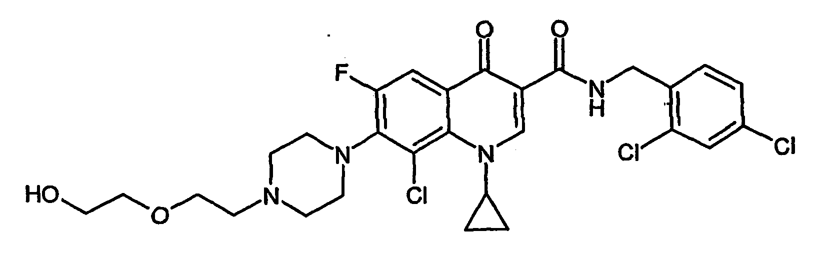

Die Herstellung erfolgt analog zu Beispiel 1 aus Beispiel 2A.
LC-MS (Methode 2): Rₜ = 1.78 min,
MS (ES+) = 611 (M+H)⁺
¹H-NMR (300 MHz, DMSO-d₆): δ = 0.9 (m, 2H), 1.2 (m, 2H), 2.6 - 2.7 (m, ca. 6H), 3.3 (Signale unter dem Lösemittel), 3.4 - 3.6 (m, ca. 6H), 4.3 (m, 1H), 4.5 (d, 2H), 7.4 (m, 2H), 7.65 (d, 1H), 7.9 (d, 1H), 8.8 (s, 1H), 10.1 (t, 1H).

### Beispiel 3

### 8-Chlor-N-(2,4-dichlorbenzyl)-6-fluor-1-[(1R,2S)-2-fluorcyclopropyl]-7-{4-[2-(2-hydroxyethoxy)-ethyl]piperazin-1-yl}-4-oxo-1,4-dihydrochinolin-3-carbonsäureamid

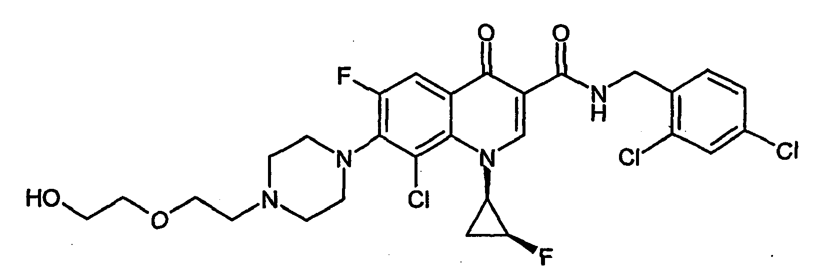

Die Herstellung erfolgt analog zu Beispiel 1 aus Beispiel 3A.
LC-MS (Methode 2): Rₜ = 1.76 min,
MS (ES+) = 629 (M+H)⁺

### Beispiel 4

### 8-Chlor-N-(2,4-dichlorbenzyl)-6-fluor-1-[(1S,2R)-2-fluorcyclopropyl]-7-{4-[2-(2-hydroxyethoxy)-ethyl]piperazin-1-yl}-4-oxo-1,4-dihydrochinolin-3-carbonsäureamid

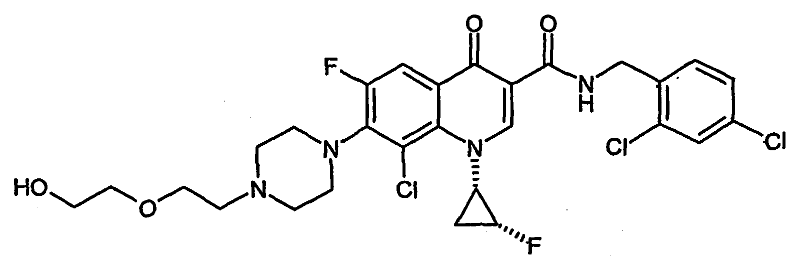

Die Herstellung erfolgt analog zu Beispiel 1 aus Beispiel 4A.
LC-MS (Methode 1): Rₜ = 1.98 min,
MS (ES+) = 629 (M+H)⁺

### Beispiel 5

### 8-Chlor-1-cyclopropyl-N-(2,4-dichlorbenzyl)-7-[(3RS,5SR)-3,5-dimethylpiperazin-1-yl]-6-fluor-4-oxo-1,4-dihydrochinolin-3-carbonsäureamid

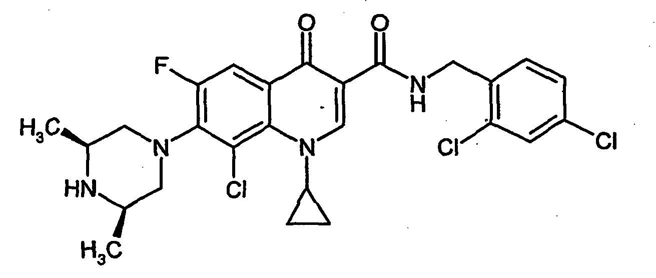

Die Herstellung erfolgt analog zu Beispiel 1 aus 8-Chlor-1-cyclopropyl-7-[(3RS,5SR)-3,5-dimethylpiperazin-1-yl]-6-fluor-4-oxo-1,4-dihydrochinolin-3-carbonsäure (Herstellung siehe: DE 3635218)
LC-MS (Methode 2): Rₜ =1.86 min,
MS (ES+) = 551 (M+H)⁺
¹H-NMR (400 MHz, CDCl₃): δ = 0.9 (m, 2H), 1.1 (d, 6H), 1.2 1.3 (m, 2H), 2.7 - 2.9 (m, 2H), 3.1-3.3 (m, 4H), 4.3 (m, 1H), 4.7 (d, 2H), 7.2 (dd, 2H), 7.4 (m, 2H), 8.0 (d, 1H), 8.9 (s, 1H), 10.2 (t, 1H).

### Beispiel 6

### 8-Chlor-N-(2,4-dichlorbenzyl)-6-fluor-1-[(1S,2R)-2-fluorcyclopropyl]-7-[4-(2-morpholin-4-yl-2-oxoethyl)piperazin-1-yl]-4-oxo-1,4-dihydrochinolin-3-carbonsäureamid

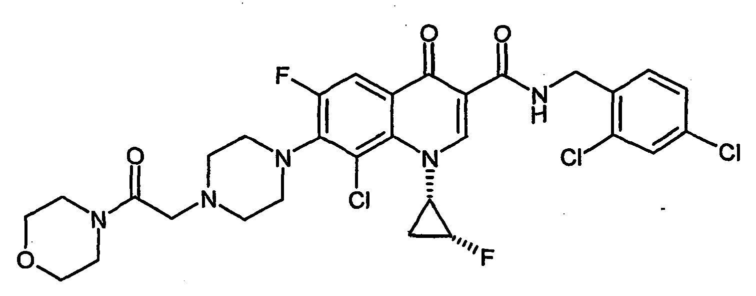

Die Herstellung erfolgt analog zu Beispiel 1 aus Beispiel 5A.
LC-MS (Methode 1): Rₜ = 2.08 min,
MS (ES+) = 668 (M+H)⁺

### Beispiel 7

### N-(2,4-Dichlorbenzyl)-6-fluor-1-[(1R,2S)-2-fluorcyclopropyl]-7-{4-[2-(2-hydroxyethoxy)-ethyl]piperazin-1-yl}-8-methoxy-4-oxo-1,4-dihydrochinolin-3-carbonsäureamid

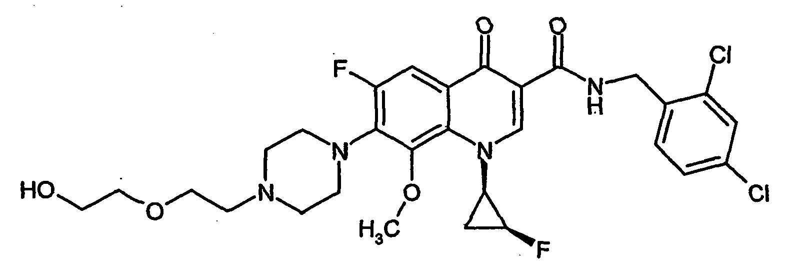

105 mg (0.14 mmol) Carbonsäure aus Beispiel 6A in 2 ml Dimethylformamid werden unter Argon mit 140 mg (0.27 mmol) 1-Benzotriazolyloxy-tripyrrolidinophosphoniumhexafluorophosphat, 47 mg (0.27 mmol) 2,4-Dichlorbenzylamin und 35 mg (0.27 mmol) Diisopropylethylamin versetzt und 2 Tage bei Raumtemperatur gerührt. Das Reaktionsgemisch wird mit 2 ml Wasser verdünnt und ohne weitere Aufarbeitung durch präparative HPLC (Methode 4) aufgereinigt. Man erhält 52 mg der Zielverbindung.
LC-MS (Methode 1): Rₜ = 1.91 min,
MS (ES+) = 625 (M+H)⁺

Analog zur Herstellungsvorschrift von Beispiel 7 werden die Beispiele 8 bis 18 hergestellt:

### Beispiel 8

### 8-Chlor-N-(4-chlor-2-methylbenzyl)-1-cyclopropyl-6-fluor-7-{4-[2-(2-hydroxyethoxy)ethyl]-piperazin-1-yl}-4-oxo-1,4-dihydrochinolin-3-carbonsäureamid

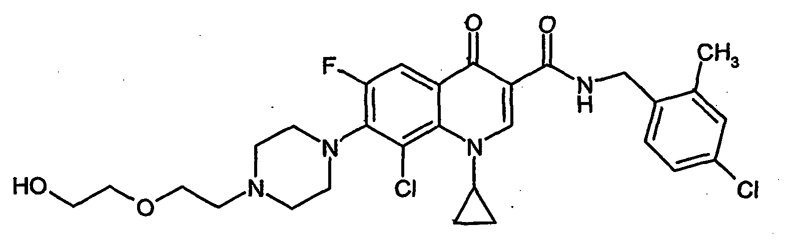

Die Herstellung erfolgt analog zu Beispiel 7 aus Beispiel 2A.
LC-MS (Methode 3): Rₜ =1.91 min,
MS (ES+) = 591 (M+H)⁺
¹H-NMR (400 MHz, CDCl₃): δ = 0.9 (m, 2H), 1.2 (m, 2H), 2.4 (s, 3H), 2.6 - 2.7 (m, ca. 6H), 3.4 (m, ca. 4H), 3.6 - 3.8 (m, 6H), 4.2 (m, 1H), 4.6 (d, 2H), 7.2 (m, 2H), 7.35 (dd, 2H), 7.9 (d, 1H), 8.9 (s, 1H), 10.0 (t, 1H).

### Beispiel 9

### 8-Chlor-N-(2,4-dichlorbenzyl)-6-fluor-1-[(1SR,2RS)-2-fluorcyclopropyl]-7-{4-[2-(2-hydroxyethyl)piperazin-1-yl]-4-oxo-1,4-dihydrochinolin-3-carbonsäureamid

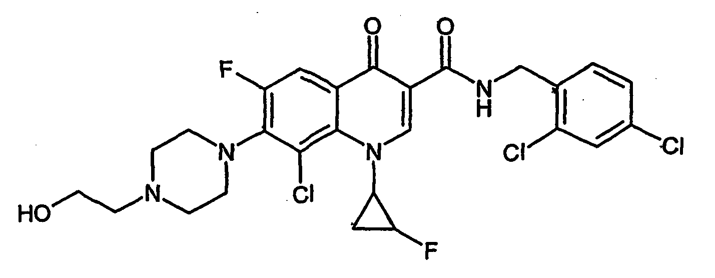

Die Herstellung erfolgt analog zu Beispiel 7 aus Beispiel 7A.
LC-MS (Methode 2): Rₜ = 1.63 min,
MS (ES+) = 585 (M+H)⁺

### Beispiel 10

### 8-Chlor-N-(2,4-dichlorbenzyl)-6-fluor-1-[(1SR,2RS)-2-fluorcyclopropyl]-7-(4-methyl-piperazin-1-yl)-4-oxo-1,4-dihydrochinolin-3-carbonsäureamid

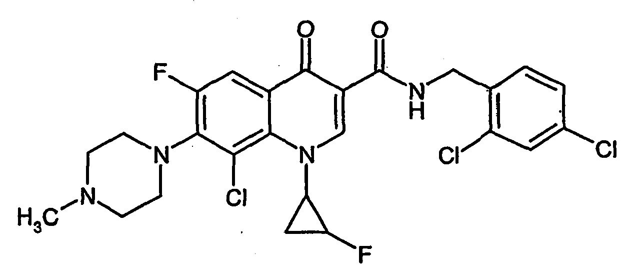

Die Herstellung erfolgt analog zu Beispiel 7 aus Beispiel 8A.
LC-MS (Methode 2): Rₜ =1.70 min,
MS (ES+) = 555 (M+H)⁺

### Beispiel 11

### 8-Chlor-N-(2,4-dichlorbenzyl)-6-fluor-1-[(1SR,2RS)-2-fluorcyclopropyl]-7-{4-[2-(2-hydroxyethoxy)ethyl]piperazin-1-yl}-4-oxo-1,4-dihydrochinolin-3-carbonsäureamid

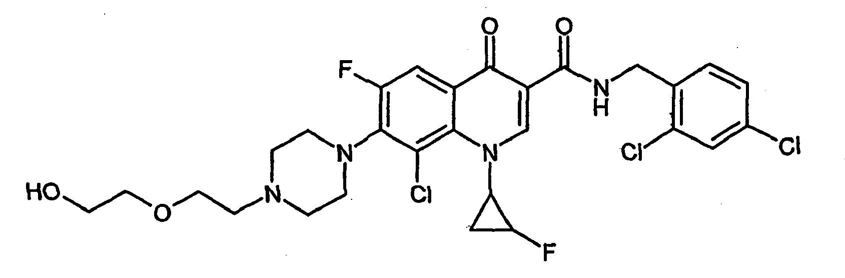

Die Herstellung erfolgt analog zu Beispiel 7 aus Beispiel 9A.
LC-MS (Methode 3): Rₜ = 1.89 min,
MS (ES+) = 629 (M+H)⁺

### Beispiel 12

### 1-Cyclopropyl-N-(2,4-dichlorbenzyl)-7-[(3RS,5SR)-3,5-dimethylpiperazin-1-yl]-6-fluor-8-methoxy-4-oxo-1,4-dihydrochinolin-3-carbonsäureamid

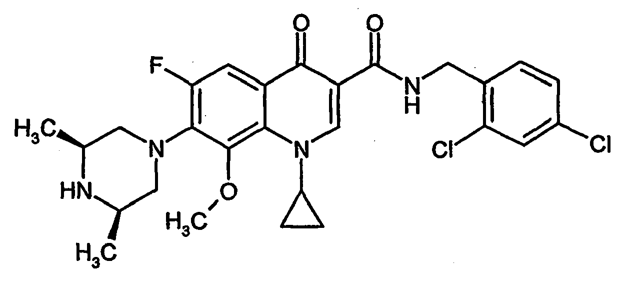

Die Herstellung erfolgt analog zu Beispiel 7 aus 1-Cyclopropyl-7-(cis-3,5-dimethylpiperazin-1-yl)-6-fluor-8-methoxy-4-oxo-1,4-dihydrochinolin-3-carbonsäure (Herstellung siehe: Journal of Medicinal Chemistry, (1995), 38(22), 4478-87).
LC-MS (Methode 2): Rₜ = 1.77 min,
MS (ES+) = 547 (M+H)⁺

### Beispiel 13

### 1-Cyclopropyl-N-(2,4-dichlorbenzyl)-8-difluormethoxy-7-[(3RS,5SR)-3,5-dimethylpiperazin-1-yl]-6-fluor-4-oxo-1,4-dihydrochinolin-3-carbonsäureamid

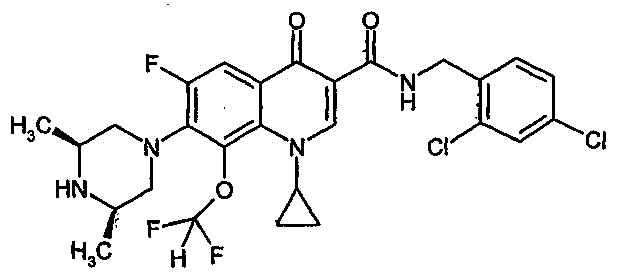

Die Herstellung erfolgt analog zu Beispiel 7 aus 1-Cyclopropyl-7-(cis-3,5-dimethylpiperazin-1-yl)-8-difluormethoxy-6-fluor-4-oxo-1,4-dihydrochinolin-3-carbonsäure (Herstellung siehe: EP 352123).
LC-MS (Methode 2): Rₜ = 2.05 min,
MS (ES+) = 583 (M+H)⁺

### Beispiel 14

### N-(4-Chlor-2-methylbenzyl)-1-cyclopropyl-6-fluor-7-{4-[2-(2-hydroxyethoxy)ethyl]-piperazin-1-yl}-8-rhethoxy-4-oxo-1,4-dihydrochinolin-3-carbonsäureamid

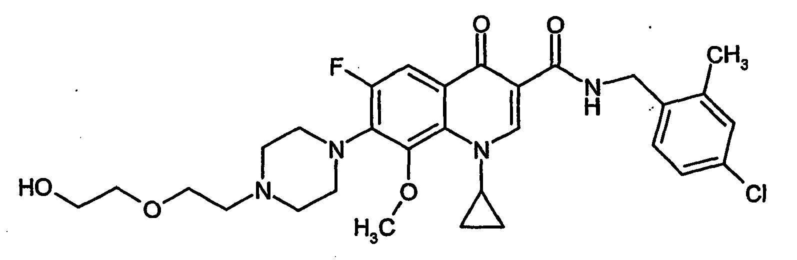

Die Herstellung erfolgt analog zu Beispiel 7 aus Beispiel 10A.
LC-MS (Methode 2): Rₜ =1.70 min,
MS (ES+) = 587 (M+H)⁺

### Beispiel 15

### 8-Chlor-N-(4-chlor-2-methylbenzyl)-6-fluor-1-[(1S,2R)-2-fluorcyclopropyl]-7-[4-(-2-morpholin-4'-yl-2-oxoethyl)piperazin-1-yl]-4-oxo-1,4-dihydrochinolin-3-carbonsäureamid

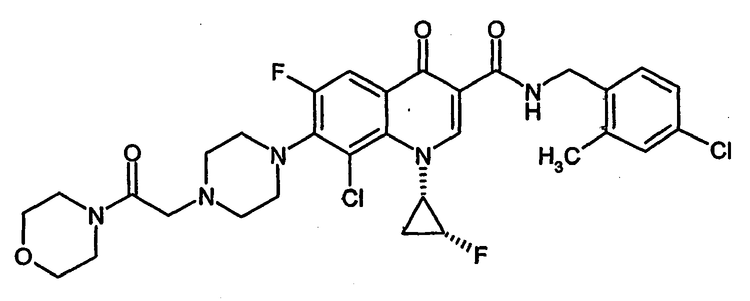

Die Herstellung erfolgt analog zu Beispiel 7 aus Beispiel 5A.
LC-MS (Methode 2): Rₜ =1.66 min,
MS (ES+) = 648 (M+H)⁺

### Beispiel 16

### N-(2,4-Dichlorbenzyl)-7-[(3RS,5SR)-3,5-dimethylpiperazin-1-yl]-6-fluor-1-[(1R,2S)-2-fluorcyclopropyl]-8-methoxy-4-oxo-1,4-dihydrochinolin-3-carbonsäureamid

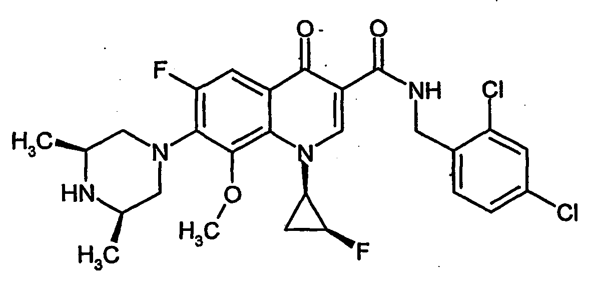

Die Herstellung erfolgt analog zu Beispiel 7 aus Beispiel 11A.
LC-MS (Methode 2): Rₜ = 1.66 min,
MS (ES+) = 565 (M+H)⁺
¹H-NMR (300 MHz, CDCl₃): δ = 1.1 (d, 6H), 1.4 -1.7 (m), 2.7 - 2.9 (m, 2H), 3.0 - 3.2 (m, 2H), 3.2 - 3.4 (m, 2H), 3.7 (s, 3H), 3.8 - 3.9 (m, 111), 4.6 - 4.9 (m, ca. 3H), 7.1 - 7.2 (dd, 1H), 7.3 - 7.5 (m, 2H), 7.8 - 7.9 (d, 1H), 8.8 (s, 1H), 10.3 -10,4 (t, 1H).

### Beispiel 17

### N-(4-Chlor-2-methylbenzyl)-7-[(3RS,5SR)-3,5-dimethylpiperazin-1-yl]-6-fluor-1-[(1R,2S)-2-fluorcyclo-propyl]-8-methoxy-4-oxo-1,4-dihydrochinolin-3-carbonsäureamid

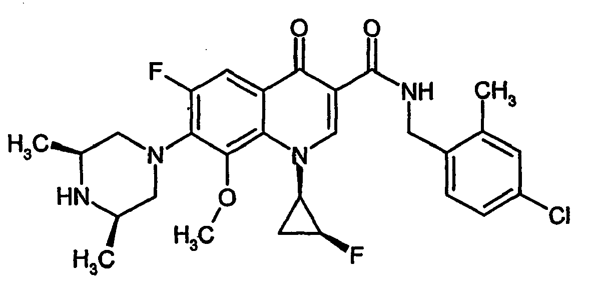

Die Herstellung erfolgt analog zu Beispiel 7 aus Beispiel 11A.
LC-MS (Methode 3): Rₜ = 1.89 min,
MS (ES+) = 545 (M+H)⁺
¹H-NMR (300 MHz, CDCl₃): δ = 1.1 (d, 6H), 1.4 - 1.7 (m), 2.4 (s, 3H), 2.7 - 2.9 (m, 2H), 3.0-3.2 (m, 2H), 3.2 - 3.4 (m, 2H), 3.7 (s, 3H), 3.8 - 3.9 (m, 1H), 4.5 - 4.65 (m, 1H), 4.65 - 5.0 (m, 2H), 7.1 - 7.2 (m, 2H), 7.3 (m, cirka 1H), 7.75 (d, 1H), 8.8 (s, 1H), 10.2 (t, 1H).

### Beispiel 18

### 1-Cyclopropyl-N-(2,4-dichlorbenzyl)-8-difluormethoxy-6-fluor-7-{4-[2-(2-hydroxy-ethoxy)ethyl] piperazin-1-yl}-4-oxo-1,4-dihydrochinolin-3-carbonsäureamid

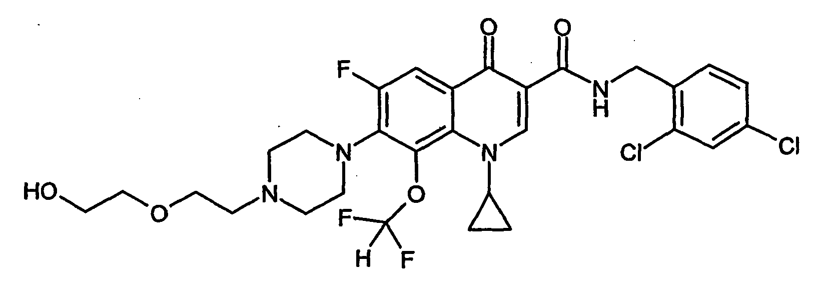

Die Herstellung erfolgt analog zu Beispiel 7 aus Beispiel 12A.
LC-MS (Methode 2): Rₜ = 1.66 min,
MS (ES+) = 643 (M+H)⁺
¹H-NMR (400 MHz, CDCl₃): δ = 0.9 (m, 2H), 1.2 (m, 2H), 2.7 (m, ca. 6H), 3.4(m, 4H), 3.6 - 3.8 (m, 6H), 4.1 (m, 1H), 4.7 (d, 2H), 6.5 (dd, 1H), 7.2 (m, 1H), 7.4 (m, 2H), 8.0 (d, 1H), 8.8(s, 1H), 10.2 (t, 1H).

### Beispiel 19

### N-(4-Brom-2-chlorbenzyl)-1-cyclopropyl-7-[(3RS,5RS-3,5-dimethyl)-piperazin-1-yl]-6-fluor-8-methoxy-4-oxo-1,4-dihydrochinolin-3-carbonsäureamid - Hydroformiat

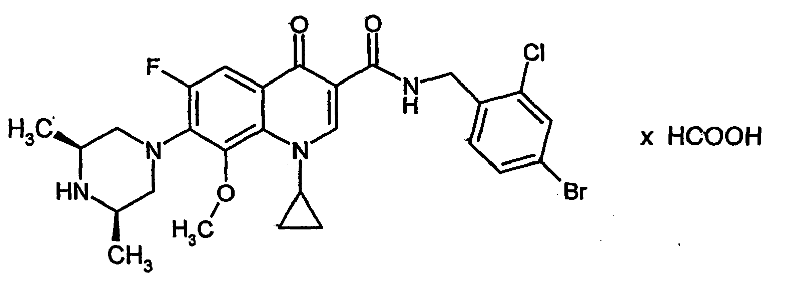

75 mg (0.19 mmol) 1-Cyclopropyl-7-(cis-3,5-dimethylpiperazin-1-yl)-6-fluor-5-methoxy-4-oxo-1,4-dihydrochinolin-3-carbonsäure (Herstellung siehe: Journal of Medicinal Chemistry, (1995), 38(22), 4478-87) in 2 ml Dimethylformamid werden unter Argon mit 130 mg (0.25 mmol) PyBOP 78 mg (0.36 mmol) 2-Brom-4-chlor-benzylamin (Beispiel 70A) und 127 mg. (0.98 mmol) N,N-Diisopropylethylamin versetzt und über Nacht bei Raumtemperatur gerührt. Das Reaktionsgemisch wird mit 2 ml Wasser verdünnt und ohne weitere Aufarbeitung durch präparative HPLC (Methode 5) aufgereinigt. Man erhält 92 mg der Titelverbindung.
LC-MS (Methode 1): Rₜ = 1.96 min, MS (ES+) = 591 (M+H)^{+,} (⁷⁹Br ³⁵Cl), 593 (M+H)^{+.} (⁸¹Br³⁵Cl).
¹H-NMR (300 MHz, CDCl₃): δ = 0.97 (m, 2H), 1.18 (m, 2H), 1.38 (d, 6H), 3.34.3.52 (m, 6H), 3.77 (s, 3H), 3.95 (m, 1H), 4.69 (d, 2H), 4.86 (m, 1H), 7.33 (m, 2H), 7.52 (s, 2H), 7.91 (d, 1H), 8.43 (s, 1H), 8.86 (s, 1H), 10.34 (t, 1H)

Aus der gleichen Säure und analog zur Herstellungsvorschrift von Beispiel 19 werden mit den entsprechenden Aminen (käuflich oder unter Beispiel 69A bis 78A beschrieben) die Beispiele 20 bis 27 hergestellt.

| **Beispiel-Nr.** | **Struktur** | **Analytikdaten** |
|---|---|---|
| | | **LC-MS (Methode)/ Meßwerte** |
| | | **HPLC (Methode)/ Meßwert** |
| | | **MS (Methode)/ Messwert** |
| | | **NMR-Spektrum** |
| **20** | 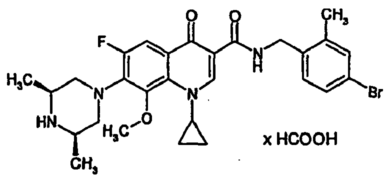 | LC-MS (Methode 1):Rₜ =1.84 min MS (ES+): m/z = 571 (M+H, ⁷⁹Br)⁺; m/z = 573 (M+H, ⁸¹Br)⁺ |
| | | ¹H-NMR (400 MHz, CDCl₃): δ = 0.97 (m, 2H), 1.18 (m, 2H), 1.34 (d, 6H), 2.37 (s, 3H), 3.21-3.51 (m, 6H), 3.77 (s, 3H), 3.95 (m, 1H), 4.56 (d, 2H), 7.21 (m, 1H), 7.31 (m, 2H), 7.88 (d, 1H), 8.47 (s, 1H), 8.86 (s, 1H), 10.16 (m, 1H) |
| **21** | 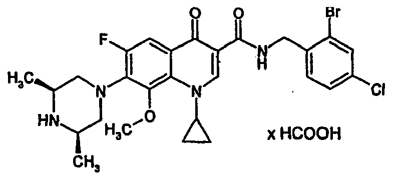 | LC-MS (Methode 3): Rₜ =1.92 min |
| | | MS (ES+): m/z = 591 (M+H, ⁷⁹Br)⁺; m/z =593 (M+H, ⁸¹Br)⁺ |
| **22** | 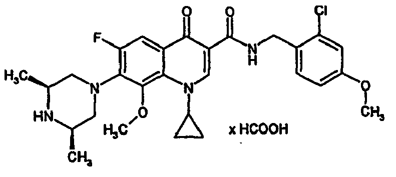 | HPLC (Methode 10): Rₜ = 4.27 min |
| | | MS (ESI): m/z = 545 (M+H)⁺ |
| **23** | 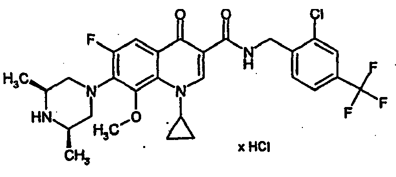 | LC-MS (Methode 1): Rₜ = 1.82 min |
| | | MS (ES+): m/z = 581 (M+H)⁺ |
| **24** | 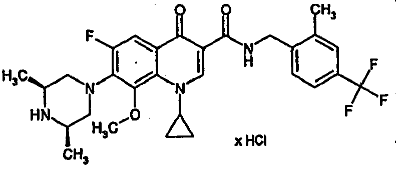 | LC-MS (Methode 3): Rₜ = 1.93 min MS (ES+): m/z = 561 (M+H)⁺ |
| | | ¹H-NMR (300 MHz, DMSO-d₆): δ = 0.98 (m, 2H), 1.12 (m, 2H), 1.28 (d, 6H), 2.41 (s, 3H) 3.15-3.56 (m 6H), 3.79 (s, 3H), 4.11 (m, 1H), 4.59 (d, 2H), 7.42 (d, 1H), 7.52 (d, 1H), 7.58(s, 1H), 7.75 (d, 1H), 8.72 (s, 1H), 10.18 (t, 1H) |
| **25** | 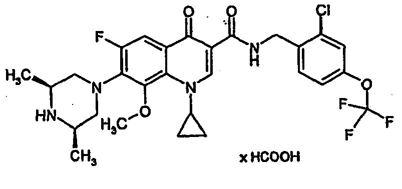 | HPLC (Methode 9): Rₜ = 4.60 min MS (ESI): m/z = 597 (M+H)⁺ |
| | | ¹H-NMR (400 MHz, CDCl₃): δ = 0.97 (m, 2H), 1.18 (m, 2H), 1.32 (d, 6H), 3.19-3.41 (m, 4H), 3.45 (d, 2H), 3.77 (s, 3H), 3.95 (m, 1H), 4.71 (d, 2H), 7.09 (d, 1H), 7.49 (d, 1H), 7.91 (d, 1H), 8.40 (s, 1H), 8.36. (s, 1H), 10.38 (t, 1H) |
| **26** | 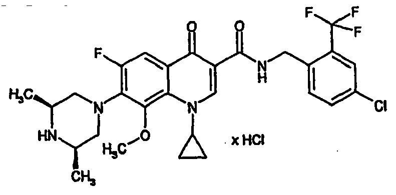 | LC-MS (Methode 3): Rₜ =1.72 min |
| | | MS (ES+): m/z = 581 (M+H)⁺ |
| **27** | 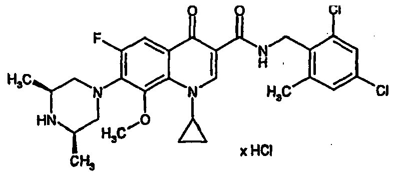 | LC-MS (Methode 3): Rₜ =1.91 min |
| | | MS (ES+): m/z = 561 (M+H)⁺ |

### Beispiel 28

### N-(2,4-Dichlorbenzyl)-7-[(3RS,5RS-3,5-dimethyl)-piperazin-1-yl]-6-fluor-1-(2-fluorethyl)-8-methoxy-4-oxo-1,4-dihydrochinolin-3-carbonsäureamid - Hydroformiat

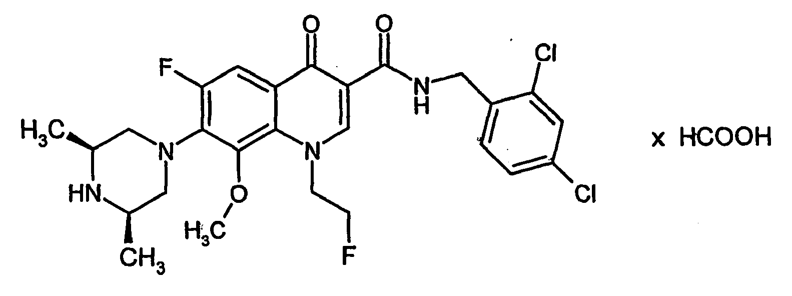

Die Herstellung erfolgt analog zu Beispiel 19 aus 7-(cis-3,5-Dimethylpiperazin-1-yl)-6-fluor-1-(2-fluorethyl)-8-methoxy-4-oxo-1,4-dihydrochinolin-3-carbonsäure (Herstellung siehe: EP 0241206) und 2,4-Dichlorbenzylamin.
HPLC (Methode 9): Rₜ = 4.46 min, MS (ESI) = 553 (M+H)⁺.
¹H-NMR (400 MHz, DMSO-d₆): δ = 1.15 (d, 6H), 2.88-3.07 (m, 2H), 3.11-3.56 (m, 4H unter dem Wassersignal des DMSO), 3.78 (s, 3H), 4.59 (d, 2H), 4.76 (dd, 2H), 4.95 (d, 2H), 7.35-7.50 (m, 2H), 7.64 (s, 1H), 7.83 (d,1H), 8.16 (s, 1H), 8.72 (s, 1H), 10.27 (t, 1H).

Analog zur Herstellungsvorschrift von Beispiel 19 werden aus verschiedenen Carbonsäuren und Benzylaminen die Beispiele 29 bis 51 hergestellt.

| **Beispiel-Nr.** | **Struktur** | **Edukt Bsp.-Nr.** | **Analytikdaten** |
|---|---|---|---|
| | | | **LC-MS (Methode)/ Meßwerte** |
| | | | **HPLC (Methode)/ Meßwert** |
| | | | **MS (Methode)/ Meßwert** |
| | | | **NMR- Spektrum** |
| **29** | 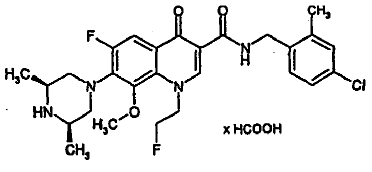 | Siehe unter Bsp.28 | HPLC (Methode 10): Rₜ = 4.33 min |
| | | | MS (ESI): m/z = 533 (M+H)⁺ |
| **30** | 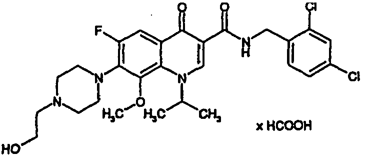 | 59A | HPLC (Methode 9): Rₜ = 4.42 min |
| | | | MS (ESI): m/z = 565 (M+H)⁺ |
| | | | ¹H-NMR (300 MHz, CDCl₃): δ = 1.52 (d, 6H), 2.98 (m, 2H), 3.08 (m, 4H), 3.62 (m, 4H), 3.79 (s, 3H), 3.91 (m, 2H), 4.70 (d, 2H), 5.76 (m, 1H), 7.21 (dd, 1H), 7.38 (s, 1H), 7.41 (d, 1H), 7.97 (d, 1H), 8.27 (s, 1H), 8.86 (s, 1H), 10.43 (t, 1H) |
| **31** | 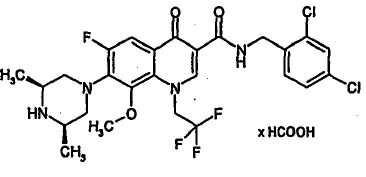 | 50A | HPLC (Methode 9): Rₜ = 4.47 min |
| | | | MS (ESI): m/z = 605 (M+H)⁺ |
| | | | ¹H-NMR (300 MHz, CDCl₃): δ = 1.33 (d, 6H), 3.21-3.45 (m, 6H), 3.82 (s, 3H), 4.70 (d, 2H), 5.25 (q, 2H), 7.21 (dd, 1H), 7.38 (d, 1H), 7.40 (d, 1H), 7.95 (d, 1H), 8.42 (s, 1H), 8.57 (s, 1H), 10.22 (t, 1H) |
| **32** | 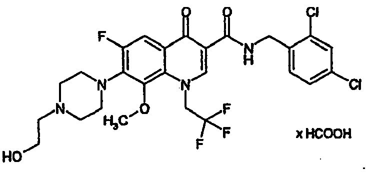 | 60A | HPLC (Methode 9): Rₜ = 4.46 min |
| | | | MS (ESI): m/z = 533 (M+H)⁺ |
| **33** | 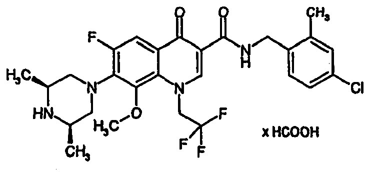 | 50A | HPLC (Methode 10): Rₜ = 4.52 min |
| | | | MS (ESI): m/z = 569 (M+H)⁺ |
| **34** | 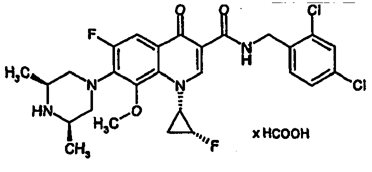 | 58A | LC-MS (Methode 1): Rₜ = 1.98 min |
| enantiomerenrein | | | MS (ES+): m/z = 565 (M+H)⁺ |
| **35** | 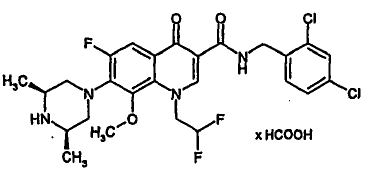 | 57A | HPLC (Methode 10): Rₜ = 4.57 min |
| | | | MS (ESI): m/z = 571 (M+H)⁺ |
| | | | ¹H-NMR (300 MHz, CDCl₃): δ = 1.35 (d, 6H), 3.31-3.48 (m, 6H), 3.85 (s, 3H), 4.1,9 (d, 2H), 4.83 (td, 2H), 6.04 (tt, 1H), 7.21 (dd, 1H), 7.37-7.42 (m, 2H), 7.98 (d, 1H), 8.41 (s, 1H), 8.59 (s, 1H), 10.26 (t, 1H) |
| **36** | 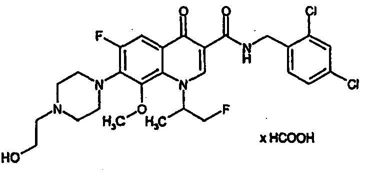 | 61A | LC-MS (Methode 1): Rₜ = 1.75 min |
| racemisch | | | MS (ES+): m/z= 583 (M+H)⁺ |
| **37** | 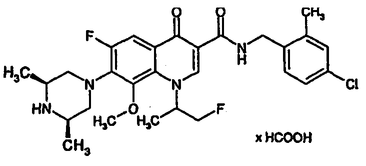 | 51A | LC-MS (Methode 1): Rₜ = 1.81 min |
| racemisch | | | MS (ES+): m/z = 547 (M+H)⁺ |
| **38** | 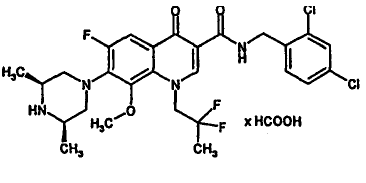 | 56A | LC-MS (Methode 2): Rₜ = 1.73 min |
| | | | MS (ES+): m/z = 565 (M+H)⁺ |
| | | | ¹H-NMR (400 MHz, DMSO-d₆): δ = 1.24 (d, 6H), 1.56 (t, 3H), 3.14 (m, 2H), 3.43 (m, 2H), 3.49 (m, 2H), 3.81 (s, 3H), 4.59 (d, 2H), 5.31 (t, 2H), 7.37-7.47 (m, 2H), 7.64 (s, 2H), 7.82 (d, 1H), 8.72 (s, 1H), 10.16 (t, 1H) |
| **39** | 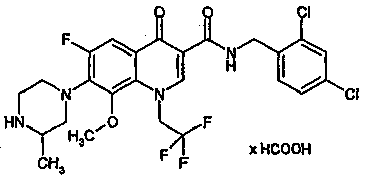 | 62A | LC-MS (Methode 3): Rₜ = 1.92 min |
| | | | MS (ES+): m/z = 575 (M+H)⁺ |
| **40** | 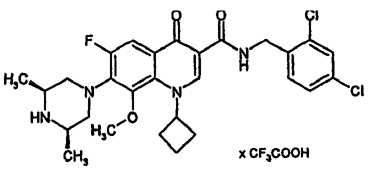 | 54A | LC-MS (Methode 1): Rₜ = 2.16 min |
| | | | MS (ES+): m/z = 561 (M+H)⁺ |
| | | | Reinigung (Methode 8) |
| **41** | 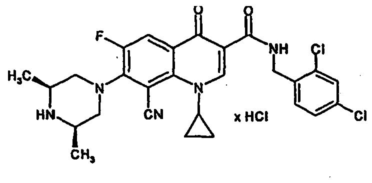 | 63A | LC-MS (Methode 1): Rₜ = 1.97 min |
| | | | MS (ES+): m/z = 542 (M+H)⁺ |
| | | | ¹H-NMR (400 MHz, DMSO-d₆): δ = 1.22 (m, 2H), 1.26-1.38 (m, 8H: darin 1.30 (d, 6H)), 3.41-3.57 (m, 4H), 3.79 (m, 2H), 4.15 (m, 1H), 4.59 (d, 2H), 7.36-7.46. (m, 2H), 7.64 (s, 1H), 8.19 (d, 1H), 8.70 (s, 1H), 10.09 (t, 1H) |
| **42** | 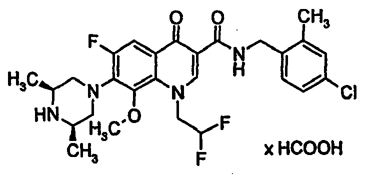 | 57A | HPLC (Methode 10): Rₜ = 4.48 min |
| | | | MS (ESI): m/z = 551 (M+H)⁺ |
| **43** | 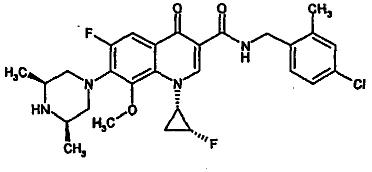 | 58A | LC-MS (Methode 3): Rₜ = 1.97 min |
| | | | MS (ES+): m/z = 545 (M+H)⁺ |
| | | | ¹H-NMR (400 MHz, DMSO-d₆): δ = 1.06 (d, 6H), 1.43-1.68 (m, 2H), 2.32 (s, 3H), 2.72-2.91 (m, 2H), 3.06 (m, 2H), 3.25 (m, 2H), 3.77 (s, 3H), 4.08 (m, 1H), 4.51 (d, 2H), 4.93/5.10 (2m, 1H), 7.19-7.32 (m, 3H), 7.71 (d, 1H), 8.68 (s, 1H), 10.08 (t, 1H) |
| **44** | 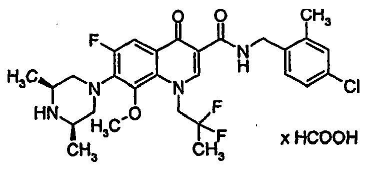 | 56A | LC-MS (Methode 2): Rₜ = 1.77 min |
| | | | MS (ES+): m/z = 585 (M+H)⁺ |
| **45** | 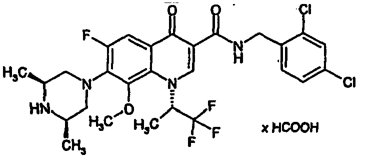 | 52A | HPLC (Methode 9): Rₜ = 4.75 min |
| (S)-Enantiomer | | | MS (ESI): m/z = 603 (M+H)⁺ |
| **46** | 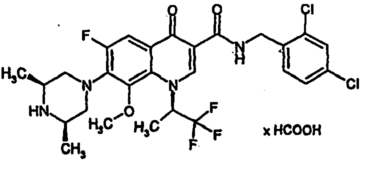 | 53A | LC-MS (Methode 2): Rₜ = 1.78 min |
| (R)-Enantiomer | | | MS (ES+): m/z = 603 (M+H)⁺ |
| **47** | 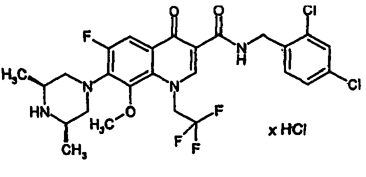 | 50A | HPLC (Methode 9): Rₜ = 4.58 min |
| HCl-Salz von Bsp. 31 | | | MS(ESI): m/z = 589 (M+H)⁺ |
| **48** | 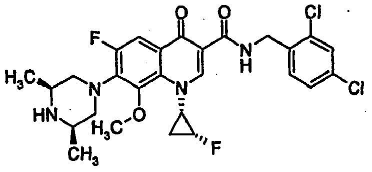 | 58A | LC-MS (Methode 3): Rₜ = 1.86 min |
| | | | MS (ES+): m/z = 565 (M+H)⁺ |
| **49** | 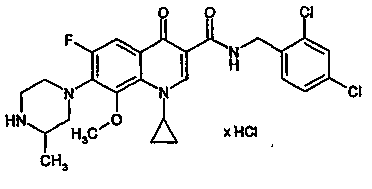 | Gatifloxacin | LC-MS (Methode 2): Rₜ = 2.05 min |
| | | | MS (ES+): m/z = 542 (M+H)⁺ |
| **50** | 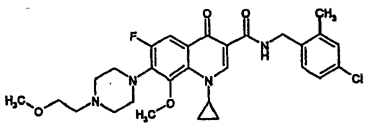 | 13A | LC-MS (Methode 2): Rₜ = 1.94 min |
| | | | MS (ES+): m/z = 557 (M+H)⁺ |
| **51** | 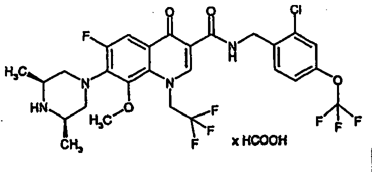 | 50A | HPLC (Methode 9): Rₜ = 4.71 min |
| | | | MS (ESI): m/z = 639 (M+H)⁺ |

### Beispiel 52

### 7-[(3RS,5SR)-3,5-Dimethylpiperazin-1-yl]-6-fluor-8-methoxy-N-[2-methyl-4-(trifluormethoxy)benzyl]-4-oxo-1-(2,2,2-trifluorethyl)-1,4-dihydrochinolin-3-carbonsäureamid

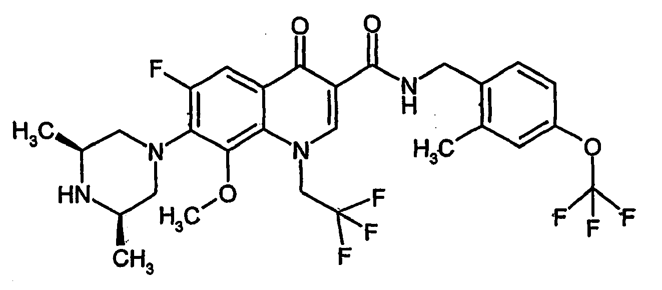

212 mg (0.49 mmol) 7-[(3RS,5SR)-3,5-Dimethylpiperazin-1-yl]-6-fluor-8-methoxy-4-oxo-1-(2,2,2-trifluorethyl)-1,4-dihydrochinolin-3-carbonsäure (Salzfreie Verbindung von dem Beispiel 50A) und 366 mg (0.98 mmol) 2-Methyl-4-trifluormethoxybenzylamin (Beispiel 69A) werden zusammen mit 357 mg (0.69 mmol) PyBOP und 30 mg (0.25 mmol) 4-Dimethylaminopyridin in 4 ml DMF gelöst und 12 h bei RT gerührt. Anschließend wird das Reaktionsgemisch durch präparative HPLC (Methode 7) gereinigt. Man erhält einen Feststoff. Ausbeute: 170 mg (56% d. Th.).
LC-MS (Methode 1): Rₜ = 2.00 min, MS (ES+): m/z = 619 (M+H)⁺
¹H-NMR (400MHz, DMSO-d₆): δ =10.0 (t, 1H), 8.85 (bs, 1H), 7.76 (d, 1H), 7.37 (d, 1H), 7.22 (m, 1H), 7.15-7.19 (m, 1H), 5.71 (q, 2H), 4.55 (d, 2H), 3.78 (s, 3H), 3.17-3.24 (m, 2H), 2.92-3.06 (m, 2H), 2.70-2.83 (m, 2H), 2.37 (s, 3H), 1.00 (d, 6H).

### Beispiel 53

### {(2RS,6SR)-4-[3-{[(2,4-Dichlorbenzyl)amino]carbonyl}-6-fluor-8-methoxy-4-oxo-1-(2,2,2-trifluorethyl)-1,4-dihydrochinolin-7-yl]-2,6-dimethylpiperazin-1-yl}acetamid

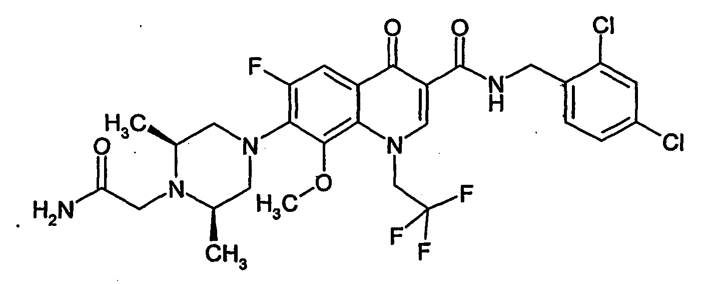

243 mg (0.41 mmol) N-(2,4-Dichlorbenzyl)-7-[(3RS,5SR)-3,5-dimethylpiperazin-1-yl]-6-fluor-8-methoxy-4-oxo-1-(2,2,2-trifluoroethyl)-1,4-dihydrochinolin-3-carbonsäureamid (freigesetzt aus dem Hydrochlorid der Verbindung aus Beispiel 47), 46.3 mg (0.49 mmol) Chloracetamid, 75 mg (0.45 mmol) Kaliumiodid und 143 mg (1.03 mmol) Kaliumcarbonat werden in 4 ml Acetonitril über Nacht unter Rückfluss gerührt. Nach Abkühlung wird die Mischung filtriert und über präparative HPLC (Methode 5) getrennt. Zur Feinreinigung wird das erhaltene Produkt in heißem Acetonitril verrührt, abgekühlt und abfiltriert. Nach Trocknung im Hochvakuum erhält man 46 mg (16% d. Th.) der Titelverbindung.
LC-MS (Methode 3): Rₜ = 2.05 min, MS (ES+): m/z = 646 (M+H)⁺.
¹H-NMR (400 MHz, CDCl₃): δ = 1.12 (d, 6H), 2.82 (m, 2H), 3.04 (m, 2H), 3.21 (m, 2H), 3.33 (m, 2H), 3.84 (s, 3H), 4.69 (d, 2H), 5.23 (m, 2H), 5.45 (s, 1H), 7.21 (m, 1H), 7.34-7.44 (m, 2H), 7.93 (d, 1H), 8.54 (s, 1H), 10.19 (m, 1H).

Analog zur Herstellung von Beispiel 53 werden aus den entsprechenden Piperazinen mit Elektrophilen die folgenden Beispiele 54 bis 75 hergestellt. Als Elektrophile werden Chloracetamid, N-Methyl-chloracetamid, N,N-Dimethyl-chloracetamid, N-Methylsulfonyl-chloracetamid (Herstellung: siehe DE 19937024), 2-Chlorpropionsäureamid, verschiedene Alkyl-chlormethylketone oder 3-Brom-propionsäureamid verwendet.

| **Beispiel-Nr.** | **Struktur** | **Edukt Bsp-Nr.** | **Analytikdaten** |
|---|---|---|---|
| | | | **LC-MS (Methode)/ Meßwert** |
| | | | **HPLC (Methode)/ Meßwert** |
| | | | **MS (Methode)/ messwert** |
| | | | **NMR-Spektrum** |
| **54** | 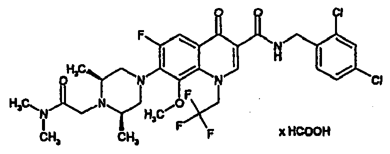 | 47 | HPLC (Methode 9): Rₜ = 4.77 min |
| | | | MS (ESI): m/z = 674 (M+H)⁺ |
| **55** | 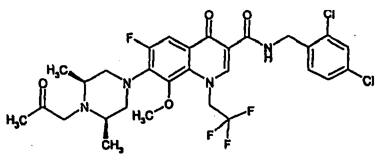 | 47 | HPLC (Methode 9): Rₜ = 4.74 min |
| | | | MS (ESI): m/z = 645 (M+H)⁺ |
| **56** | 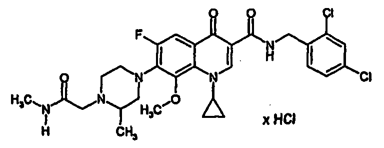 | 49 | LC-MS (Methode 3): Rₜ = 2.05 min |
| | | | MS (ES+): m/z = 604 (M+H)⁺ |
| **57** | 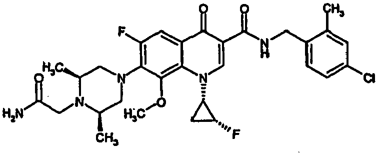 | 43 | LC-MS (Methode 1): Rₜ = 2.02 min |
| | | | MS (ES+): m/z = 603 (M+H)⁺ |
| **58** | 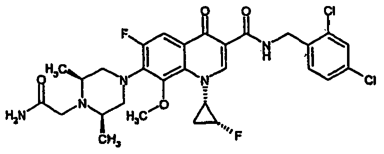 | 48 | LC-MS (Methode 1): Rₜ = 1.91 min |
| | | | MS (ES+): m/z = 622 (M+H)⁺ |
| **59** | 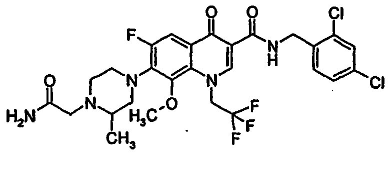 | 39 | LC-MS (Methode 2): Rₜ = 1.83 min |
| | | | MS (ES+): m/z = 632 (M+H)⁺ |
| **60** | 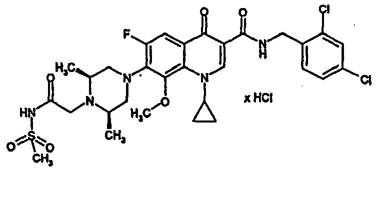 | 12 | LC-MS (Methode 3): Rₜ = 2.22 min |
| | | | MS (ES+): m/z = 542 (M+H)⁺ |
| | | | ¹H-NMR (400 MHz, DMSO-d₆): δ = 0.97 (m, 2H), 1.11 (m, 2H), 1.28 (d, 6H), 3.12-3.33 (m, 5H: darin 1.29 (s, 3H)), 3.75 (m, 2H), 3.79 (s, 3H), 4.09 (m, 1H), 4.15 (m, 2H), 4.58 (d, 2H), 7.33-7.48 (m, 2H), 7.63 (s, 2H), 7.78 (d, 1H), 8.68 (s, 1H), 10.22 (t, 1H) |
| **61** | 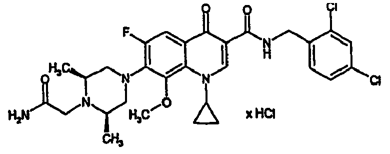 | 12 | LC-MS (Methode 2): Rₜ = 1.86 min |
| | | | MS (ES+): m/z = 604 (M+H)⁺ |
| **62** | 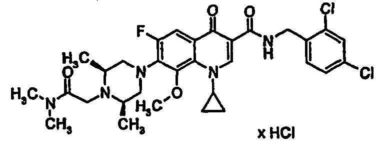 | 12 | LC-MS (Methode 3): Rₜ = 1.91 min |
| | | | MS (ES+): m/z = 632 (M+H)⁺ |
| **63** | 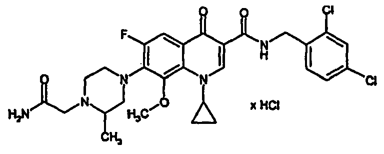 | 49 | LC-MS (Methode 1): Rₜ = 1.97 min |
| | | | MS (ES+): m/z = 542 (M+H)⁺ |
| **64** | 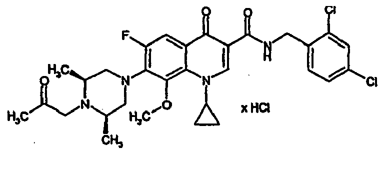 | 12 | LC-MS (Methode 2): Rₜ = 1.67 min |
| | | | MS (ES+): m/z = 590 (M+H)⁺ |
| | | | ¹H-NMR (300 MHz, CDCl₃): δ = 0.96 (m, 2H), 1.17 (m, 2H), 1.49 (d, 6H), 2.32 (s, 3H), 3.42-3.58 (m, 2H), 3.81 (s, 3H), 3.95 (m, 1H), 4.0-4.15 (m, 2H), 4.23 (m, 2H), 4.31 (m, 2H), 4.68 (d, 2H), 7.21 (dd, 1H), 7.39 (m, 2H), 7.94 (d, 1H), 8.85 (s, 1H), 10.31 (t, 1H), 13.08 (bs, 1H) |
| **65** | 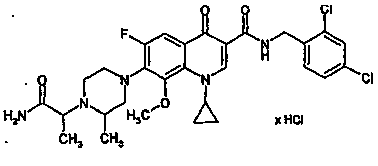 | 49 | LC-MS (Methode 2): Rₜ = 1.89 min |
| | | | MS (ES+): m/z = 604 (M+H)⁺ |
| **66** | 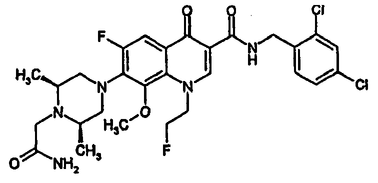 | 28 | HPLC (Methode 9): Rₜ = 4.65 min |
| | | | MS (ESI+): m/z = 610 (M+H)⁺ |
| **67** | 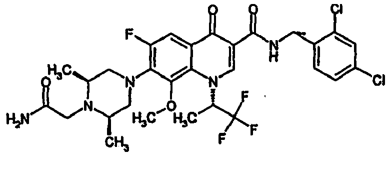 | 45 | HPLC (Methode 9): Rₜ = 4.38 min |
| | | | MS (ESI+): m/z = 660 (M+H)⁺ |
| **68** | 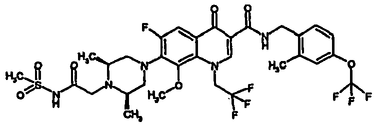 | 52 | LC-MS (Methode 1): Rₜ = 2.34 min |
| | | | MS (ES+): m/z = 754 (M+H)⁺ |
| **69** | 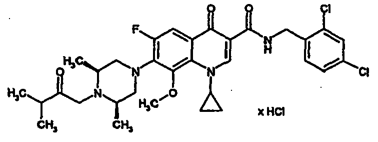 | 12 | LC-MS (Methode 3): Rₜ = 2.07 min |
| | | | MS (ES+): m/z = 631 (M+H)⁺ |
| **70** | 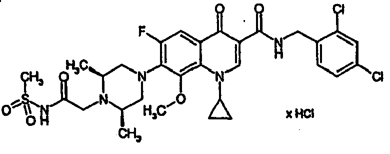 | 47 | HPLC (Methode 9): Rₜ = 4.66 min |
| | | | MS (ES+): m/z = 724 (M+H)⁺ |
| **71** | 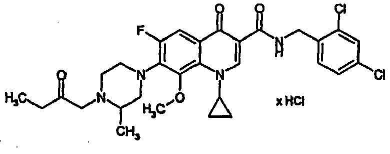 | 49 | LC-MS (Methode 3): Rₜ =1.95 min |
| | | | MS (ES+): m/z = 603 (M+H)⁺ |
| **72** | 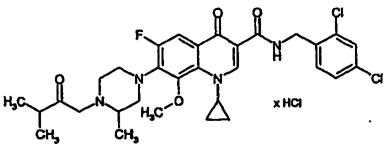 | 49 | LC-MS (Methode 3): Rₜ = 2.04 min |
| | | | MS (ES+): m/z = 617 (M+H)⁺ |
| **73** | 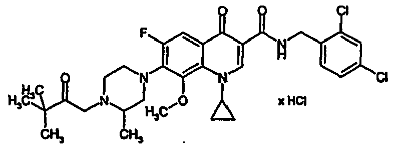 | 49 | LC-MS (Methode 3): Rₜ = 2.07 min |
| | | | MS (ES+): m/z = 631 (M+H)⁺ |
| **74** | 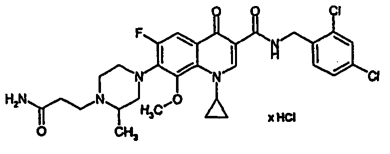 | 49 | LC-MS (Methode 3): Rₜ =1.85 min |
| | | | MS (ES+): m/z = 604 (M+H)⁺ |
| **75** | 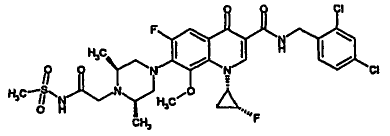 | 48 | LC-MS (Methode 2): Rₜ = 2.01 min |
| | | | MS (ES+): m/z = 700 (M+H)⁺ |

### Beispiel 76

### N-Ethyl-{4-[1-cyclopropyl-3-{[(2,4-dichlorbenzyl)amino]carbonyl}-6-fluor-8-methoxy-4-oxo-1,4-dihydrochinolin-7-yl]-(2RS,6SR)-2,6-dimethylpiperazin-1-yl}acetamid-Hydrochlorid

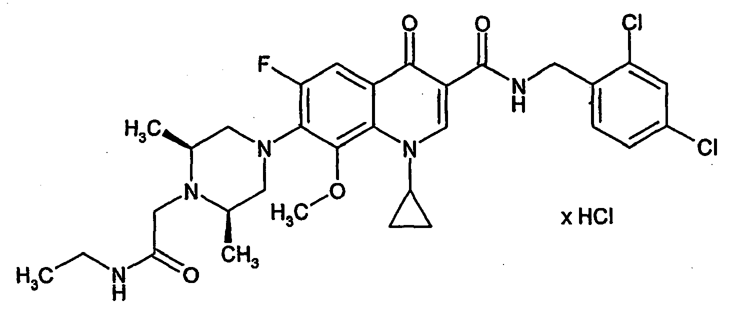

In 2 ml DMF werden 50 mg der Verbindung aus Beispiel 84A vorgelegt. Man versetzt mit einer Ethylamin-Lösung (2M in THF), mit 103 mg PyBOP und 35 µl Hünigsbase und lässt 24 h bei RT rühren. Die gesamte Reaktionsmischung wird durch präparative HPLC (Methode 6) getrennt. Man erhält 36 mg (69% d. Th.) der Titelverbindung.
LC-MS (Methode 3): Rₜ = 2.17 min, MS (ES+): m/z = 632 (M+H)⁺
¹H-NMR (400 MHz, DMSO-d₆): δ = 0.97 (m, 2H), 1.05-1.14 (m, 5H), 1.33 (d, 6H), 3.20 (m, 2H), 3.49 (d, 2H), 3.60 (t, 2H), 3.74-3.80 (m, 5H, darunter 3.78 (s, 3H)), 4.02-4.10 (m, 3H), 4.58 (d, 2H), 7.37-7.43 (m, 2H), 7.52 (s, 1H), 7.77 (d, 1H), 8.69 (s, 1H), 10.22.(t, 1H).

Analog zu Beispiel 76 werden mit den entsprechenden Aminen folgende Verbindungen erhalten:

| **Beispiel-Nr.** | **Struktur** | **Analytikdaten** |
|---|---|---|
| | | **LC-MS (Methode)/ Meßwert** |
| | | **HPLC (Methode)/ Meßwert** |
| | | **MS (Methode)/ Meßwert** |
| **77** | 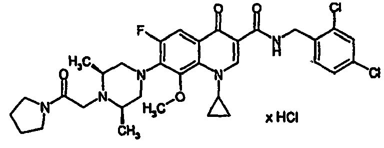 | LC-MS (Methode 3): Rₜ = 2.05 min |
| | | MS-(ES+):m/z=658 (M+H)⁺ |
| **78** | 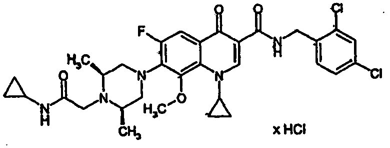 | LC-MS Methode 3): Rₜ = 2.10 min |
| | | MS (ES+): m/z = 644 (M+H)⁺ |

### Beispiel 79

### 1-Cyclopropyl-N-(2,4-dichlorbenzyl)-6-fluor-7-[(3RS,5SR)-4-glycyl-3,5-dimethylpiperazin-1-yl]-8-methoxy-4-oxo-1,4-dihydrochinolin-3-carbonsäureamid - Hydrochlorid

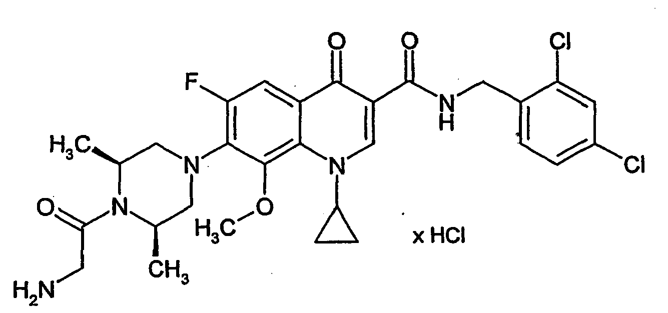

46.0 mg (0.07 mmol) der Verbindung aus Beispiel 82A werden in 2 ml Tetrahydrofuran vorgelegt, die Lösung- auf 0°C gekühlt, 21.1 mg (0.08 mmol) Triphenylphosphin, gelöst in 1 ml Tetrahydrofuran, zugetropft, auf Raumtemperatur erwärmt und es wird über Nacht bei dieser Temperatur nachgerührt. Zur Aufarbeitung wird das Lösungsmittel vollständig am Rotationsverdampfer entfernt, der Rückstand über die präparative RP-HPLC (Methode 6) vorgereinigt und nach der Feinreinigung über Kieselgel 60 mittels Säulenchromatographie (Laufmittel: Dichlormethan: Ethanol 90:10) und einengen der Fraktionen unter Zusatz von Salzsäure werden 24 mg der Zielverbindung erhalten.
LC-MS (Methode 1): Rₜ = 1.86 min, MS (ES+): m/z = 604 (M+H)⁺
¹H-NMR (400 MHz, DMSO-d₆): δ = 0.95 (m, 2H), 1.09 (m, 2H), 1.38 (d, 3H), 1.46 (d, 3H), 3.39 (m, 2H), 3.57 (s, 2H), 3.70 (s, 3H), 3.75 (m, 1H), 4.03 (m, 1H), 4.10 (m, 2H), 4:55 (m, 1H), 4.59 (d, 2H), 7.37-7.47 (m, 2H), 7.65 (d, 1H), 7.78 (d, 1H), 8.05 (m, 2H), 8.70 (s, 1H), 10.25 (t, 1H).

### Beispiel 80

### N-(2,4-Dichlorbenzyl)-6-fluor-7-[(3RS,5SR)-4-glycyl-3,5-dimethylpiperazin-1-yl]-8-methoxy-1-(2,2,2-trifluorethyl)-4-oxo-1,4-dihydrochinolin-3-carbonsäureamid - Hydrochlorid

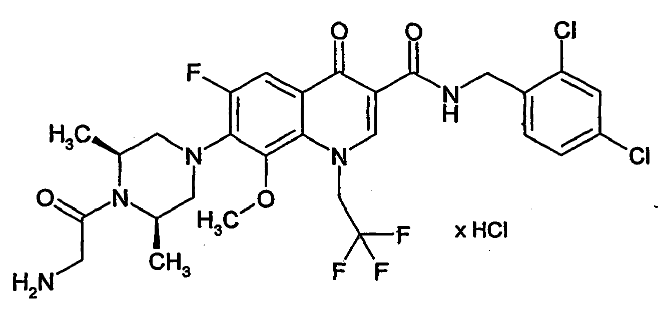

Die Titelverbindung wird analog zu Beispiel 79 aus der Verbindung aus Beispiel 83A hergestellt.
LC-MS (Methode 3): Rₜ = 2.03 min; MS (ES+): m/z = 646 (M+H)⁺.

### Beispiel 81

### 7-[(3RS,5SR)-4-(Azetidin-1-ylacetyl)-3,5-dimethylpiperazin-1-yl]-1-cyclopropyl-N-(2,4-dichlorbenzyl)-6-fluor-8-methoxy-4-oxo-1,4-dihydrochinolin-3-carbonsäureamid

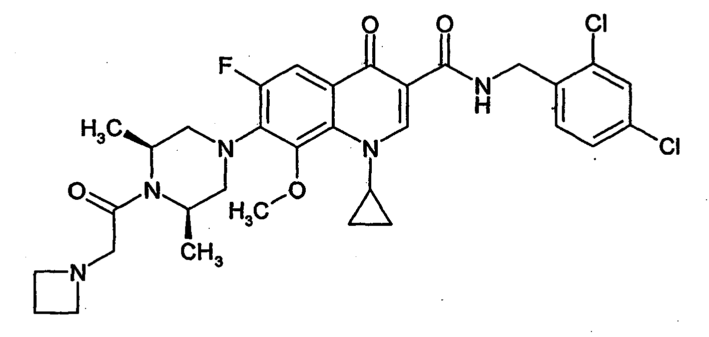

50 mg (0.08 mmol) der Verbindung aus Beispiel 80A und 22.9 mg (0.40 mmol) Azetidin werden in 3 ml Ethanol im geschlossenen Reaktionsgefäß über Nacht bei 90°C gerührt. Zur Aufarbeitung wird das Lösungsmittel vollständig am Rotationsverdampfer entfernt und der Rückstand wird über die präparative RP-HPLC (Methode 6) gereinigt. Es wird die Zielverbindung mit 24 mg erhalten.
LC-MS (Methode 1): Rₜ = 1.97 min, MS (ES+): m/z = 644 (M+H)⁺
¹H-NMR (400 MHz, DMSO-d₆): δ = 0.95 (m, 2H), 1.08 (m, 2H), 1.37 (d, 3H), 1.48(d, 3H), 2.23-2.58 (m, 2H), 3.22-3.49 (m, 4H), 3.70 (s, 3H), 3.87 (m, 1H), 3.95-4.38 (m, 6H), 4.45-4.67 (m, 4H: darin 4.58 (d, 2H)), 7.35-7.47 (m, 2H), 7.64 (d, 1H), 7.78 (d, 1H), 8.69 (s, 1H), 10.25 (t, 1H).

Analog zu Beispiel 81 werden die folgenden Beispiele 82 bis 84 hergestellt:

| **Beispiel-Nr.** | **Struktur** | **Analytik** |
|---|---|---|
| | | **LC-MS (Methode)/ Meßwert** |
| | | **MS (Methode)/ Meßwert** |
| **82** | 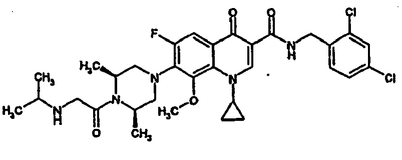 | LC-MS (Methode 1): Rₜ = 1.95 min |
| | | MS (ES+): m/z = 646 (M+H)⁺ |
| **83** | 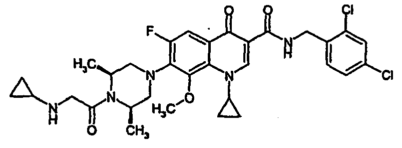 | LC-MS (Methode 1): Rₜ = 1.95 min |
| | | MS (ES+): m/z = 644 (M+H)⁺ |
| **84** | 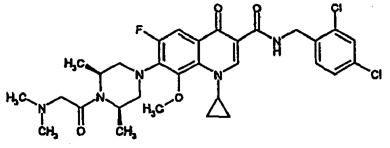 | LC-MS (Methode 1): Rₜ = 1.91 min |
| | | MS (ES+): m/z=632 (M+H)⁺ |

### Beispiel 85

### 8-Chlor-1-cyclopropyl-N-(2,4-dichlorbenzyl)-7-[4-acetyl-piperazin-1-yl]-6-fluor-4-oxo-1,4-dihydrochinolin-3-carbonsäureamid

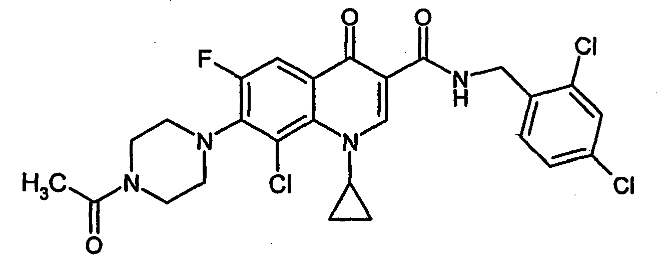

45.6 mg (0.1 mmol) 8-Chlor-1-cyclopropyl-N-(2,4-dichlorbenzyl)-6,7-difluor-4-oxo-1,4-dihydrochinolin-3-carbonsäureamid (Beispiel 79A) werden in 400 µl DMF mit 28 µl (0.2 mmol) Triethylamin und 12.8 mg (0.1 mmol) 1-Acetylpiperazin versetzt. Es wird 14 h bei 100°C gerührt, filtriert und mittels präparativer LC-MS (Methode 12) aufgereinigt.

Analog zu Beispiel 85 werden die in der folgenden Tabelle aufgeführten Beispiele 86 bis 88 hergestellt.

| **Beispiel-Nr.** | **Struktur** | **Analytikdaten** |
|---|---|---|
| | | **LC-MS (Methode)/ Meßwert** |
| **86** | 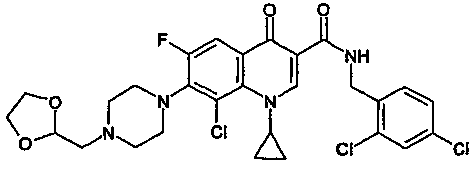 | LC-MS (Methode 11): Rₜ = 1.73 min |
| | | MS (ES+): m/z = 606 (M+H)⁺ |
| **87** | 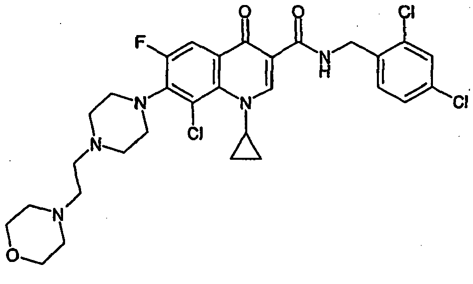 | LC-MS (Methode 11): Rₜ = 1.67 min |
| | | MS (ES+): m/z = 636 (M+H)⁺ |
| **88** | 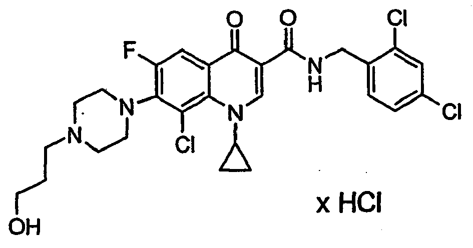 | LC-MS (Methode 2): Rₜ = 1.66 min |
| (in DMSO bei 120°C statt in DMF hergestellt) | | MS (ES+): m/z = 581 (M+H)⁺ |

### Beispiel 89

### 1-Cyclopropyl-N-(2,4-dichlorbenzyl)-6-fluor-7-[4-(2-hydroxy-2-methyl-propyl)-3-methylpiperazin-1-yl]-8-methoxy-4-oxo-1,4-dihydrochinolin-3-carbonsäureamid-Hydrochlorid

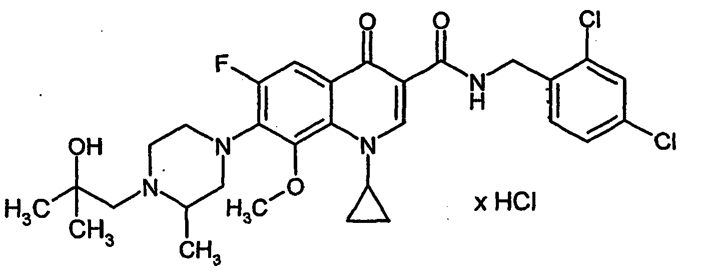

100 mg 1-Cyclopropyl-N-(2,4-dichlorbenzyl)-6-fluor-8-methoxy-7-(3-methylpiperazin)-4-oxo-1,4-dihydrochinolin-3-carbonsäureamid (freie Base von Beispiel 49), werden mit 152 mg Isobutylenoxid (2 Äq.) und 75 mg Lithiumperchlorat (4 Äq.) über Nacht in 10 ml Acetonitril unter Rückfluss gerührt. Die Reaktionsmischung wird nach Abkühlung direkt über die präparative RP-HPLC (Methode 6) gereinigt.
LC-MS (Methode 2): Rₜ =1.62 min, MS (ESI): m/z = 605 (M+H)⁺

### Beispiel 90

### 1-Cyclopropyl-N-(2,4-dichlorbenzyl)-7-[(3RS,5SR)-3,5-dimethyl-4-(2-hydroxy-2-methyl-propyl)-piperazin-1-yl]-6-fluor-8-methoxy-4-oxo-1,4-dihydrochinolin-3-carbonsäureamid - Hydrochlorid

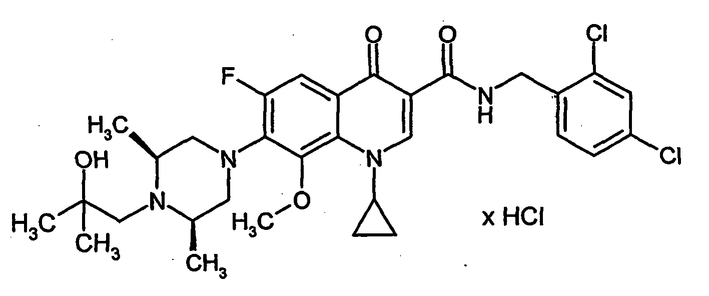

Die Verbindung wird analog zu Beispiel 89 aus der Verbindung aus Beispiel 12 und 1,2-Epoxy-2-methyl-propan hergestellt.
LC-MS (Methode 3): Rₜ =1.95 min, MS (ES+): m/z = 619 (M+H)⁺

### Beispiel 91

### 1-Cyclopropyl-N-(2,4-dichlorbenzyl)-7-{(3RS;5SR)-4-[(2R)-2,3-dihydroxypropyl]-3,5-dimethylpiperazin-1-yl}-6-fluor-8-methoxy-4-oxo-1,4-dihydrochinolin-3-carbonsäureamid - Hydrochlorid

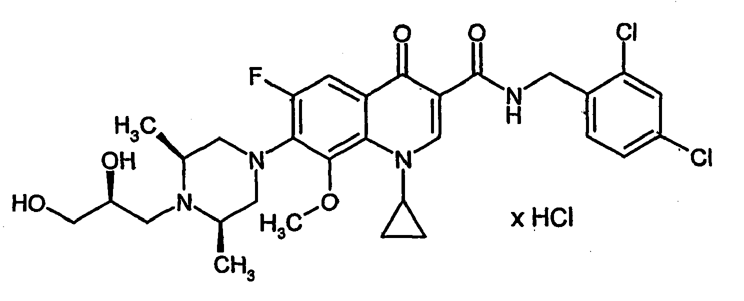

Die Titelverbindung wird aus der Verbindung aus Beispiel 12 analog zu Beispiel 89 mit (2R)-3-Butanoyloxy-1,2-epoxypropan und anschließender Hydrolyse des Butyrats mit 1 Äquivalent 1M Lithiumhydroxid-Lösung bei 70°C für 1 h hergestellt. Zur Aufarbeitung wird das Lösungsmittel einrotiert, der Rückstand mit 1N Salzsäure und Puffer pH 7 neutral gestellt und mit Dichlormethan extrahiert. Die Reinigung erfolgt durch RP-HPLC (Methode 6).
LC-MS (Methode 3): Rₜ =1.77 min, MS (ES+): m/z = 621 (M+H)⁺
¹H-NMR (400 MHz, DMSO-d₆): δ = 0.96 (m, 2H), 1.12 (m, 2H), 1.39 (d, 3H), 1.45 (d, 3H), 3.22-3.38 (m, 3H), 3.39-3.78 (m, 7H), 3.80 (s, 3H), 3.85 (m, 1H), 4.02 (m, 1H), 4.11 (m, 1H), 4.58 (d, 2H), 7.38 (d, 1H), 7.42 (dd, 1H), 7.63 (d, 1H), 7.76 (d, 1H), 8.69 (s, 1H), 10.22 (t, 1H), 10.61 (bs, 1H).

Analog zu Beispiel 91 wird das folgende Beispiele 92 hergestellt.

| **Beispiel-Nr.** | **Struktur** | **Edukt Beispiel-Nr.** | **Analytikdaten** |
|---|---|---|---|
| | | | **LC-MS (Methode)/ Meßwerte** |
| **92** | 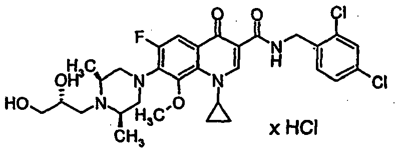 | 12 | LC-MS (Methode 3): Rₜ = 1.86 min |
| | | | MS (ES+): m/z = 621 (M+H)⁺ |

### Beispiel 93

### 7-[(3RS,5SR)-4-(3-Amino-3-oxopropyl)-3,5-dimethylpiperazin-1-yl]-6-fluor-8-methoxy-N-[2-methyl-4-(trifluormethoxy)benzyl]-4-oxo-1-(2,2,2-trifluorethyl)-1,4-dihydrochinolin-3-carbonsäureamid - Hydroformiat

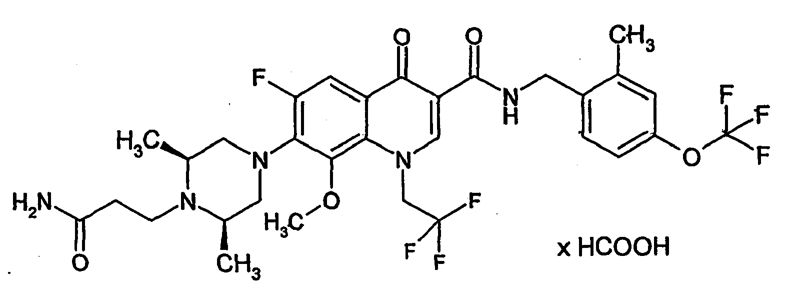

55 mg der Verbindung aus Beispiel 52, 18 mg Acrylamid und 35 mg Lithiumperchlorat werden bei RT mit ein paar Tropfen Acetonitril versetzt, so dass eine rührbare Suspension entsteht. Es wird dann auf 70°C über Nacht erhitzt und man lässt abkühlen. Nach Zusatz von DMSO wird die gesamte Mischung über präparative HPLC (Methode 5) getrennt. Nach dem Einengen der geeigneten Fraktionen und Trocknung im Hochvakuum werden 30 mg (40% d. Th.) der Titelverbindung erhalten.
LC-MS (Methode 1): Rₜ = 2.00 min, MS (ES+) = 690 (M+H)⁺.

### Beispiel 94

### 1-Cyclopropyl-7-{(3RS,5SR)-4-[(cyclopropylamino)carbonyl]-3,5-dimethylpiperazin-1-yl}-N-(2,4-dichlorbenzyl)-6-fluor-8-methoxy-4-oxo-1,4-dihydrochinolin-3-carbonsäureamid

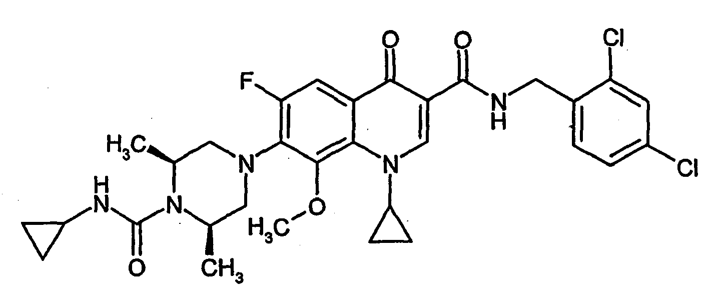

30 µl (24.3 mg, 0.29 mmol) Cyclopropylisocyanat werden in Dichlormethan gelöst, 80.0 mg (0.146 mmol) der Verbindung aus Beispiel 12 dazugegeben und es wird über Nacht bei RT nachgerührt. Zur Aufarbeitung wird das Lösungsmittel vollständig entfernt und nach der Feinreinigung über die präparative RP-HPLC (Methode 6) werden 55 mg der Zielverbindung erhalten.
LC-MS (Methode 3): Rₜ = 2.97 min, MS (ES+) = 630 (M+H)⁺.
¹H-NMR (400 MHz, DMSO-d₆): δ = 0.41 (m, 2H), 0.55 (m, 2H), 0.94 (m, 2H), 1.09 (m, 2H), 1.28 (d, 6H), 3.22 (m, 2H), 3.32 (m, 2H), 3.69 (s, 3H), 4.11 (m, 4H), 4.58 (d, 2H), 6.49 (bs, 1H), 7.39 (d, 1H), 7.43 (dd, 1H), 7.63 (d, 1H), 7.74 (d, 1H), 8.68 (s, 1H), 10.25 (t, 1H).

Analog zu Beispiel 94 werden die folgenden Beispiele 95 bis 97 hergestellt.

| **Beispiel-Nr.** | **Struktur** | **Analytikdaten** |
|---|---|---|
| | | **LC-MS (Methode)/ Meßwert** |
| | | **MS (Methode)/ Meßwert** |
| **95** | 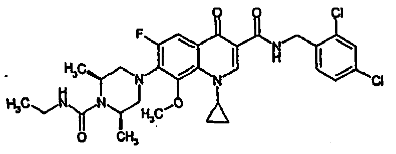 | LC-MS (Methode 1): Rₜ = 2.81 min |
| | | MS (ES+): m/z = 618 (M+H)⁺ |
| **96** | 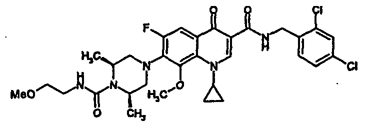 | LC-MS (Methode 1): Rₜ = 2.73 min |
| | | MS (ES+): m/z = 648 (M+H)⁺ |
| **96 97** | 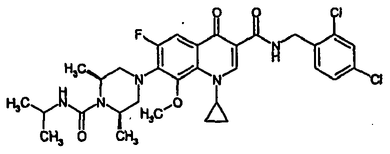 | LC-MS (Methode 1): Rₜ = 2.96 min |
| | | MS (ES+): m/z = 632 (M+H)⁺ |

### Beispiel 98

### 6-Fluor-7-[(3RS,5SR)-4-glycyl-3,5-dimethylpiperazin-1-yl]-8-methoxy-N(2-methy1-4-trifluormethoxy)-1-(2,2,2-trifluorethyl)-4-oxo-1,4-dihydrochinolin-3-carbonsäureamid - Hydrochlorid

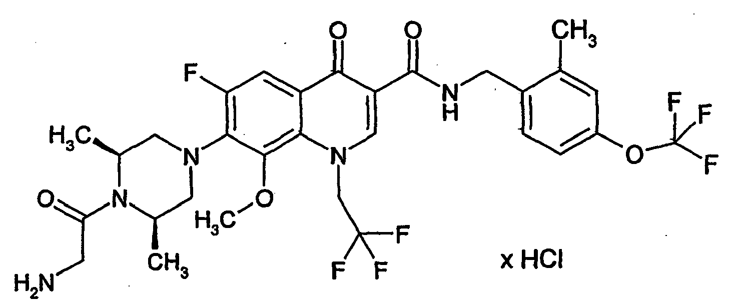

Die Titelverbindung wird analog zu Beispiel 79 aus der Verbindung aus Beispiel 87A hergestellt.
LC-MS (Methode 13): Rₜ = 3.51 min; MS (ES+): m/z = 676 (M+H)⁺.

### Beispiel 99

### N-(2,4-Dichlorbenzyl)-7-{(3RS,5SR)-3,5-dimethyl-4-[(5-oxo-4,5-dihydro-1H-1,2,4-triazol-3-yl)methyl]piperazin-1-yl}-6-fluor-8-methoxy-4-oxo-1-(2,2,2-trifluorethyl)-1,4-dihydrochinolin-3-carbonsäureamid

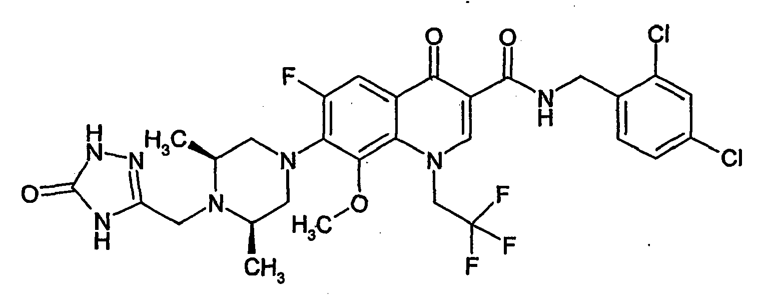

50.0 mg (0.09 mmol) der freien Base der Verbindung aus Beispiel werden in Acetonitril vorgelegt, mit 17.0 mg (0.13 mmol) 5-(Chlormethyl)-2,4-dihydro-3H-1,2,4-triazol-3-on (Herstellung: siehe Cowden, Camaron J.; Tetrahedron Lett;, 41 (44), 2000; 8661-8665), 16.9 mg (0.10 mmol) Kaliumiodid und 35.2 mg (0.25 mmol) Kaliumcarbonat versetzt, und es wird über Nacht bei 50°C gerührt. Zur Aufarbeitung wird die abgekühlte Reaktionsmischung über Kieselgur filtriert, mit Acetonitril und Dichlormethan/Methanol (10/1) nachgespült, das Filtrat am Rotationsverdampfer entfernt und aus dem erhaltenen Rückstand wird nach der Feinreinigung über Kieselgel 60 (Eluent: Dichlormethan/Ethanol 100/1 → 50/1 → 20/1 → 10/1) 23 mg (40% d. Th.) Produkt erhalten.
LC-MS (Methode 1): Rₜ = 2.10 min, MS (ES+): m/z = 686 (M+H)⁺;
¹H-NMR (400 MHz, DMSO-d₆): δ = 1.09 (d, 6H), 2.81 (m, 2H), 2.95 (m, 2H), 3.22 (m, 2H), 3.76 (s, 3H), 4.59 (d, 2H), 5.69 (q, 2H), 7.39 (d, 1H), 7.43 (dd, 1H), 7.64 (d, 1H), 7.76 (d, 1H), 8.82 (s, 1H), 10.12 (t, 1H), 11.22 (s, 1H), 11.28 (s, 1H).

### Beispiel 100

### 1-Cyclopropyl-N-(2,4-dichlorbenzyl)-6-fluor-8-methoxy-7-[3-methyl-4-(2-{[(meihylamino)-carbonyl]amino}-2-oxoethyl)piperazin-1-yl]-4-oxo-1,4-dihydrochinolin-3-carbonsäuremid Hydrochlorid

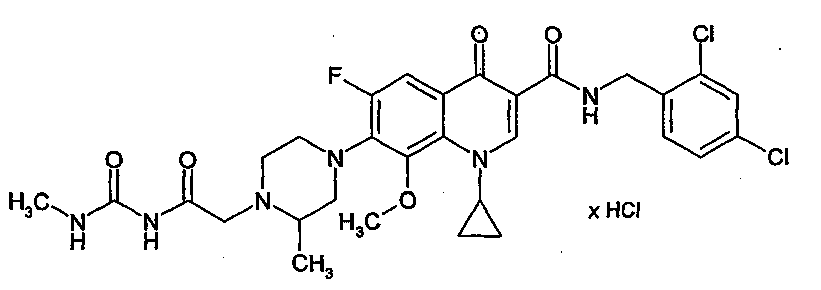

Analog der Vorschrift von Beispiel 99 wird die Titelverbindung aus 80.0 mg (0.14 mmol) der Verbindung aus Beispiel 49 und 25.4 mg (0.17 mmol) 2-Chlor-N-[(methylamino)carbonyl]-acetamid (Herstellung: siehe Patent DE 167138) mit 60 mg (62% d. Th.) erhalten.
LC-MS (Methode 1): Rₜ = 2.27 min, MS (ES+): m/z = 647 (M+H)⁺;
¹H-NMR (300 MHz, DMSO-d₆): δ = 0.95 (m, 2H), 1.11 (m, 2H), 1.32 (m, 3H), 2.72 (d, 2H), 3.27-3.95 (m, 9H: darin 3.79 (s, 3H)), 4.11 (m, 1H), 4.25 (m, 1H), 4.41 (m, 1H), 4:58 (d, 2H), 7.38 (d, 1H), 7.43 (dd, 1H), 7.64 (d, 1H), 7.71-7.98 (m, 2H: darin 7.78 (d, 1H)), 8.79 (s, 1H), 10.22 (t, 1H), 10.81 (s, 1H).

Analog zu Beispiel 91 werden die folgenden Beispiel 101 und 102 hergestellt.

| **Beispiel-Nr.** | **Struktur** | **Edukt Beispiel-Nr.** | **Analytikdaten** |
|---|---|---|---|
| | | | **LC-MS (Methode)/ Meßwerte** |
| | | | **NMR-Spektrum** |
| **101** | 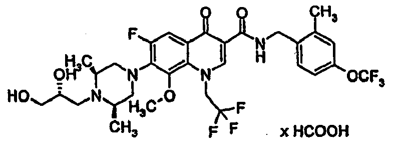 | 52 | LC-MS (Methode 3): Rₜ = 1.98 min |
| | | | MS (ES+): m/z = 693 (M+H)⁺ |
| | | | ¹H-NMR (400 MHz, CDCl₃): δ = 1.33 (d, 3H), 2.40 (s, 3H), 2.85 (br.d, 1H), 3.05-3.22 (m, 3H), 3.28-3.47 (m, 4H), 3.57 (dd, 1H), 3.76 (dd, 1H), 3.81 (s, 3H), 3.98 (m,1H), 4.52 (d, 2H), 5.23 (q, 2H), 7.02 (d, 1H), 7.03 (s, 1H), 7.37 (d, 1H), 7.91 (d, 1H), 8.25 (s, 1H, HCOOH), 8:60 (s, 1H), 10.2 (t, 1H). |
| **102** | 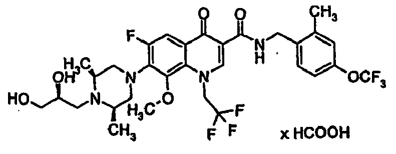 | 52 | LC-MS (Methode 3): Rₜ = 1.97 min |
| | | | MS (ES+): m/z = 693 (M+H)⁺ |

### B. Bewertung der physiologischen Wirksamkeit

Die *in vitro-*Wirkung der erfindungsgemäßen Verbindungen kann in folgenden Assays gezeigt werden:

### Anti-HCMV-(Anti-Humanes Cytomegalo-Virus) Zytopathogenitätstests

Die Testverbindungen werden als 50 millimolare (mM) Lösungen in Dimethysulfoxid (DMSO) eingesetzt. Ganciclovir^{®}, Foscarnet^{®} und Cidofovir^{®} dienen als Referenzverbindungen. Nach der Zugabe von jeweils 2 µl der 50, 5, 0.5 und 0.05 mM DMSO-Stammlösungen zu je 98 µl Zellkulturmedium in der Reihe 2 A-H in Doppelbestimmung werden 1:2-Verdünnungen mit je 50 µl Medium bis zur Reihe 11 der 96-Well-Platte durchgeführt. Die Wells in den Reihen 1 und 12 enthalten je 50 µl Medium. In die Wells werden dann je 150 µl einer Suspension von 1 x 10⁴ Zellen (humane Vorhautfibroblasten [NHDF]) pipettiert (Reihe 1 = Zellkontrolle) bzw. in die Reihen 2-12 ein Gemisch von HCMV-infizierten und nichtinfizierten NHDF-Zellen (M.O.I. = 0.001 - 0.002), d.h. 1-2 infizierte Zellen auf 1000 nicht-infizierte Zellen. Die Reihe 12 (ohne Substanz) dient als Viruskontrolle. Die End-Testkonzentrationen liegen bei 250 - 0.0005 µM. Die Platten werden 6 Tage bei 37°C / 5% CO₂ inkubiert, d.h. bis in den Viruskontrollen alle Zellen infiziert sind (100% cytopathogener Effekt [CPE]). Die Wells werden dann durch Zugabe eines Gemisches von Formalin und Giemsa's Farbstoff fixiert und gefärbt (30 Minuten), mit aqua bidest. gewaschen und im Trockenschrank bei 50°C getrocknet. Danach werden die Platten mit einem Overhead-Mikroskop (Plaque Multiplier der Firma Technomara) visuell ausgewertet.

Die folgenden Daten können von den Testplatten ermittelt werden:
CC₅₀ (NHDF) = Substanzkonzentration in µM, bei der im Vergleich zur unbehandelten Zellkontrolle keine sichtbaren cytostatischen Effekte auf die Zellen erkennbar sind;
EC₅₀ (HCMV) = Substanzkonzentration in µM, die den CPE (cytopathischen Effekt) um 50% im Vergleich zur unbehandelten Viruskontrolle hemmt;
SI (Selektivitätsindex) = CC₅₀ (NHDF) / EC₅₀ (HCMV).

Repräsentative in-vitro-Wirkdaten für die erfindungsgemäßen Verbindungen sind in Tabelle A wiedergegeben:

**Tabelle A**

| **Beispiel-** | **NHDF** | **HCMV** | **SI** |
|---|---|---|---|
| **Nr.** | **CC₅₀ [µM]** | **EC₅₀ [µM]** | **HCMV** |
| **1** | 2.5 | 0.20 | 12.5 |
| **5** | 6.0 | 0.23 | 26 |
| **8** | 7.7 | 0.19 | 41 |
| **16** | 7.8 | 0.10 | 78 |
| **22** | 8.4 | 0.38 | 22 |
| **24** | 4.7 | 0.25 | 19 |
| **28** | 6.1 | 0.10 | 63 |
| **31** | 4.5 | 0.032 | 151 |
| **34** | 8.4 | 0.019 | 442 |
| **35** | 4.8 | 0.0365 | 138 |
| **38** | 11.0 | 0.053 | 208 |
| **40** | 11.0 | 0.11 | 100 |
| **45** | 2.4 | 0.077 | 31 |
| **51** | 5.3 | 0.011 | 482 |
| **52** | 7.7 | 0.019 | 405 |
| **53** | 4.3 | 0.01 | 430 |
| **54** | 21.0 | 0.0085 | 2471 |
| **68** | 24.0 | 0.002 | 12000 |
| **74** | 2.6 | 0.092 | 28 |
| **79** | 7.7 | 0.053 | 145 |
| **92** | 5.3 | 0.031 | 171 |

Die Eignung der erfindungsgemäßen Verbindungen zur Behandlung von HCMV-Infektionen kann im folgenden Tiermodell gezeigt werden:

### HCMV Xenograft-Gelfoam^{®}-Modell

### Tiere:

5-6 Wochen alte immundefiziente Mäuse (16-20 g), Fox Chase SCID.NOD oder NOD.CB17-Prkdc/J werden von kommerziellen Züchtern (Taconic M&B, Dänemark; Jackson, USA) bezogen.

Die Tiere werden unter sterilen Bedingungen (einschließlich Streu und Futter) in Isolatoren gehalten.

### Virusanzucht:

Humanes Cytomegalovirus (HCMV), Stamm Davis oder AD169, wird *in vitro* auf humanen embryonalen Vorhautfibroblasten (NHDF-Zellen) angezüchtet. Nach Infektion der NHDF-Zellen mit einer Multiplizität der Infektion (M.O.I) von 0.01-0.03 werden die virusinfizierten Zellen 5-10 Tage später geerntet und in Gegenwart von Minimal Essential Medium (MEM), 20% foetalem Kälberserum (FKS) (v/v), 1% Glutamin (v/v), 1% Pen/Strep (v/v) mit 10% DMSO bei -80°C aufbewahrt. Nach serieller Verdünnung der virusinfizierten Zellen in Zehnerschritten erfolgt die Titerbestimmung auf 24-Well-Platten konfluenter NHDF-Zellen nach Fixierung und Färbung mit einer Giemsa-Formaldehyd-Lösung.

### Vorbereitung der Schwämme, Transplantation, Behandlung und Auswertung:

1×1×1 cm große Kollagenschwämme (Gelfoam^{®}; Fa. Peasel & Lorey, Best.-Nr. 407534; K.T. Chong et al., Abstracts of 39^{th} Interscience Conference on Antimicrobial Agents and Chemotherapy, 1999, S. 439) werden zunächst mit Phosphat-gepufferter Saline (PBS) benetzt, die eingeschlossenen Luftblasen durch Entgasen entfernt und dann in MEM, 10% FKS (v/v), 1 % Glutamin (v/v), 1% Pen/Strep (v/v) aufbewahrt. 1 x 10⁶ virusinfizierte NHDF-Zellen (Infektion mit HCMV-Davis oder HCMV AD169 M.O.I = 0.03) werden 3 Stunden nach Infektion abgelöst und in 20 µl MEM, 10% FKS (v/v), 1% Glutamin (v/v), 1% Pen/Strep (v/v) auf einen feuchten Schwamm getropft. Die Schwämme werden 3-4 Stunden inkubiert, um das adherieren der Zellen zu ermöglichen. Anschließend werden die Schwämme nach Zugabe von Medium (MEM, 10% FKS) (v/v), 1% Glutamin (v/v), 1% Pen/Strep (v/v)) über Nacht inkubiert. Zur Transplantation werden die immundefizienten Mäuse mit Avertin oder mit einer Ketamin/Xylazin/Azepromazin Mischung narkotisiert, das Rückenfell mit Hilfe eines Rasierers entfernt, die Oberhaut 1-2 cm geöffnet, entlastet und die feuchten Schwämme unter die Rückenhaut transplantiert. Die Operationswunde wird mit Gewebekleber oder Klammern verschlossen. 4-6 Stunden nach der Transplantation können die Mäuse zum ersten Mal behandelt werden (am Tag der Operation wird einmal behandelt). An den folgenden Tagen wird über einen Zeitraum von 8 Tagen dreimal täglich (7.00 Uhr und 14.00 Uhr und 19.00 Uhr), zweimal täglich (8 Uhr und 18 Uhr) oder einmal täglich (9 Uhr) peroral mit Substanz behandelt. Die Tagesdosis beträgt beispielsweise 1 oder 3 oder 10 oder 30 oder 100 mg/kg Körpergewicht, das Applikationsvolumen ist 10 ml/kg Körpergewicht. Die Formulierung der Substanzen erfolgt in Form einer 0.5%-igen Tylosesuspension / PBS mit 2% DMSO oder einer anderen geeigneten Mischung, die die Löslichkeit der Substanzen unterstützt, z. B. 2 % Ethanol, 2.5% Solutol, 95.5% PBS. 10 Tage nach Transplantation und ca. 16 Stunden nach der letzten Substanzapplikation werden die Tiere schmerzlos getötet und der Schwamm entnommen. Die virusinfizierten Zellen werden durch Kollagenaseverdau (330 U/1.5 ml) aus dem Schwamm freigesetzt und in Gegenwart von MEM, 10% FKS (v/v), 1% Glutamin (v/v), 1% Pen/Strep (v/v), 10% DMSO bei -140°C aufbewahrt. Die Auswertung erfolgt nach serieller Verdünnung der virusinfizierten Zellen in Zehnerschritten durch Titerbestimmung auf 24-Well-Platten konfluenter NHDF-Zellen nach Fixierung und Färbung mit einer Giemsa-Formaldehyd-Lösung. Ermittelt wird die Anzahl infizierter Zellen bzw. infektiöser Viruspartikel (infectious center assay) nach Substanzbehandlung im Vergleich zur placebobehandelten Kontrollgruppe. Die statistische Auswertung erfolgt mittels geeigneter Computerprogramme, z. B. GraphPad Prism.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus Wirkstoff, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der Verbindung von Beispiel 1, 1000 mg Ethanol (96%), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, der Wirkstoff wird der Suspension zugefügt. Unter Rühren erfolgt die Zugabe des Wassers. Bis zum Abschluss der Quellung des Rhodigels wird ca. 6h gerührt.

### Intravenös applizierbare Lösung:

### Zusammensetzung:

10-500 mg der Verbindung von Beispiel 1, 15 g Polyethylenglykol 400 und 250 g Wasser für Injektionszwecke.

### Herstellung:

Die Verbindung von Beispiel 1 wird zusammen mit Polyethylenglykol 400 in dem Wasser unter Rühren gelöst. Die Lösung wird sterilfiltriert (Porendurchmesser 0.22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 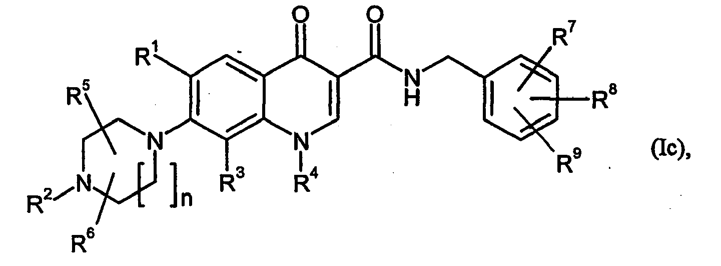 in welcher
n für eine Zahl 1 oder 2 steht,
R¹ für Fluor, Chlor oder Trifluormethyl steht,
R² für Wasserstoff oder C₁-C₆-Alkyl steht,
wobei Alkyl substituiert sein kann mit 1 bis 2 Substituenten, wobei die Substituenten unabhängig voneinander ausgewählt werden aus der Gruppe, bestehend aus Hydroxy, Aminocarbonyl, C₁-C₆-Alkoxy, C₁-C₆-Alkylcarbonyl, C₁-C₆-Alkylaminocarbonyl, C₃-C₈-Cycloalkylaminocarbonyl, C₁-C₆-Alkylaminocarbonylaminocarbonyl, C₁-C₆-Alkylsulfonylaminocarbonyl, Phenoxy, 5- oder 6-gliedriges Heteroaryloxy, 5- bis 7-gliedriges Heterocyclyl, 5- oder 6-gliedriges Heteroaryl, 5- bis 7-gliedriges Heterocyclylcarbonyl und 5- oder 6-gliedriges Heteroarylcarbonyl,
worin Alkoxy seinerseits substituiert sein kann mit einem Substituenten, wobei der Substituent ausgewählt wird aus der Gruppe, bestehend aus Hydroxy, Phenyl, 5- bis 7-gliedriges Heterocyclyl und 5- oder 6-gliedriges Heteroaryl,
oder
R² für C₁-C₆-Alkylcarbonyl, gegebenenfalls einmal mit C₁-C₄-Alkoxy substituiertes C₁-C₆-Alkylaminocarbonyl oder C₃-C₈-Cycloalkylaminocarbonyl steht,
wobei Alkylcarbonyl substituiert sein kann mit einem Substituenten, wobei der Substituent ausgewählt wird aus der Gruppe, bestehend aus Amino, C₁-C₆-Alkylamino, C₃-C₈-Cycloalkylamino und 4- bis 7-gliedriges Heterocyclyl,
R³ für Halogen, Cyano, Methoxy, Monofluormethoxy, Difluormethoxy, Trifluormethoxy oder Ethinyl steht,
R⁴ für C₁-C₆-Alkyl oder C₃-C₈-Cycloalkyl steht,
wobei Alkyl substituiert sein kann mit 1 bis 3 Substituenten, wobei die Substituenten unabhängig voneinander ausgewählt werden aus der Gruppe, bestehend aus Halogen, Hydroxy, Amino, Cyano, Trifluormethyl, Hydroxycarbonyl, Aminocarbonyl, C₁-C₆-Alkoxy, C₁-C₆-Alkylamino, C₁-C₆-Alkylcarbonyl und C₁-C₆-Alkoxycarbonyl,
und
wobei Cycloalkyl substituiert sein kann mit 1 bis 3 Substituenten, wobei die Substituenten unabhängig voneinander ausgewählt werden aus der Gruppe, bestehend aus Halogen, Hydroxy, Amino, Cyano, Trifluormethyl, Hydroxycarbonyl, Aminocarbonyl, C₁-C₆-Alkyl, C₁-C₆-Alkoxy, C₁-C₆-Alkylamino, C₁-C₆-Alkylcarbonyl und C₁-C₆-Alkoxycarbonyl,
R⁵ und R⁶ unabhängig voneinander für Wasserstoff, Methyl oder Ethyl stehen,
R⁷ und R⁸ unabhängig voneinander für Halogen, Hydroxy, Cyano, Trifluormethyl, Monofluormethoxy, Difluormethoxy, Trifluormethoxy, C₁-C₃-Alkyl oder C₁-C₃-Alkoxy stehen,
R⁹ für Wasserstoff, Halogen, Hydroxy, Cyano, Trifluormethyl, Monofluormethoxy, Difluormethoxy, Trifluormethoxy, C₁-C₃-Alkyl oder C₁-C₃-Alkoxy steht,
oder eines ihrer Salze, ihrer Solvate oder der Solvate ihrer Salze.

2. Verbindung nach Anspruch 1, **dadurch gekennzeichnet, dass** sie der Formel 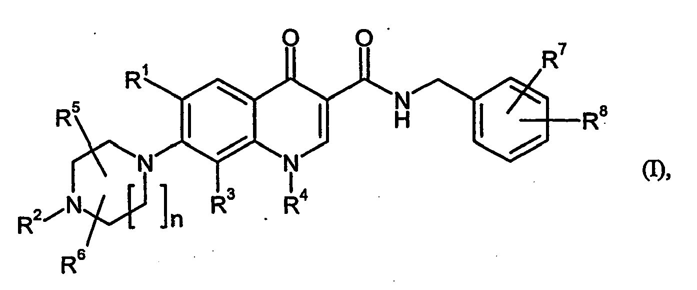 entspricht,
in welcher
n für eine Zahl 1 oder 2 steht,
R¹ für Fluor, Chlor oder Trifluormethyl steht,
R² für Wasserstoff oder C₁-C₆-Alkyl steht,
wobei Alkyl substituiert sein kann mit 1 bis 2 Substituenten, wobei die Substituenten unabhängig voneinander ausgewählt werden aus der Gruppe, bestehend aus Hydroxy, Aminocarbonyl, C₁-C₆-Alkoxy, C₁-C₆-Alkylcarbonyl, C₁-C₆-Alkylaminocarbonyl, C₃-C₈-Cycloalkylaminocarbonyl, C₁-C₆-Alkylsulfonylaminocarbonyl, Phenoxy, 5- oder 6-gliedriges Heteroaryloxy, 5- bis 7-gliedriges Heterocyclyl, 5- oder 6-gliedriges Heteroaryl, 5- bis 7-gliedriges Heterocyclylcarbonyl und 5- oder 6-gliedriges Heteroarylcarbonyl,
worin Alkoxy seinerseits substituiert sein kann mit einem Substituenten, wobei der Substituent ausgewählt wird aus der Gruppe, bestehend aus Hydroxy, Phenyl, 5- bis 7-gliedriges Heterocyclyl und 5- oder 6-gliedriges Heteroaryl,
oder
R² für C₁-C₆-Alkylcarbonyl, gegebenenfalls einmal mit C₁-C₄-Alkoxy substituiertes C₁-C₆-Alkylaminocarbonyl oder C₃-C₈-Cycloalkylaminocarbonyl steht,
wobei Alkylcarbonyl substituiert sein kann mit einem Substituenten, wobei der Substituent ausgewählt wird aus der Gruppe, bestehend aus Amino, C₁-C₆-Alkylamino, C₃-C₈-Cycloalkylamino und 4- bis 7-gliedriges Heterocyclyl,
R³ für Halogen, Cyano, Methoxy, Monofluormethoxy, Difluormethoxy, Trifluormethoxy oder Ethinyl steht,
R⁴ für C₁-C₆-Alkyl oder C₃-C₈-Cycloalkyl steht,
wobei Alkyl substituiert sein kann mit 1 bis 3 Substituenten, wobei die Substituenten unabhängig voneinander ausgewählt werden aus der Gruppe, bestehend aus Halogen, Hydroxy, Amino, Cyano, Trifluormethyl, Hydroxycarbonyl, Aminocarbonyl, C₁-C₆-Alkoxy, C₁-C₆-Alkylamino, C₁-C₆-Alkylcarbonyl und C₁-C₆-Alkoxycarbonyl,
und
wobei Cycloalkyl substituiert sein kann mit 1 bis 3 Substituenten, wobei die Substituenten unabhängig voneinander ausgewählt werden aus der Gruppe, bestehend aus Halogen, Hydroxy, Amino, Cyano, Trifluormethyl, Hydroxycarbonyl, Aminocarbonyl, C₁-C₆-Alkyl, C₁-C₆-Alkoxy, C₁-C₆-Alkylamino, C₁-C₆-Alkylcarbonyl und C₁-C₆-Alkoxycarbonyl,
R⁵ und R⁶ unabhängig voneinander für Wasserstoff, Methyl oder Ethyl stehen,
R⁷ und R⁸, unabhängig voneinander für Halogen, Hydroxy, Cyano, Trifluormethyl, Monofluormethoxy, Difluormethoxy, Trifluormethoxy, C₁-C₃-Alkyl oder C₁-C₃-Alkoxy stehen,
oder eines ihrer Salze, ihrer Solvate oder der Solvate ihrer Salze.

3. Verbindung nach Anspruch 2, **dadurch gekennzeichnet, dass** sie der Formel 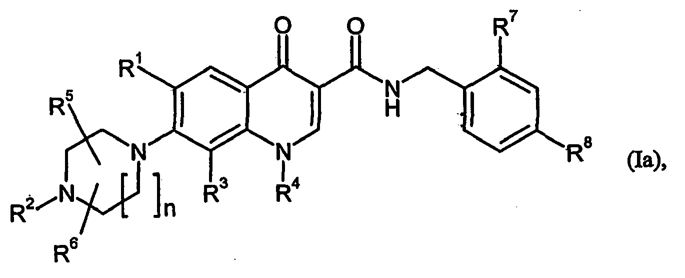 entspricht,
in welcher
n für eine Zahl 1 oder 2 steht,
R¹ für Fluor steht,
R² für Wasserstoff oder C₁-C₆-Alkyl steht,
wobei Alkyl substituiert sein kann mit 1 bis 2 Substituenten, wobei die Substituenten unabhängig voneinander ausgewählt werden aus der Gruppe, bestehend aus Hydroxy, C₁-C₆-Alkoxy, 5- oder 6-gliedriges Heterocyclyl und 5- oder 6-gliedriges Heterocyclylcarbonyl,
worin Alkoxy seinerseits substituiert sein kann mit einem Substituenten, wobei der Substituent ausgewählt wird aus der Gruppe, bestehend aus Hydroxy und 5- oder 6-gliedriges Heterocyclyl,
oder
R² für C₁-C₆-Alkylcarbonyl steht,
wobei Alkylcarbonyl substituiert ist mit einem Substituenten Amino,
R³ für Fluor, Chlor, Methoxy, Monofluormethoxy, Difluormethoxy, Trifluormethoxy oder Ethinyl steht,
R⁴ für C₁-C₄-Alkyl oder C₃-C₅-Cycloalkyl steht,
wobei Alkyl substituiert sein kann mit 1 bis 3 Substituenten, wobei die Substituenten unabhängig voneinander ausgewählt werden aus der Gruppe, bestehend aus Hydroxy und C₁-C₃-Alkoxy,
und
wobei Cycloalkyl substituiert sein kann mit 1 bis 3 Substituenten, wobei die Substituenten unabhängig voneinander ausgewählt werden aus der Gruppe, bestehend aus Halogen, Hydroxy, Trifluormethyl, C₁-C₃-Alkyl und C₁-C₃-Alkoxy,
R⁵ und R⁶ unabhängig voneinander für Wasserstoff oder Methyl stehen,
R⁷ und R⁸ unabhängig voneinander für Fluor, Chlor, Cyano, Trifluormethyl, Difluormethoxy, Trifluormethoxy, C₁-C₃-Alkyl oder C₁-C₃-Alkoxy stehen.

4. Verbindung nach Anspruch 2 oder 3, **dadurch gekennzeichnet, dass** sie der Formel 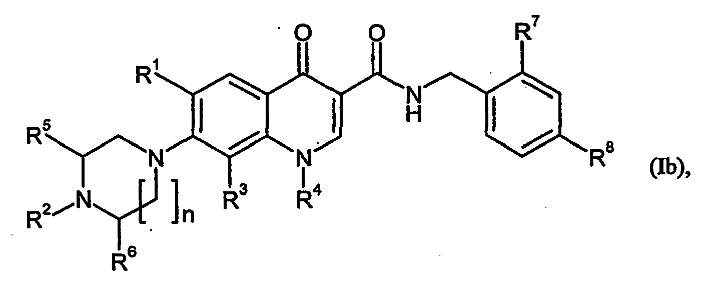 entspricht,
in welcher
n für eine Zahl 1 oder 2 steht,
R¹ für Fluor steht,
R² für Wasserstoff oder C₁-C₃-Alkyl steht,
wobei Alkyl substituiert sein kann mit 1 bis 2 Substituenten, wobei die Substituenten unabhängig voneinander ausgewählt werden aus der Gruppe, bestehend aus Hydroxy, Morpholin-2-ylcarbonyl, Morpholin-3-ylcarbonyl, Morpholin-4-ylcarbonyl, Piperidin-1-ylcarbonyl, Piperidin-2-ylcarbonyl, Piperidin-3-ylcarbonyl, Piperidin-4-ylcarbonyl, Pyrrolidin-2-ylcarbonyl, Pyrrolidin-3-ylcarbonyl und gegebenenfalls mit einem Substituenten Hydroxy substituiertes C₁-C₃-Alkoxy,
oder
R² für C₁-C₄-Alkylcarbonyl steht,
wobei Alkylcarbonyl substituiert ist mit einem Substituenten Amino,
R³ für Chlor, Methoxy, Difluormethoxy oder Trifluormethoxy steht,
R⁴ für Methyl, Ethyl oder Cyclopropyl steht,
wobei Ethyl substituiert sein kann mit 1 bis 3 Substituenten Fluor,
und
wobei Cyclopropyl substituiert sein kann mit 1 bis 2 Substituenten Fluor,
R⁵ und R⁶ unabhängig voneinander für Wasserstoff oder Methyl stehen,
R⁷ und R⁸ unabhängig voneinander für Chlor, Trifluormethyl, Trifluormethoxy oder Methyl stehen.

5. Verfahren zur Herstellung einer Verbindung der Formel (Ic) nach Anspruch 1, **dadurch gekennzeichnet, dass** eine Verbindung der Formel 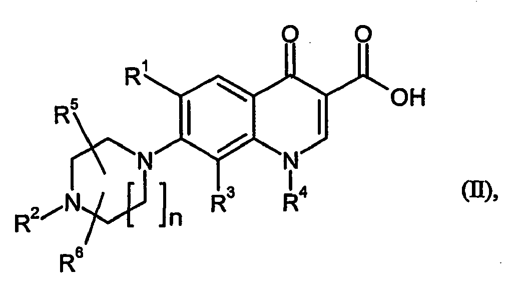 in welcher
n, R¹, R², R³, R⁴, R⁵ und R⁶ die in Anspruch 1 angegebene Bedeutung aufweisen,
mit einer Verbindung der Formel 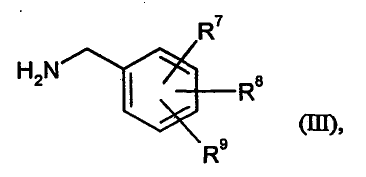 in welcher
R⁷, R⁸ und R⁹ die in Anspruch 1 angegebene Bedeutung aufweisen,
umgesetzt wird.

6. Verbindung nach einem der Ansprüche 1 bis 4 zur Behandlung und/oder Prophylaxe von Krankheiten.

7. Arzneimittel enthaltend eine Verbindung nach einem der Ansprüche 1 bis 4 in Kombination mit einem inerten, nichttoxischen, pharmazeutisch geeigneten Hilfsstoff.

8. Verwendung einer Verbindung nach einem der Ansprüche 1 bis 4 zur Herstellung eines Arzneimittels zur Behandlung und/oder Prophylaxe von Virusinfektionen.

9. Verwendung nach Anspruch 8, **dadurch gekennzeichnet, dass** die Virusinfektion eine Infektion mit dem humanen Cytomegalovirus (HCMV) oder einem anderen Vertreter der Gruppe der Herpes viridae ist

10. Arzneimittel nach Anspruch 7 zur Behandlung und/oder Prophylaxe von Virusinfektionen.

11. Verwendung einer antiviral wirksamen Menge mindestens einer Verbindung nach einem der Ansprüche 1 bis 4, eines Arzneimittels nach Anspruch 7 oder eines nach Anspruch 8 oder 9 erhaltenen Arzneimittels zur Herstellung eines Arzneimittels zur Bekämpfung von Virusinfektionen in Menschen und Tieren.

## Claims

1. Compound of formula 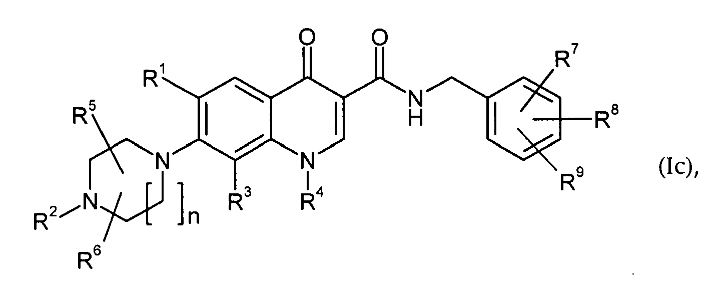 in which
n represents a number 1 or 2,
R¹ represents fluorine, chlorine or trifluoromethyl,
R² represents hydrogen or C₁-C₆-alkyl,
whereby alkyl can be substituted with 1 to 2 substituents, whereby the substituents are selected independently of one another from the group consisting of hydroxy, aminocarbonyl, C₁-C₆-alkoxy, C₁-C₆-alkylcarbonyl, C₁-C₆-alkylaminocarbonyl, C₃-C₈-cycloalkylaminocarbonyl, C₁-C₆-alkylaminocarbonylaminocarbonyl, C₁-C₆-alkylsulfonylaminocarbonyl, phenoxy, 5- or 6-membered heteroaryloxy, 5- to 7-membered heterocyclyl, 5- or 6-membered heteroaryl, 5- to 7-membered heterocyclylcarbonyl and 5- or 6-membered heteroarylcarbonyl,
wherein alkoxy in turn can be substituted with a substituent, whereby the substituent is selected from the group consisting of hydroxy, phenyl, 5- to 7-membered heterocyclyl and 5- or 6-membered heteroaryl,
or
R² represents C₁-C₆-alkylcarbonyl, optionally once C₁-C₄-alkoxy-substituted C₁-C₆-alkylaminocarbonyl, or C₃-C₈-cycloalkylaminocarbonyl,
whereby alkylcarbonyl can be substituted with a substituent, whereby the substituent is selected from the group consisting of amino, C₁-C₆-alkylamino, C₃-C₈-cycloalkylamino, and 4- to 7-membered heterocyclyl,
R³ represents halogen, cyano, methoxy, monofluoromethoxy, difluoromethoxy, trifluoromethoxy or ethynyl,
R⁴ represents C₁-C₆-alkyl or C₃-C₈-cycloalkyl,
whereby alkyl can be substituted with 1 to 3 substituents, whereby the substituents are selected independently of one another from the group consisting of halogen, hydroxy, amino, cyano, trifluoromethyl, hydroxycarbonyl, aminocarbonyl, C₁-C₆-alkoxy, C₁-C₆-alkylamino, C₁-C₆-alkylcarbonyl and C₁-C₆-alkoxycarbonyl,
and
whereby cycloalkyl can be substituted with 1 to 3 substituents, whereby the substituents are selected independently of one another from the group consisting of halogen, hydroxy, amino, cyano, trifluoromethyl, hydroxycarbonyl, aminocarbonyl, C₁-C₆-alkyl, C₁-C₆-alkoxy, C₁-C₆-alkylamino, C₁-C₆-alkylcarbonyl and C₁-C₆-alkoxycarbonyl,
R⁵ and R⁶ independently of one another represent hydrogen, methyl or ethyl,
R⁷ and R⁸ independently of one another represent halogen, hydroxy, cyano, trifluoromethyl, monofluoromethoxy, difluoromethoxy, trifluoromethoxy, C₁-C₃-alkyl or C₁-C₃-alkoxy,
R⁹ represents hydrogen, halogen, hydroxy, cyano, trifluoromethyl, mono-fluoromethoxy, difluoromethoxy, trifluoromethoxy, C₁-C₃-Alkyl or C₁-C₃-alkoxy,
or one of its salts, its solvates, or the solvates of its salts.

2. Compound according to claim 1, **characterized in that** it corresponds to formula 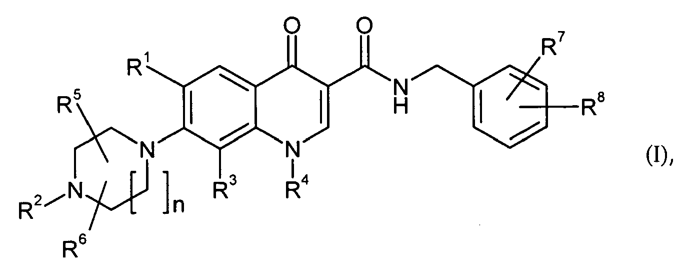 in which
n represents a number 1 or 2,
R¹ represents fluorine, chlorine or trifluoromethyl,
R² represents hydrogen or C₁-C₆-alkyl,
whereby alkyl can be substituted with 1 to 2 substituents, whereby the substituents are selected independently of one another from the group consisting of hydroxy, aminocarbonyl, C₁-C₆-alkoxy, C₁-C₆-alkylcarbonyl, C₁-C₆-alkylaminocarbonyl, C₃-C₈-cycloalkylaminocarbonyl, C₁-C₆-alkylsulfonylaminocarbonyl, phenoxy, 5- or 6-membered heteroaryloxy, 5- to 7-membered heterocyclyl, 5- or 6-membered heteroaryl, 5- to 7-membered heterocyclylcarbonyl and 5- or 6-membered heteroarylcarbonyl,
wherein alkoxy in turn can be substituted with a substituent, whereby the substituent is selected from the group consisting of hydroxy, phenyl, 5- to 7-membered heterocyclyl and 5- or 6-membered heteroaryl,
or
R² represents C₁-C₆-alkylcarbonyl, optionally once C₁-C₄-alkoxy-substituted C₁-C₆-alkylaminocarbonyl, or C₃-C₈-cycloalkylaminocarbonyl,
whereby alkylcarbonyl can be substituted with a substituent, whereby the substituent is selected from the group consisting of amino, C₁-C₆-alkylamino, C₃-C₈-cycloalkylamino, and 4- to 7-membered heterocyclyl,
R³ represents halogen, cyano, methoxy, monofluoromethoxy, difluoromethoxy, trifluoromethoxy or ethynyl,
R⁴ represents C₁-C₆-alkyl or C₃-C₈-cycloalkyl,
whereby alkyl can be substituted with 1 to 3 substituents, whereby the substituents are selected independently of one another from the group consisting of halogen, hydroxy, amino, cyano, trifluoromethyl, hydroxycarbonyl, aminocarbonyl, C₁-C₆-alkoxy, C₁-C₆-alkylamino, C₁-C₆-alkylcarbonyl and C₁-C₆-alkoxycarbonyl,
and
whereby cycloalkyl can be substituted with 1 to 3 substituents, whereby the substituents are selected independently of one another from the group consisting of halogen, hydroxy, amino, cyano, trifluoromethyl, hydroxycarbonyl, aminocarbonyl, C₁-C₆-alkyl, C₁-C₆-alkoxy, C₁-C₆-alkylamino, C₁-C₆-alkylcarbonyl and C₁-C₆-alkoxycarbonyl,
R⁵ and R⁶ independently of one another represent hydrogen, methyl or ethyl,
R⁷ and R⁸ independently of one another represent halogen, hydroxy, cyano, trifluoromethyl, monofluoromethoxy, difluoromethoxy, trifluoromethoxy, C₁-C₃-alkyl or C₁-C₃-alkoxy,
or one of its salts, its solvates or the solvates of its salts.

3. Compound according to claim 2, **characterized in that** it corresponds to formula 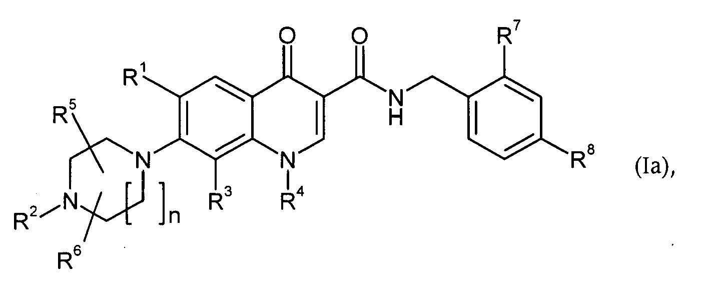 in which
n represents a number 1 or 2,
R¹ represents fluorine,
R² represents hydrogen or C₁-C₆-alkyl,
whereby alkyl can be substituted with 1 or 2 substituents, whereby the substituents are selected independently of one another from the group consisting of hydroxy, C₁-C₆-alkoxy, 5- or 6-membered heterocyclyl and 5- or 6-membered heterocyclylcarbonyl,
wherein alkoxy in turn can be substituted with a substituent, whereby the substituent is selected from the group consisting of hydroxy and 5- or 6-membered heterocyclyl,
or
R² represents C₁-C₆-alkylcarbonyl,
whereby alkylcarbonyl is substituted with an amino substituent,
R³ represents fluorine, chlorine, methoxy, monofluoromethoxy, difluoromethoxy, trifluoromethoxy or ethynyl,
R⁴ represents C₁-C₄-alkyl or C₃-C₅-cycloalkyl,
whereby alkyl can be substituted with 1 to 3 substituents, whereby the substituents are selected independently of one another from the group consisting of hydroxy and C₁-C₃-alkoxy,
and
whereby cycloalkyl can be substituted with 1 to 3 substituents, whereby the substituents are selected independently of one another from the group consisting of halogen, hydroxy, trifluoromethyl, C₁-C₃-alkyl and C₁-C₃-alkoxy,
R⁵ and R⁶ independently of one another represent hydrogen or methyl,
R⁷ and R⁸ independently of one another represent fluorine, chlorine, cyano, trifluoromethyl, difluoromethoxy, trifluoromethoxy, C₁-C₃-alkyl or C₁-C₃-alkoxy.

4. Compound according to claim 2 or 3, **characterized in that** it corresponds to formula 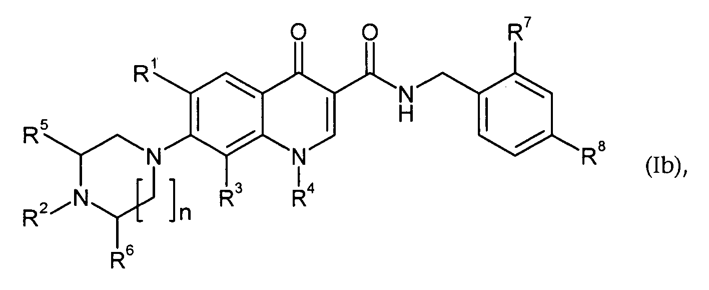 in which
n represents a number 1 or 2,
R¹ represents fluorine,
R² represents hydrogen or C₁-C₃-alkyl,
whereby alkyl can be substituted with 1 or 2 substituents, whereby the substituents are selected independently of one another from the group consisting of hydroxy, morpholin-2-ylcarbonyl, morpholin-3-ylcarbonyl, morpholin-4-ylcarbonyl, piperidin-1-ylcarbonyl, piperidin-2-ylcarbonyl, piperidin-3-ylcarbonyl, piperidin-4-ylcarbonyl, pyrrolidin-2-ylcarbonyl, pyrrolidin-3-ylcarbonyl and C₁-C₃-alkoxy which is optionally substituted with a hydroxy substituent,
or
R² represents C₁-C₄-alkylcarbonyl,
whereby alkylcarbonyl is substituted with an amino substituent,
R³ represents chlorine, methoxy, difluoromethoxy or trifluoromethoxy,
R⁴ represents methyl, ethyl or cyclopropyl,
whereby ethyl can be substituted with 1 to 3 fluorine substituents,
and
whereby cyclopropyl can be substituted with 1 to 2 fluorine substituents,
R⁵ and R⁶ independently of one another represent hydrogen or methyl,
R⁷ and R⁸ independently of one another represent chlorine, trifluoromethyl, trifluoromethoxy or methyl.

5. Process for preparing a compound of formula (Ic) according to claim 1, **characterized in that** a compound of formula 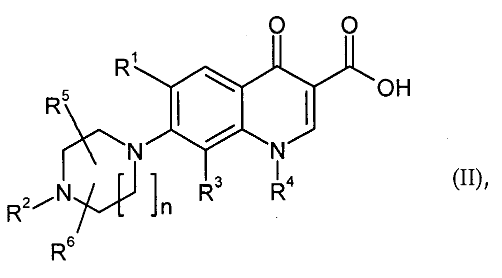 in which
n, R¹, R², R³, R⁴, R⁵ and R⁶ have the meaning indicated in claim 1,
is reacted with a compound of formula 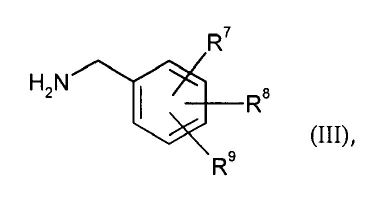 in which
R⁷, R⁸ and R⁹ have the meaning indicated in claim 1.

6. Compound according to one of claims 1 to 4 for the treatment and/or prophylaxis of diseases.

7. Medicament comprising a compound according to one of claims 1 to 4 in combination with an inert, non-toxic, pharmaceutically acceptable excipient.

8. Use of a compound according to one of claims 1 to 4 for the production of a medicament for the treatment and/or prophylaxis of viral infections.

9. Use according to claim 8, **characterized in that** the viral infection is an infection with the human cytomegalovirus (HCMV) or another representative of the group of herpes viridae.

10. Medicament according to claim 7 for the treatment and/or prophylaxis of viral infections.

11. Use of an antivirally effective amount of at least one compound according to one of Claims 1 to 4, of a medicament according to claim 7 or of a medicament obtained according to claim 8 or 9 for the production of a medicament for controlling viral infections in humans and animals.

## Revendications

1. Composé de formule 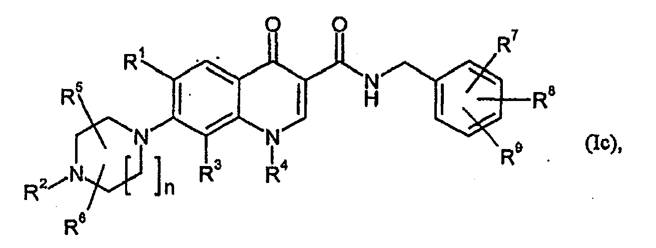 dans laquelle
n est le nombre 1 ou 2,
R¹ représente un fluor, un chlore ou un groupe trifluorométhyle,
R² représente l'hydrogène ou un groupe alkyle en C₁-C₆,
où le groupe alkyle peut être substitué par 1 à 2 substituants, les substituants étant sélectionnés indépendamment l'un de l'autre dans le groupe composé des groupes hydroxy, aminocarbonyle, alcoxy en C₁-C₆, (alkyle en C₁-C₆)-carbonyle, (alkyle en C₁-C₆)-aminocarbonyle, (cycloalkyle en C₃-C₈)-aminocarbonyle, (alkyle en C₁-C₆)-aminocarbonylaminocarbonyle, (alkyle en C₁-C₆)-sulfonylaminocarbonyle, phénoxy, hétéroaryloxy à 5 ou 6 chaînons, hétérocyclyle de 5 à 7 chaînons, hétéroaryle à 5 ou 6 chaînons, hétérocyclylcarbonyle de 5 à 7 chaînons et hétéroarylcarbonyle à 5 ou 6 chaînons,
où le groupe alcoxy peut lui-même être substitué par un substituant, le substituant étant sélectionné dans le groupe composé des groupes hydroxy, phényle, hétérocyclyle de 5 à 7 chaînons et hétéroaryle à 5 ou 6 chaînons,
ou
R² représente un groupe (alkyle en C₁-C₆)-carbonyle, (alkyle en C₁-C₆)-aminocarbonyle ou (cycloalkyle en C₃-C₈)-aminocarbonyle éventuellement monosubstitué par un groupe alcoxy en C₁-C₄,
où le groupe alkylcarbonyle peut être substitué par un substituant, le substituant étant sélectionné dans le groupe composé des groupes amino, (alkyle en C₁-C₆)-amino, (cycloalkyle en C₃-C₈)-amino et hétérocyclyle de 4 à 7 chaînons,
R³ représente un halogène, un groupe cyano, méthoxy, monofluorométhoxy, difluorométhoxy, trifluorométhoxy ou éthinyle,
R⁴ représente un groupe alkyle en C₁-C₆ ou cycloalkyle en C₃-C₈,
où le groupe alkyle peut être substitué par 1 à 3 substituants, les substituants étant sélectionnés indépendamment les uns des autres dans le groupe composé d'un halogène, des groupes hydroxy, amino, cyano, trifluorométhyle, hydroxycarbonyle, aminocarbonyle, alcoxy en C₁-C₆, (alkyle en C₁-C₆)-amino, (alkyle en C₁-C₆)-carbonyle et (alcoxy en C₁-C₆)-carbonyle,
et
où le groupe cycloalkyle peut être substitué par 1 à 3 substituants, les substituants étant sélectionnés indépendamment les uns des autres dans le groupe composé d'un halogène, des groupes hydroxy, amino, cyano, trifluorométhyle, hydroxycarbonyle, aminocarbonyle, alkyle en C₁-C₆, alcoxy en C₁-C₆, (alkyle en C₁-C₆)amino, (alkyle en C₁-C₆)carbonyle, et (alcoxy en C₁-C₆)carbonyle,
R⁵ et R⁶ représentent indépendamment l'un de l'autre l'hydrogène, un groupe méthyle ou éthyle,
R⁷ et R⁸ représentent indépendamment l'un de l'autre un halogène, un groupe hydroxy, cyano, trifluorométhyle, monofluorométhoxy, difluorométhoxy, trifluorométhoxy, alkyle en C₁-C₃ ou alcoxy en C₁-C₃,
R⁹ représente l'hydrogène, un halogène, un groupe hydroxy, cyano, trifluorométhyle, monofluorométhoxy, difluorométhoxy, trifluorométhoxy, alkyle en C₁-C₃ ou alcoxy en C₁-C₃,
ou un de ses sels, ses solvates ou les solvates de ses sels.

2. Composé selon la revendication 1, **caractérisé en ce qu'**il correspond à la formule 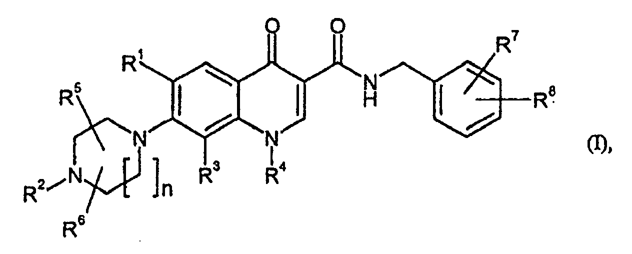 dans laquelle
n est le nombre 1 ou 2,
R¹ représente un fluor, un chlore ou un groupe trifluorométhyle,
R² représente l'hydrogène ou un groupe alkyle en C₁-C₆,
où le groupe alkyle peut être substitué par 1 à 2 substituants, les substituants étant sélectionnés indépendamment l'un de l'autre dans le groupe composé des groupes hydroxy, aminocarbonyle, alcoxy en C₁-C₆, (alkyle en C₁-C₆)-carbonyle, (alkyle en C₁-C₆)-aminocarbonyle, (cycloalkyle en C₃-C₈)-aminocarbonyle, (alkyle en C₁-C₆)-sulfonylaminocarbonyle, phénoxy, hétéroaryloxy à 5 ou 6 chaînons, hétérocyclyle de 5 à 7 chaînons, hétéroaryle à 5 ou 6 chaînons, hétérocyclylcarbonyle de 5 à 7 chaînons et hétéroarylcarbonyle à 5 ou 6 chaînons,
où le groupe alcoxy peut lui-même être substitué par un substituant, le substituant étant sélectionné dans le groupe composé des groupes hydroxy, phényle, hétérocyclyle de 5 à 7 chaînons et hétéroaryle à 5 ou 6 chaînons,
ou
R² représente un groupe (alkyle en C₁-C₆)-carbonyle, (alkyle en C₁-C₆)-aminocarbonyle ou (cycloalkyle en C₃-C₈)-aminocarbonyle éventuellement monosubstitué par un groupe alcoxy en C₁-C₄,
où le groupe alkylcarbonyle peut être substitué par un substituant, le substituant étant sélectionné dans le groupe composé des groupes amino, (alkyle en C₁-C₆)-amino, (cycloalkyle en C₃-C₈)-amino et hétérocyclyle de 4 à 7 chaînons,
R³ représente un halogène, un groupe cyano, méthoxy, monofluorométhoxy, difluorométhoxy, trifluorométhoxy ou éthinyle,
R⁴ représente un groupe alkyle en C₁-C₆ ou un cycloalkyle en C₃-C₈,
où le groupe alkyle peut être substitué par 1 à 3 substituants, les substituants étant sélectionnés indépendamment les uns des autres dans le groupe composé d'un halogène, des groupes hydroxy, amino, cyano, trifluorométhyle, hydroxycarbonyle, aminocarbonyle, alcoxy en C₁-C₆, (alkyle en C₁-C₆)-amino, (alkyle en C₁-C₆)-carbonyle et (alcoxy en C₁-C₆)-carbonyle,
et
où le groupe cycloalkyle peut être substitué par 1 à 3 substituants, les substituants étant sélectionnés indépendamment les uns des autres dans le groupe composé d'un halogène, des groupes hydroxy, amino, cyano, trifluorométhyle, hydroxycarbonyle, aminocarbonyle, alkyle en C₁-C₆, alcoxy en C₁-C₆, (alkyle en C₁-C₆)-amino, (alkyle en C₁-C₆)-carbonyle, et (alcoxy en C₁-C₆)-carbonyle,
R⁵ et R⁶ représentent indépendamment l'un de l'autre l'hydrogène, un groupe méthyle ou éthyle,
R⁷ et R⁸ représentent indépendamment l'un de l'autre un halogène, un groupe hydroxy, cyano, trifluorométhyle, monofluorométhoxy, difluorométhoxy, trifluorométhoxy, alkyle en C₁-C₃ ou alcoxy en C₁-C₃,
ou un de ses sels, ses solvates ou les solvates de ses sels.

3. Composé selon la revendication 2, **caractérisé en ce qu'**il correspond à la formule 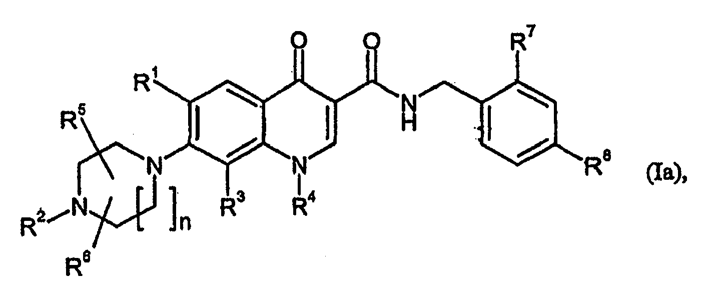 dans laquelle
n est le nombre 1 ou 2,
R¹ représente un fluor,
R² représente l'hydrogène ou un groupe alkyle en C₁-C₆,
où le groupe alkyle peut être substitué par 1 à 2 substituants, les substituants étant sélectionnés indépendamment les uns des autres dans le groupe composé des groupes hydroxy, alcoxy en C₁-C₆, hétérocyclyle à 5 ou 6 chaînons et hétérocyclylcarbonyle à 5 ou 6 chaînons,
où le groupe alcoxy peut lui-même être substitué par un substituant, le substituant étant sélectionné dans le groupe composé des groupes hydroxy et hétérocyclyle à 5 ou 6 chaînons,
ou
R² représente un (alkyle en C₁-C₆)-carbonyle,
où le groupe alkylcarbonyle peut être substitué par un substituant amino,
R³ représente un fluor, un chlore, un groupe méthoxy, monofluorométhoxy, difluorométhoxy, trifluorométhoxy ou éthinyle,
R⁴ représente un groupe alkyle en C₁-C₄ ou cycloalkyle en C₃-C₅,
où le groupe alkyle peut être substitué par 1 à 3 substituants, les substituants étant sélectionnés indépendamment les uns des autres dans le groupe composé des groupes hydroxy et alcoxy en C₁-C₃,
et
où le groupe cycloalkyle peut être substitué par 1 à 3 substituants, les substituants étant sélectionnés indépendamment les uns des autres dans le groupe composé d'un halogène, des groupes hydroxy, trifluorométhyle, alkyle en C₁-C₃ et alcoxy en C₁-C₃,
R⁵ et R⁶ représentent indépendamment l'un de l'autre l'hydrogène ou un groupe méthyle,
R⁷ et R⁸ représentent indépendamment l'un de l'autre un fluor, un chlore, un groupe cyano, trifluorométhyle, difluorométhoxy, trifluorométhoxy, alkyle en C₁-C₃ ou alcoxy en C₁-C₃.

4. Composé selon la revendication 2 ou 3, **caractérisé en ce qu'**il correspond à la formule 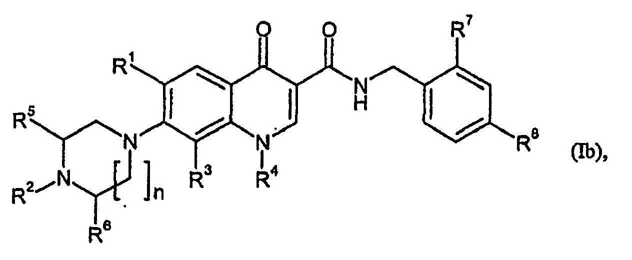 dans laquelle
n est le nombre 1 ou 2,
R¹ représente un fluor,
R² représente l'hydrogène ou un groupe alkyle en C₁-C₃,
où le groupe alkyle peut être substitué par 1 à 2 substituants, les substituants étant sélectionnés indépendamment l'un de l'autre dans le groupe composé des groupes hydroxy, morpholin-2-ylcarbonyle, morpholin-3-ylcarbonyle, morpholin-4-ylcarbonyle, pipéridin-1-ylcarbonyle, pipéridin-2-ylcarbonyle, pipéridin-3-ylcarbonyle, pipéridin-4-ylcarbonyle, pyrrolidin-2-ylcarbonyle, pyrrolidin-3-ylcarbonyle et alcoxy en C₁-C₃ éventuellement substitué par un hydroxy,
ou
R² représente un groupe (alkyle en C₁-C₄)-carbonyle,
où le groupe alkylcarbonyle peut être substitué par un substituant amino,
R³ représente un chlore, un groupe méthoxy, difluorométhoxy ou trifluorométhoxy,
R⁴ représente un groupe méthyle, éthyle ou cyclopropyle,
où le groupe éthyle peut être substitué par 1 à 3 substituants fluor,
et
où le groupe cyclopropyle peut être substitué par 1 à 2 substituants fluor,
R⁵ et R⁶ représentent indépendamment l'un de l'autre l'hydrogène ou un groupe méthyle,
R⁷ et R⁸ représentent indépendamment l'un de l'autre un chlore, un groupe trifluorométhyle, trifluorométhoxy ou méthyle.

5. Procédé de préparation d'un composé de formule (Ic) selon la revendication 1, **caractérisé en ce que** l'on fait réagir un composé de formule 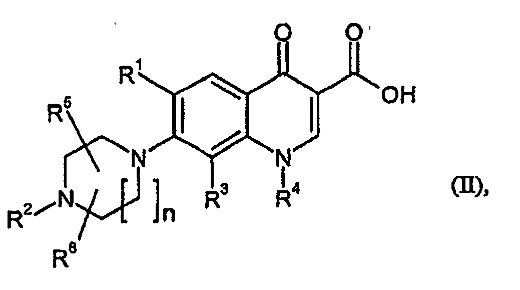 dans laquelle
n, R¹, R², R³, R⁴, R⁵ et R⁶ ont la signification indiquée dans la revendication 1,
avec un composé de formule 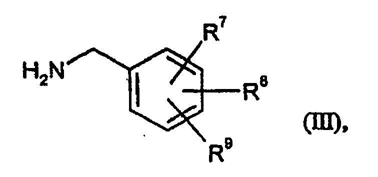 dans laquelle
R⁷, R⁸ et R⁹ ont la signification indiquée dans la revendication 1.

6. Composé selon l'une des revendications 1 à 4 destiné au traitement et/ou à la prophylaxie de maladies.

7. Médicament contenant un composé selon l'une des revendications 1 à 4 en combinaison avec un auxiliaire inerte, non toxique et approprié sur le plan pharmaceutique.

8. Utilisation d'un composé selon l'une des revendications 1 à 4 pour préparer un médicament destiné au traitement et/ou à la prophylaxie d'infections virales.

9. Utilisation selon la revendication 8, **caractérisée en ce que** l'infection virale est une infection par le cytomégalovirus humain (CMVH) ou un autre représentant du groupe des virus de l'herpès.

10. Médicament selon la revendication 7 destiné au traitement et/ou à la prophylaxie d'infections virales.

11. Utilisation d'une quantité efficace sur le plan antiviral d'au moins un composé selon l'une des revendications 1 à 4, d'un médicament selon la revendication 7 ou d'un médicament obtenu selon la revendication 8 ou 9 pour la fabrication d'un médicament destiné à combattre des infections virales chez l'homme et l'animal.
